(19) Europäisches Patentamt
European Patent Office
Office européen des brevets

(11) EP 2 325 218 A1

(12) **EUROPEAN PATENT APPLICATION**

(43) Date of publication:
**25.05.2011 Bulletin 2011/21**

(21) Application number: **11000960.2**

(22) Date of filing: **22.07.2005**

(51) Int Cl.:
***C08F 226/06*** *(2006.01)*
***C08F 2/38*** *(2006.01)*
***C08F 220/34*** *(2006.01)*
***C08F 230/02*** *(2006.01)*
***A61K 31/765*** *(2006.01)*
***C08F 220/18*** *(2006.01)*
***C08F 220/36*** *(2006.01)*

(84) Designated Contracting States:
**AT BE BG CH CY CZ DE DK EE ES FI FR GB GR HU IE IS IT LI LT LU LV MC NL PL PT RO SE SI SK TR**

(30) Priority: **23.07.2004 US 590434 P**

(62) Document number(s) of the earlier application(s) in accordance with Art. 76 EPC:
**05857895.6 / 1 771 183**

(71) Applicant: **The Trustees Of The University Of Pennsylvania**
**Philadelphia PA 19104-6283 (US)**

(72) Inventors:
- **Kuroda, Kenichi**
  **Ann Arbor**
  **Michigan 48105 (US)**
- **Degrado, William F.**
  **Moylan, PA 19065 (US)**

(74) Representative: **Luderschmidt, Schüler & Partner**
**Patentanwälte**
**John-F.-Kennedy-Strasse 4**
**65189 Wiesbaden (DE)**

Remarks:
This application was filed on 07-02-2011 as a divisional application to the application mentioned under INID code 62.

(54) **Antimicrobial copolymers and uses thereof**

(57) The present invention discloses random copolymers and methods of their use, including use of the copolymers as antimicrobial agents in pharmaceutical and non-pharmaceutical applications. The present invention also discloses compositions of the copolymers, and methods of preparing the copolymers

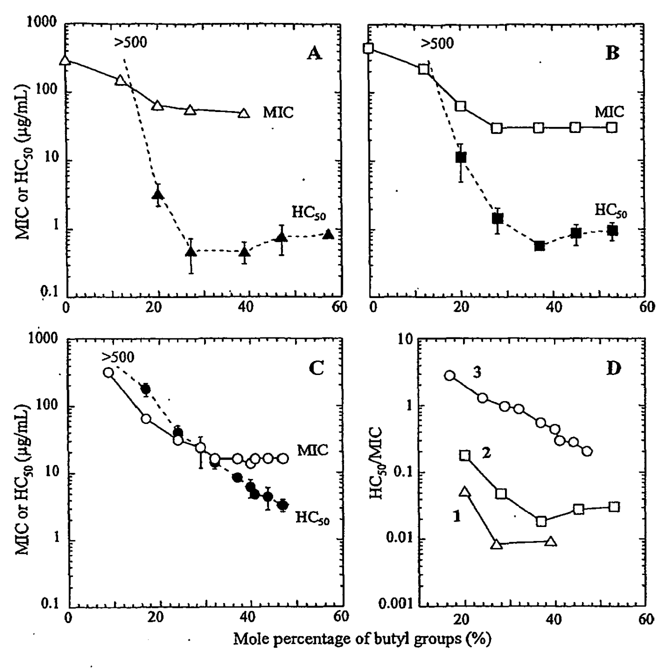


**FIGs. 6A-6D**

Printed by Jouve, 75001 PARIS (FR)

EP 2 325 218 A1

## Description

Field of the Invention

The present invention relates to amphiphilic random copolymers that exhibit antimicrobial activity. The copolymers are useful as antimicrobial agents in a number of pharmaceutical and non-pharmaceutical applications.

Related Art

The host defense peptides constitute a class of peptides that have the specific function of protecting the host from bacterial infection. (Zasloff, M., Nature 415:289 (2002).) These compounds represent the first line of defense against microbes for many species, including plants, insects, worms, and mammals (Boman, H.G., Immunol. Rev. 173:5-16 (2000); Hancock, R.E., and Lehrer, R., Trends Biotechnol. 16:82-88 (1998)). In mammals, the peptides are produced and secreted by skin, mucosal surfaces and neutrophils. There are many different classes of natural host defense peptides (Zasloff, M., Curr. Opin. Immunol. 4:3-7 (1992); Zasloff, M., Trends Pharmacol. Sci. 21:236-238 (2000); Steiner, H., et al., Nature, 292:246-248 (1981); Ganz, T., et al., Eur. J. Haematol. 44:1-8 (1990); Tang, Y.Q., et al., Science 286: 498-502 (1999); Ganz, T., et al., J. Clin. Invest. 76:1427-1435 (1985); Landon, C., et al., Protein Sci. 6:1878-1884 (1997); Zhao, C., et al., FEBS Lett. 346:285-288 (1994); Peggion, E., et al., Biopolymers (Peptide Science) 43:419-431 (1998); Dempsey, C.E.,Biochim. Biophys. Acta 1031:143-161 (1990)), but, in general, most contain between 20-40 amino acid residues and adopt an amphiphilic secondary structure.

Although host defense peptides are found in a wide variety of species and are composed of many different sequences, their physiochemical properties are remarkably similar. They adopt an amphiphilic architecture with positively charged groups segregated to one side of the secondary structure and hydrophobic groups on the opposite surface, giving them the ability to disrupt the lipid bilayers of bacterial membranes. By adopting a standard helical conformation upon binding to cell membranes, they display a facially amphiphilic structure wherein positively charged cationic side chain groups and hydrophobic side chain groups are regularly distributed on the different sides of helix surface. The cationic groups on the peptides allow the peptides to selectively interact with bacterial membrane surfaces via electrostatic interactions with the dense population of negatively charged lipids typically found on the surface of bacterial cells, compared to the lesser charge observed on animal and plant cell surfaces. The hydrophobic groups are integrated into the lipid bilayer, inducing formation of an extended peptide-lipid complex, and resulting in membrane defects and cell death.

In addition to antibacterial activity, several of the host defense peptides possess antifungal activity (see, e.g., DeLucca, A.J., and Walsh, T.J., Antimicob. Agents Chemother. 43:1-11 (1999)) and antiviral activity (see, e.g., Belaid, A., et al., J. Med. Virol. 66:229-234 (2002); Egal, M., et al., Int. J. Antimicrob. Agents 13:57-60 (1999); Andersen, J.H., et al., Antiviral Rs. 51:141-149 (2001); and Bastian, A., and Schafer, H., Regul. Pept. 15:157-161 (2001)).

Using protein design principles, investigators have designed synthetic antimicrobial peptides by idealizing the amphiphilic $\alpha$-helical arrangement of sidechains observed in natural host defense peptides, leading to a large number of potent and selective antimicrobial compounds (Tossi, A., et al., Biopolymers 55:4-30 (2000); DeGrado, W.F., Adv. Protein. Chem. 39:51-124 (1988); Maloy, W.L., and Kari, U.P., Biopolymers 37:105-122 (1995); Zasloff, M., Curr. Opin. Immunol. 4:3-7 (1992); Boman, H.G., et al., Eur. J. Biochem. 201:23-31 (1991); Oren, Z., and Shai, Y., Biopolymers 47: 451-463 (1998)). As a consequence, synthetic antimicrobial polymers have typically been designed to have a relatively rigid polymer backbone coupled with a regular distribution of hydrophobic and hydrophobic groups along the polymer backbone chain (Liu, D., et al., Angew. Chem. Int. Ed., 43:1158 (2004); Tew, G.N., et al., PNAS 99:5110 (2002); Ilker, M.F., et al., Macromolecules 37:694 (2004); Arnt, L., and Tew, G.N., J. Am. Chem. Soc. 124:7664 (2002)). A recent report, however, has suggested that a regular pattern of substitution is not required, since a random distribution of tertiary amine and alkyl groups along a polystyrene backbone also demonstrates antimicrobial activity (Gelman, M.A., et al., Org. Lett. 6:557 (2004)).

There exists a need for effective antimicrobial compounds that provide an economic as well as a human health benefit.

BRIEF SUMMARY OF THE INVENTION

The present invention provides random copolymers and methods of their use, including use of the copolymers as antimicrobial agents in pharmaceutical and non-pharmaceutical applications. The present invention also discloses compositions of the copolymers and methods of preparing the copolymers.

Thus, the present invention is directed to a random copolymer having a monomer unit of Formula **I:**

$$-[CH_2-C(R^1)(B_1)]_n- \qquad \textbf{I}$$

and a monomer unit of Formula **II:**

$$-[CH_2-C(R^2)(D_2)]_m- \qquad \textbf{II}$$

or an acceptable salt or solvate thereof, wherein the copolymer has a degree of polymerization of about 5 to about 50; wherein a chain transfer agent A is used to control the degree of polymerization of the copolymer; and wherein $R^1$, $B_1$, $R^2$, $D_2$, m and n are as defined below.

The invention is also directed to a random copolymer of Formula III:

$$\mathbf{A\text{-}(B)_{n1}\text{-}(D)_{m1}\text{-}H} \qquad \textbf{III}$$

or an acceptable salt or solvate thereof, wherein the copolymer is a random copolymer of B and D monomers; wherein the copolymer has a degree of polymerization of about 5 to about 50; and wherein A, $B_{n1}$, and $D_{m1}$ are as defined below.

The invention is further directed to a pharmaceutical composition comprising a copolymer of Formula III, as defined below, and a pharmaceutically acceptable carrier or diluent.

The invention is also directed to a method of treating a microbial infection in an animal in need thereof, the method comprising administering to the animal an effective amount of a pharmaceutical composition comprising a copolymer of Formula III, as defined below, and a pharmaceutically acceptable carrier or diluent.

The invention is directed to a method of providing an antidote to low molecular weight heparin overdose in an animal, the method comprising administering to the animal a pharmaceutical composition comprising a copolymer of Formula III, as defined below, and a pharmaceutically acceptable carrier or diluent.

The invention is further directed to a method of killing or inhibiting the growth of a microorganism, the method comprising contacting the microorganism with an effective amount of a copolymer of Formula III, as defined below. The invention is also directed to the method of killing or inhibiting the growth of the microorganism, wherein the copolymer is covalently attached to the substrate or wherein the copolymer is present on a substrate.

The invention is also directed to an antimicrobial composition comprising a copolymer of Formula III, as defined below, and a composition selected from the group consisting of paints, lacquers, coatings, varnishes, caulks, grouts, adhesives, resins, films, cosmetics, soaps, lotions, handwashes, and detergents..

The invention is also directed to any of the above methods wherein the microorganism is a bacterial cell, a fungus, or a virus.

BRIEF DESCRIPTION OF THE DRAWINGS/FIGURES

FIGs. 1A and 1B show a general scheme illustrating free-radical polymerization of a polymer in the presence of a chain transfer agent. FIG. 1A presents the general steps occurring during free-radical polymerization. FIG. 1B presents the equations used for calculating polymer length, or degree of polymerization, of a polymer synthesized by free-radical polymerization.

FIG. 2 shows a plot of the reciprocal of average degrees of polymerization ("DP") of polymethacrylate homopolymer 2 in Scheme 2 in Example 1 obtained at different chain transfer agent (CTA) concentrations against [CTA]/[1]. The chain transfer constant, CT, determined from the slope is 0.86.

FIG. 3 shows the molecular weights (number average molecular weights) for each series of the n-butyl methacrylate random copolymers of Example 3. Molecular weights were calculated from the DP (average degree of polymerization) determined by NMR for each copolymer and are plotted as a function of the percentage of butyl group of copolymer in each series.

FIG. 4 shows the results of antimicrobial assays performed with the n-butyl methacrylate random copolymers of Example 3. The minimum inhibitory concentration ("MIC") of each copolymer is plotted as a function of the mole percent of copolymer hydrophobic group (butyl group).

FIG. 5 shows the solubility limit in antimicrobial assay media (Mueller-Hinton), in μg/mL, for each of the three series of n-butyl methacrylate random copolymers of Example 3 plotted as a function of the mole percent of copolymer hydrophobic (butyl) group.

FIGs. 6A-6D show the results of hemolytic assays and antimicrobial assays performed with each of the three sets of n-butyl methacrylate random copolymers of Example 3. FIG. 6A shows hemolytic assay and antimicrobial assay results for series 1 (P-1-8.7K). FIG. 6B shows hemolytic and antimicrobial assay results for series 2 (P-1-5K), and FIG. 6C shows hemolytic and antimicrobial assay results for series 3 (P-1-1.6K). The MIC and $HC_{50}$ values measured for each copolymer are plotted as a function of the mole percent of copolymer hydrophobic group (butyl group). FIG. 6D shows the selectivity indexes ($HC_{50}$/MIC) calculated for the three series of copolymers.

FIGs. 7A and 7B show the results of molecular weigh determinations and the results of hemolytic and antimicrobial assays performed with the C-1 series of copolymers described in Example 4. FIG. 7A shows the molecular weights (number average molecular weights) for the SH30 copolymers of the C-1 series as a function of the mole percent of copolymer hydrophobic group. FIG. 7B shows the results of antimicrobial and hemolytic assays for the SH30 copolymers. The MIC and $HC_{50}$ values measured for each copolymer are plotted as a function of the mole percent of copolymer hydrophobic group.

FIGs. 8A-8D show the results of molecular weigh determinations and the results of hemolytic and antimicrobial assays performed with the C-2 series of copolymers described in Example 4. FIG. 8A shows the molecular weights (number average molecular weights) for the SH10, SH20, and SH30 copolymers of the C-2 series as a function of the mole percent of copolymer hydrophobic group. FIG. 8B shows the antimicrobial and hemolytic assay results for the SH10 copolymers, FIG. 8C shows the assay results for the SH20 copolymers, and FIG. 8D shows the assay results for the SH30 copolymers. The MIC and $HC_{50}$ values measured for each copolymer are plotted as a function of the mole percent of copolymer hydrophobic group.

FIGs. 9A-9D show the results of molecular weigh determinations and the results of hemolytic and antimicrobial assays performed with the C-3 series of copolymers described in Example 4. FIG. 9A shows the molecular weights (number average molecular weights) for the SH10, SH20, and SH30 copolymers of the C-3 series as a function of the mole percent of copolymer hydrophobic group. FIG. 9B shows the antimicrobial and hemolytic assay results for the SH10 copolymers, FIG. 9C shows the assay results for the SH20 copolymers, and FIG. 9D shows the assay results for the SH30 copolymers. The MIC and $HC_{50}$ values measured for each copolymer are plotted as a function of the mole percent of copolymer hydrophobic group.

FIGs. 10A-10D show the results of molecular weigh determinations and the results of hemolytic and antimicrobial assays performed with the C-4 series of copolymers described in Example 4. FIG. 10A shows the molecular weights (number average molecular weights) for the SH10, SH20, and SH30 copolymers of the C-4 series as a function of the mole percent of copolymer hydrophobic group. FIG. 10B shows the antimicrobial and hemolytic assay results for the SH10 copolymers, FIG. 10C shows the assay results for the SH20 copolymers, and FIG. 10D shows the assay results for the SH30 copolymers. The MIC and $HC_{50}$ values measured for each copolymer are plotted as a function of the mole percent of copolymer hydrophobic group.

## DETAILED DESCRIPTION OF THE INVENTION

The present invention provides non-peptidic, amphiphilic, random copolymers and methods of using the copolymers in a number of applications, including their use in pharmaceutical and non-pharmaceutical applications as antimicrobial agents. The present invention also provides compositions comprising the random copolymers and methods for preparing the random copolymers.

The copolymers of the present invention include random copolymers having a monomer unit of Formula **I:**

$$-[CH_2-C(R^1)(B_1)]_n- \qquad \textbf{I}$$

and a monomer unit of Formula **II:**

$$-[CH_2-C(R^2)(D_2)]_m- \qquad \textbf{II}$$

wherein $B_1$, $D_2$, $R^1$, $R^2$, n and m are as defined below.

In some embodiments, the copolymers of the invention are random copolymers of Formula **III**:

$$\mathbf{A\text{-}(B)_{n1}\text{-}(D)_{m1}\text{-}H} \qquad \textbf{III}$$

wherein B is defined as $-[CH_2-C(R^{11})(B_{11})]-$, D is defined as $-[CH_2-C(R^{21})(D_{21})]-$, and A, $B_{11}$, $D_{21}$, $R^{11}$, $R^{21}$, $n_1$ and $m_1$ are as defined below.

The copolymers of the present invention are random copolymers composed of monomer units with hydrophilic side chains and monomer units with hydrophobic side chains, the two types of monomer units randomly distributed along the copolymer backbone. For example, the copolymers of the present invention include random copolymers prepared by the copolymerization of hydrophobic and polar acrylamide or styryl monomer precursors. Thus, in some embodiments of the present invention, the repeating monomer unit of Formula **I**, or **B** of Formula III, contains a hydrophilic cationic group ($B_1$ or $B_{11}$), and the repeating monomer unit of Formula II, or D of Formula III, contains a hydrophobic group ($D_1$ or $D_{11}$).

The random copolymers of the invention can be synthesized using a chain transfer agent to control the degree of polymerization and, accordingly, have average degrees of polymerization and average molecular weights that are lower than those of copolymers synthesized without a chain transfer agent. Copolymers of the present invention typically

have average degrees of polymerization of about four (4) or five (5) to about 50 to 100. Preferred copolymers have average degrees of polymerization ranging from about 4 or 5 to about 20, or from about 5 to about 30.

Use of a chain transfer agent to control the degree of polymerization results in the preparation of the low molecular weight copolymers of the present invention at relatively high yields and avoids the necessity of time-intensive fractionation by column chromatography, which is usually required to obtain low molecular weight polymers in polymerizations performed without a chain transfer agent. The copolymers of the present invention are thus easy to prepare, inexpensive, and suitable for industrial-scale production.

The copolymers of the present invention are amphiphilic and capable of disrupting the integrity of the cell membrane of microorganisms, which results in the inhibition of growth or the death of the microorganisms. As a consequence, the copolymers possess antimicrobial activity, including antibacterial, antifungal, and antiviral activity, and are useful as antimicrobial agents. The copolymers of the invention have a broad range of antimicrobial activity and are effective against a variety of microorganisms, including gram-positive and gram-negative bacterial, fungi, yeast, mycoplasmas, mycobacteria, protozoa, and the like. Moreover, through selection of the molecular weight and/or the hydrophobic side chain, the relative antimicrobial and hemolytic properties of the copolymers of the present invention can be controlled to produce antimicrobial copolymers that are non-toxic to mammals.

The copolymers of the present invention are useful as antimicrobial agents in a number of applications. For example, copolymers of the present invention can be used therapeutically to treat microbial infections in animals, including humans and non-human vertebrates such as wild, domestic and farm animals. The microbial infection in an animal is treated by administering to the animal an effective amount of a pharmaceutical composition of a copolymer of the present invention. Because the copolymers have a broad range of antimicrobial activity, they are useful in treating a variety of infections in an animal. The copolymer compositions can be administered systemically or topically and can be administered to any body site or tissue. For example, the copolymers of the present invention can be used to treat oral diseases, such as periodontal disease, or used as a preventative for such oral diseases. For this application, the copolymers can be incorporated into a number of products, including, e.g., but not limited to, mouthwash, chewing gum, toothpaste or gel. Alternatively, for this application, the copolymers can be incorporated into a device, polymer or substratum that can be implanted in the gums for slow release or direct killing of microorganisms.

The amphiphilicity of the random copolymers of the present invention form the basis for another therapeutic use, the use of the copolymers as antidotes for hemorrhagic complications associated with heparin therapy. Thus, the copolymers of the present invention can be used in a method of providing an antidote to heparin overdose in an animal by administering to the animal an effective amount of a pharmaceutical composition of the copolymer.

The random copolymers of the present invention also can be used as disinfectants or as preservatives. The copolymers of the present invention can thus be used in a method of killing or inhibiting the growth of a microorganism by contacting the microorganism with an effective amount of the copolymer. For example, the copolymers of the present invention can be used as disinfectants or preservatives in, for example, cosmetics, soaps, lotions, handwashes, paints, cleansers, and polishers, and the like, or in, for example, foodstuffs, food containers, and food-handling implements. The copolymers are administered for these purposes as a solution, dispersion, or suspension. The copolymers of the present invention can also be incorporated into plastics that can be molded or shaped into articles, or attached or immobilized on a surface, to provide a surface-mediated microbicide that kills or inhibits the growth of microorganisms in contact with the surface. Moreover, by selecting the molecular weight and/or hydrophobic group of the copolymers of the present invention, the physical properties of the copolymers can be optimized for specific applications. For example, by altering the molecular weight and hydrophobic groups of the copolymers, the viscosity of solutions of the copolymers can be controlled, allowing the copolymers to also act as thickeners in those applications in which the copolymers are incorporated into, e.g., foodstuffs or paints.

The present invention discloses amphiphilic random copolymers. Polymers are generally defined as synthetic compounds assembled from monomer subunits and are polydisperse in molecular weight Polymers are most commonly prepared by one-pot synthetic procedures. The term "polymer," as used herein, refers to a macromolecule comprising a plurality of repeating monomers or monomer units. The term "polymer" can include homopolymers, which are formed from a single type of monomer, and copolymers, which are formed from two or more different monomers. The term "copolymer" includes polymers in which the monomers are distributed randomly (random copolymer), in alternating fashion (alternating copolymers), or in blocks (block copolymer). The copolymers of the present invention are random copolymers. The term "random copolymer," as used herein, refers to copolymers in which the monomers are distributed randomly.

The random copolymers of the present invention have a monomer unit of Formula **I:**

$$-[CH_2-C(R^1)(B_1]_n- \qquad \mathbf{I}$$

wherein:

$R^1$ is hydrogen or $C_{1-4}$ alkyl;
$B_1$ is $-X_1-Y_1-Z_1$, wherein:

$X_1$ is carbonyl (-C(=O)-) or optionally substituted $C_{1-6}$ alkylene; or $X_1$ is absent;
$Y_1$ is O, NH, or optionally substituted $C_{1-6}$ alkylene; or $Y_1$ is absent;
$Z_1$ is $-Z_{1A}-Z_{1B}$, wherein:
$Z_{1A}$ is alkylene, arylene, or heteroarylene, any of which is optionally substituted; or $Z_{1A}$ is absent;
$Z_{1B}$ is -guanidino, -amidino, $-N(R^3)(R^4)$ or $-N^+(R^3)(R^4)(R^5)$, wherein $R^3$, $R^4$, and $R^5$ are independently hydrogen, alkyl, aminoalkyl, aryl, heteroaryl, heterocyclic, or aralkyl; or
$Z_1$ is pyridinium

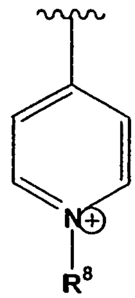
,

or phosphonium

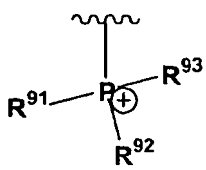
,

wherein $R^8$, $R^{91}$, $R^{92}$, and $R^{93}$ are independently hydrogen or alkyl;
n is 1-m, wherein m is as defined below; and a monomer unit of Formula **II**:

$$-[CH_2-C(R^2)(D_2)]_m- \qquad \textbf{II}$$

or an acceptable salt or solvate thereof,
wherein:

$R^2$ is hydrogen or $C_{1-4}$ alkyl;
$D_2$ is $-X_2-Y_2-Z_2$, wherein

$X_2$ is carbonyl (-C(=O)-) or optionally substituted $C_{1-6}$ alkylene, or $X_2$ is absent;
$Y_2$ is O, NH, or optionally substituted $C_{1-6}$ alkylene; or $Y_2$ is absent;
$Z_2$ is alkyl, cycloalkyl, alkoxy, aryl, or aralkyl, any of which is optionally substituted; and
m is about 0.1 to about 0.9;

wherein the copolymer has a degree of polymerization of about 5 to about 50.

A chain transfer agent A can be used to control the degree of polymerization of the copolymer. Any conventional chain transfer agent A can be used in the synthesis of the copolymers of the present invention. However, preferred chain transfer agents A are thiol compounds, such as, for example, alkylthio or arylthiol compounds. For example, preferred copolymers of the present invention are synthesized using a chain transfer agent A selected from the group consisting of

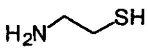 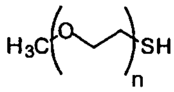 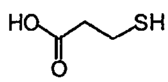 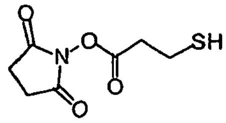 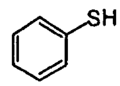 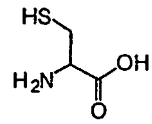

n=3-120

Cys

and an alkoxycarbonylalkylthiol. Alkoxycarbonylalkylthiols, such as, for example, methyl 3-mercaptopropionate and ethyl 3-mercaptopropionate, are especially preferred as chain transfer agents.

In some embodiments of the invention, preferred copolymers are those wherein the repeating unit of Formula I is cationic. Thus, in some embodiments, preferred copolymers are those wherein $Z_1$ is $-Z_{1A}-Z_{1B}$, wherein $Z_{1A}$ is $C_{1-6}$ alkylene, e.g., ethylene, propylene, or butylene, optionally substituted with $C_{1-4}$ alkyl or aryl; and $Z_{1B}$ is $-N(R^3)(R^4)$ or $-N^+(R^3)(R^4)(R^5)$, wherein $R^3$, $R^4$, and $R^5$ are independently hydrogen, $C_{1-4}$ alkyl, amino($C_{1-4}$) alkyl, $C_{1-6}$ aryl, or $C_{1-6}$ ar($C_{1-4}$)alkyl. Especially preferred copolymers are those wherein $Z_1$ is $-Z_{1A}-Z_{1B}$, wherein $Z_{1A}$ is $C_{1-4}$ alkylene optionally substituted with, methyl or ethyl; and $Z_{1B}$ is $-N^+(R^3)(R^4)(R^5)$, wherein $R^3$, $R^4$, and $R^5$ are independently hydrogen or methyl.

In some embodiments, preferred values of $Z_{1A}$ are optionally substituted $C_{1-10}$ alkylene, *i.e.*, optionally substituted $-(CH_2)_p-$, wherein p is 1 to 10. Thus, in some embodiments of the present invention, preferred values of $Z_1$ are $-(CH_2)_p$-guanidino, $-(CH_2)_p$-amidino, $-(CH_2)_pN(R^3)(R^4)$ or $-(CH_2)_pN^+(R^3)(R^4)(R^5)$, wherein $R^3$, $R^4$, and $R^5$ are independently hydrogen, alkyl, aminoalkyl, aryl, heteroaryl, heterocyclic, or aralkyl; p is 0 to 10; and $-(CH_2)_p-$ is optionally substituted. Especially preferred copolymers in these embodiments are those wherein $Z_1$ is $-(CH_2)_pN(R^3)(R^4)$ or $-(CH_2)_pN^+(R^3)(R^4)(R^5)$, wherein $R^3$, $R^4$, and $R^5$ are independently hydrogen, $C_{1-4}$ alkyl, amino($C_{1-4}$) alkyl, $C_{1-6}$ aryl, or $C_{1-6}$ ar($C_{1-4}$)alkyl; and p is 0 to 10; and $-(CH_2)_p-$ is optionally substituted with $C_{1-4}$ alkyl, amino, hydroxy, or halo, including, for example, chloro or bromo. Most preferred are those copolymers wherein $Z_1$ is $-(CH_2)_pN^+(R^3)(R^4)(R^5)$, p is 1-4, $R^3$, $R^4$, and $R^5$ are each hydrogen, and $-(CH_2)_p-$ is optionally substituted with methyl, ethyl, or halo.

In some embodiments, preferred copolymers are those wherein $Z_1$ is pyridinium

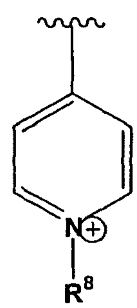

or phosphonium

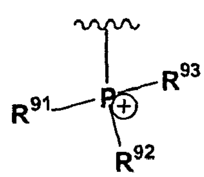,

wherein $R^8$, $R^{91}$, $R^{92}$, and $R^{93}$ are independently hydrogen or alkyl. In these embodiments, especially preferred values of $R^8$, $R^{91}$, $R^{92}$, and $R^{93}$ are hydrogen and $C_{1-4}$ alkyl.

Preferred values of $X_1$ in the copolymers of the present invention are carbonyl and optionally substituted $C_{1-4}$ alkylene. An especially preferred value of $X_1$ is carbonyl. In some embodiments, a preferred value of $X_1$ is $-CH_2-$. In other embodiments, $X_1$ is absent (*i.e.*, $X_1$ is a covalent bond).

Preferred values of $Y_1$ are O and NH, with O being especially preferred. In some embodiments, a preferred value of $Y_1$ is $-CH_2-$. In other embodiments, $Y_1$ is absent (*i.e.*, $Y_1$ is a covalent bond).

In some embodiments of the invention, preferred copolymers are those wherein the repeating unit of Formula II is hydrophobic. Thus, preferred copolymers of the present invention are those copolymers having a repeating unit of Formula II wherein $X_2$ is carbonyl or optionally substituted $C_{1-4}$ alkylene. Especially preferred are those copolymers wherein $X_2$ is carbonyl. In some embodiments, a preferred value of $X_2$ is $-CH_2-$. In other embodiments, $X_2$ is absent (*i.e.*, $X_1$ is a covalent bond).

Preferred values of $Y_2$ are O and NH. Especially preferred are those copolymers wherein $Y_2$ is O. In some embodiments, a preferred value of $Y_2$ is $-CH_2-$. In other embodiments, $Y_2$ is absent (*i.e.*, $Y_2$ is a covalent bond).

Preferred values of $Z_2$ are $C_{1-6}$ alkyl, $C_{1-6}$ aryl, or $C_{1-6}$ ar($C_{1-4}$)alkyl. Especially preferred values of $Z_2$ include methyl, ethyl, n-butyl, isobutyl, hexyl, and benzyl.

Thus, in some embodiments of the present invention, preferred copolymers include those copolymers having a repeating monomer unit of Formula I wherein:

$X_1$ and $X_2$ are carbonyl;
$Y_1$ and $Y_2$ are O;
$Z_1$ is $-Z_{1A}-Z_{1B}$, wherein $Z_{1A}$ is optionally substituted $C_{1-6}$ alkylene, and $Z_{1B}$ is $-N(R^3)(R^4)$ or $-N^+(R^3)(R^4)(R^5)$, wherein $R^3$, $R^4$, and $R^5$ are independently hydrogen, $C_{1-4}$ alkyl, amino($C_{1-4}$) alkyl, $C_{1-6}$ aryl, or $C_{1-6}$ ar($C_{1-4}$)alkyl;
$Z_2$ is $C_{1-6}$ alkyl, $C_{1-6}$ aryl, or $C_{1-6}$ ar($C_{1-4}$)alkyl; and
$R^1$ and $R^2$ are independently hydrogen or methyl.

In other embodiments of the present invention, preferred copolymers include those copolymers having a repeating monomer unit of Formula I wherein:

$X_1$ and $X_2$ are carbonyl;
$Y_1$ and $Y_2$ are O;
$Z_1$ is $-(CH_2)_pN(R^3)(R^4)$ or $-(CH_2)_pN^+(R^3)(R^4)(R^5)$, wherein $R^3$, $R^4$, and $R^5$ are independently hydrogen, $C_{1-4}$ alkyl, amino($C_{1-4}$) alkyl, $C_{1-6}$ aryl, or $C_{1-6}$ ar($C_{1-4}$)alkyl; and p is 0 to 10; and $-(CH_2)_p-$ is optionally substituted;
$Z_2$ is $C_{1-6}$ alkyl, $C_{1-6}$ aryl, or $C_{1-6}$ ar($C_{1-4}$)alkyl; and
$R^1$ and $R^2$ are independently hydrogen or methyl.

Preferred copolymers of the present invention are also those wherein the average degree of polymerization ("DP") is about 4 to about 50, about 4 to about 30, about 5 to about 25, about 5 to about 20, about 5 to about 15, about 5 to about 10, about 5 to about 12, about 5 to about 10, or about 6 to about 8. In some aspects of the invention, preferred copolymers are those wherein the DP is about 4 to about 15, or about 4 to about 10. Especially preferred are those copolymers wherein DP is about 4 to about 10, or about 6 to about 8.

In some embodiments of the present invention, preferred copolymers are those wherein DP is about 5 to about 50, about 5 to about 30, about 5 to about 20, about 6 to about 20, about 6 to about 15, about 6 to about 12, about 6 to about 10, or about 6 to about 8. Especially preferred are those copolymers wherein DP is about 6 to about 10, or about 6 to about 8

Preferred copolymers of the present invention are those wherein n is 1-m, and m is about 0.1 to about 0.9, about 0.1 to about 0.6, about 0.35 to about 0.60, about 0.35 to about 0.55, about 0.50 to about 0.60, about 0.45 to about 0.55, or about 0.35 to about 0.45.

In some embodiments, the copolymers of the present invention are random copolymers of Formula III:

$$\mathbf{A\text{-}(B)_{n1}\text{-}(D)_{m1}\text{-}H} \qquad \mathbf{III}$$

or an acceptable salt or solvate thereof, wherein:

A is the residue of a chain transfer agent;
B is $-[CH_2-C(R^{11})(B_{11})]-$, wherein $B_{11}$ is $-X_{11}-Y_{11}-Z_{11}$, and
$X_{11}$ is carbonyl (-C(=O)-) or optionally substituted $C_{1-6}$ alkylene; or $X_{11}$ is absent;
$Y_{11}$ is O, NH, or optionally substituted $C_{1-6}$ alkylene; or $Y_{11}$ is absent;
$Z_{11}$ is $-Z_{11A}-Z_{11B}$ , wherein:

$Z_{11A}$ is alkylene, arylene, or heteroarylene, any of which is optionally substituted; or $Z_{11A}$ is absent;
$Z_{11B}$ is -guanidino, -amidino, $-N(R^3)(R^4)$ or $-N^+(R^3)(R^4)(R^5)$, wherein $R^3$, $R^4$, and $R^5$ are independently hydrogen, alkyl, aminoalkyl, aryl, heteroaryl, heterocyclic, or aralkyl; or
$Z_{11}$ is pyridinium

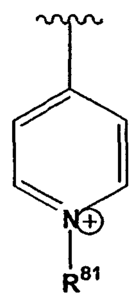
,

or phosphonium

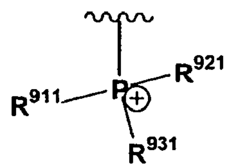

wherein $R^{81}$, $R^{911}$, $R^{921}$, and $R^{931}$ are independently hydrogen or alkyl;
D is $-[CH_2-C(R^{21})(D_{21})]-$, wherein $D_{21}$ is $-X_{21}-Y_{21}-Z_{21}$, and
$X_{21}$ is carbonyl (-C(=O)-) or optionally substituted $C_{1-6}$ alkylene; or $X_{21}$ is absent;
$Y_{21}$ is O, NH, or optionally substituted $C_{1-6}$ alkylene, or $Y_{21}$ is absent;
$Z_{21}$ is alkyl, cycloalkyl, alkoxy, aryl, or aralkyl, any of which is optionally substituted;
$R^{11}$ and $R^{21}$ are independently hydrogen or $C_{1-4}$ alkyl;
$m_1$, the mole fraction of D monomer, is about 0.1 to about 0.9; and
$n_1$, the mole fraction of B monomer, is $1-m_1$;

wherein the copolymer is a random copolymer of B and D monomers, and wherein the copolymer has a degree of polymerization of about 5 to about 50.

In preferred copolymers of Formula III, A is a residue of a thiol chain transfer agent, including, but not limited to, a residue of any one of the following thiol chain transfer agents:

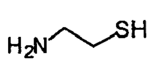 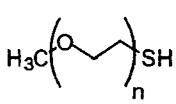 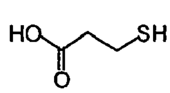 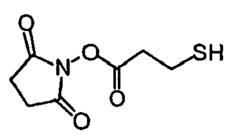 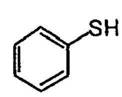 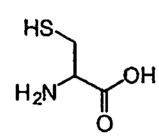

n=3-120

Cys

or an alkoxycarbonylalkylthiol, such as methyl 3-mercaptopropionate and ethyl 3-mercaptopropionate. Especially preferred is the chain transfer agent methyl 3-mercaptopropionate.

For example, in preferred copolymers of Formula III, A is alkoxycarbonylalkylthio. In especially preferred copolymers of Formula III, A is $C_{1-4}$ alkoxycarbonyl($C_{1-4}$)alkylthio. An especially preferred value of A in Formula III is methyloxycarbonylethylthio, a residue derived from the chain transfer agent methyl 3-mercaptopropionate.

Preferred copolymers of Formula III are those wherein B is a hydrophilic residue, and D is a hydrophobic residue. In some embodiments of the invention, preferred copolymers of Formula III are those wherein B is a cationic residue. Accordingly, preferred values of $X_{11}$ and $Y_{11}$ are the preferred values listed for $X_1$ and $Y_1$, respectively, of Formula I, as described above. Preferred values of $Z_{11}$, $R^{11}$, $R^{31}$, $R^{41}$, $R^{51}$, $R^{81}$, $R^{911}$, $R^{921}$, $R^{931}$, and $n_1$ are the preferred values listed for $Z_1$, $R^1$, $R^3$, $R^4$, $R^5$, $R^8$, $R^{91}$, $R^{92}$, $R^{93}$, and n, respectively, of Formula I, as described above.

Preferred copolymers of Formula III are also those wherein D is a hydrophobic residue. Accordingly, preferred values of $R^{21}$, $X_{21}$, $Y_{21}$, and $m_1$ are the preferred values listed for $R^2$, $X_2$, $Y_2$, and m, respectively, of Formula II.

Preferred copolymers of Formula III are those wherein the average degree of polymerization (DP) is about 4

to about 50, about 5 to about 50, about 4 to about 30, about 5 to about 30, about 5 to about 25, about 5 to about 20, about 6 to about 20, about 5 to about 15, about 6 to about 15, about 5 to about 12, about 6 to about 12, about 5 to about 10, about 6 to about 10, or about 6 to about 8. Especially preferred are those copolymers of Formula II wherein DP is about 4 to about 10, about 5 to about 10, about 6 to about 10, or about 6 to about 8.

In some embodiments of the present invention, preferred copolymers are those wherein the copolymer is a random copolymer of Formula IIIa:

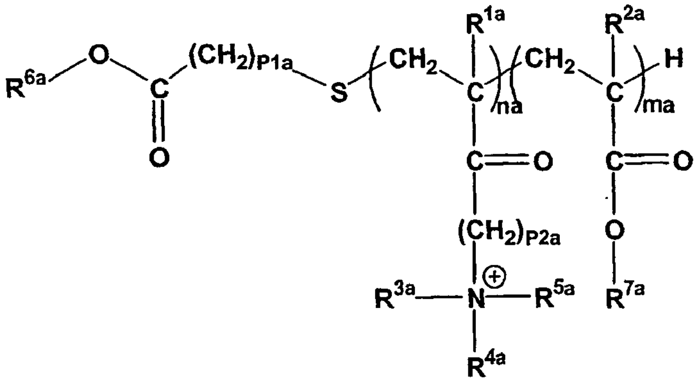

IIIa

or an acceptable salt or solvate thereof, wherein:

$R^{1a}$ and $R^{2a}$ are independently hydrogen or methyl;
$R^{3a}$, $R^{4a}$, and $R^{5a}$ are independently hydrogen or $C_{1-4}$ alkyl;
$R^{6a}$ is $C_{1-4}$ alkyl;
$R^{7a}$ is $C_{1-10}$ alkyl, $C_{1-6}$ aryl, or $C_{1-6}$ ar($C_{1-4}$)alkyl;
$p_{1a}$ is 1 to 4;
$p_{2a}$ is 1 to 6; and
$m_a$ is about 0.35 to about 0.55; and $n_a$ is 1-$m_a$;

wherein the copolymer has a degree of polymerization of about 5 to about 25.

For example, in some embodiments of the present invention, preferred copolymers are those of Formula IIIa wherein:

$R^{1a}$ and $R^{2a}$ are independently methyl;
$R^{3a}$, $R^{4a}$, and $R^{5a}$ are independently hydrogen or methyl;
$R^{6a}$ is methyl or ethyl;
$R^{7a}$ is $C_{1-4}$ alkyl, e.g., methyl, ethyl, propyl, or butyl;
$p_{1a}$ is 1 or 2;
$p_{2a}$ is 1, 2 or 3;
$m_a$ is about 0.35 to about 0.55; and $n_a$ is 1-$m_a$; and

the degree of polymerization is about 5 to about 25, about 5 to about 10, about 5 to about 15, or about 5 to about 10.

Thus, in some embodiments of the present invention, preferred copolymers are those wherein the copolymer is a random copolymer of Formula IIIb:

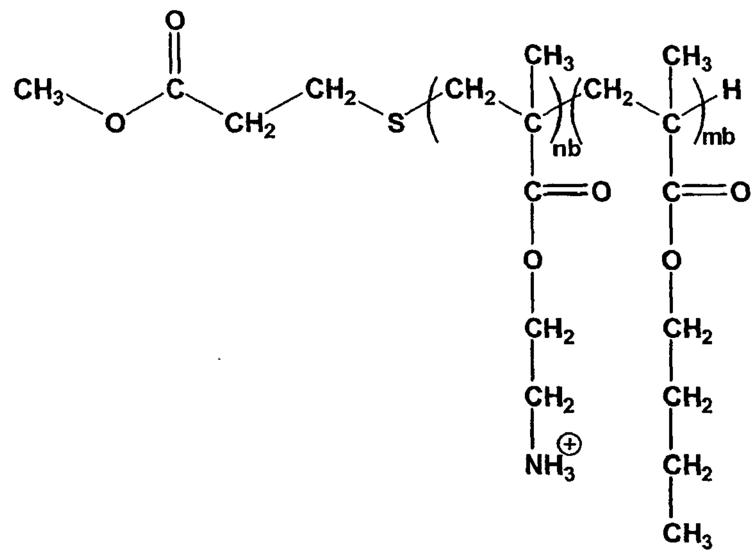

IIIb

or an acceptable salt or solvate thereof, wherein:

$m_b$ is about 0.45 to about 0.55; $n_b$ is 1-$m_b$; and
the degree of polymerization is about 5 to about 15, about 5 to about 10, or about 6 to about 8.

In yet other embodiments of the present invention, preferred copolymers are those wherein the copolymer is a random copolymer of Formula IIIc:

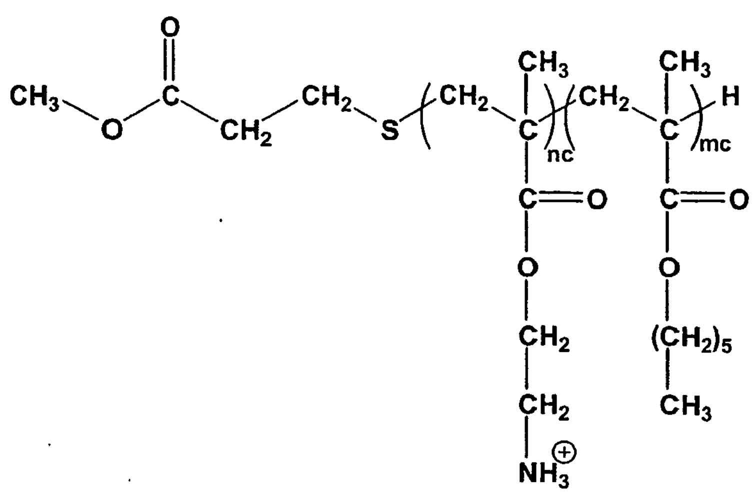

IIIc

or an acceptable salt or solvate thereof, wherein:

$m_c$ is about 0.35 to about 0.45; $n_c$ is 1-$m_c$; and
the degree of polymerization is about 5 to about 15, about 5 to about 10, or about 6 to about 8.

In some embodiments of the present invention, preferred copolymers are those wherein the copolymer is a random copolymer of Formula IIId:

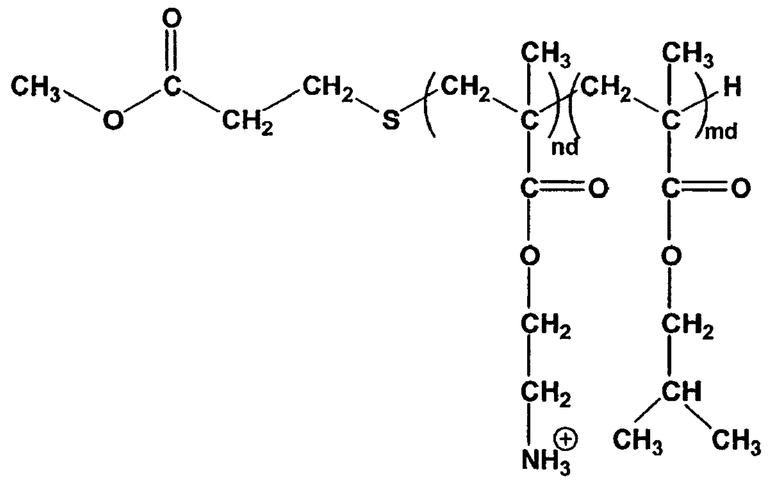

IIId

or an acceptable salt or solvate thereof, wherein:

$m_d$ is about 0.45 to about 0.55; $n_d$ is $1-m_d$; and
the degree of polymerization is about 5 to about 15, about 5 to about 10, or about 6 to about 8.

In other embodiments of the present invention, preferred copolymers are those wherein the copolymer is a random copolymer of Formula IIIe:

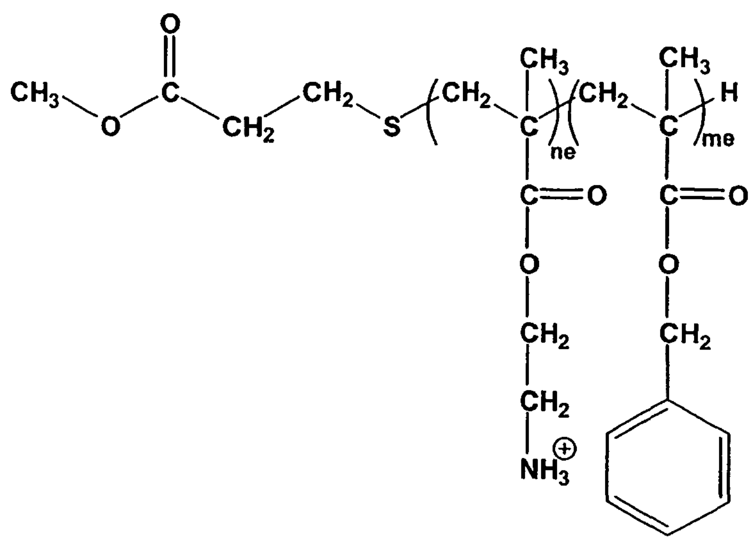

IIIe

or an acceptable salt or solvate thereof, wherein:

$m_e$ is about 0.50 to about 0.60; $n_e$ is $1-m_e$; and
the degree of polymerization is about 5 to about 15, about 5 to about 10, or about 6 to about 8.

The copolymers of the present invention have about 4 monomer units to about 50 to 100 monomer units, with average molecular weights that range from about 500 Daltons to about 10,000 to 20,000 Daltons, or about 1,000 Daltons to about 10,000 to 20,000 Daltons. Preferred copolymers are those having about 4 to about 30 monomer units, about 5 to about 30 monomer units, about 4 to about 20 monomer units, or about 5 to about 20 monomer units, with average molecular weights that range from about 500 Daltons to about 10,000 Daltons, about 1,000 Daltons to about 10,000 Daltons, about 1,000 Daltons to about 5,000 Daltons, or about 1,000 Daltons to about 4,000 Daltons. Especially preferred copolymers are those having about 5 to about 10 monomer units, or about 6 to about 8 monomer units, with average

molecular weights that range from about 500 Daltons to about 2,000 Daltons, or about 1,000 Daltons to about 2,000 Daltons.

The term "copolymer backbone" or "backbone" as used herein refers to that portion of the copolymer which is a continuous chain comprising the bonds formed between monomers upon polymerization. The composition of the copolymer backbone can be described in terms of the identity of the monomers from which it is formed without regard to the composition of branches, or side chains, of the copolymer backbone.

The term "copolymer side chain" or "side chain" refers to portions of the monomer which, following polymerization, forms an extension of the copolymer backbone.

The term "amphiphilic" as used herein describes a structure having discrete hydrophobic and hydrophilic regions. An amphiphilic copolymer requires the presence of both hydrophobic and hydrophilic elements along the copolymer backbone.

The term "microorganism" as used herein includes bacteria, algae, fungi, yeast, mycoplasmas, mycobacteria, parasites and protozoa.

The term "antimicrobial," "microbiocidal," or "biocidal" as used herein means that the materials inhibit, prevent, or destroy the growth or proliferation of microorganisms. This activity can be either bacteriocidal or bacteriostatic. The term "bactoriocidal" as used herein means the killing of microorganisms. The term "bacteriostatic" as used herein refers to inhibiting the growth of microorganisms which can be reversible under certain conditions.

The term "alkyl" as used herein by itself or as part of another group refers to both straight and branched-chain aliphatic hydrocarbon radicals from 1 to 12 carbons, such as methyl, ethyl, propyl, isopropyl, butyl, t-butyl, isobutyl, pentyl, hexyl, isohexyl, heptyl, 4,4-dimethylpentyl, octyl, 2,2,4-trimethylpentyl, nonyl, decyl, undecyl, dodecyl.

The term "alkylene" as used herein refers to straight chain or branched divalent aliphatic hydrocarbon radicals from 1 to 20 carbon atoms in length, or, more preferably, from 1 to 10 carbon atoms, or from 1 to 6 carbon atoms in length. Examples of alkylene radicals include, but are not limited to, methylene ($-CH_2-$), ethylene ($-CH_2CH_2-$), propylene isomers (e.g., $-CH_2CH_2CH_2-$ and $-CH(CH_3)CH_2-$), and the like.

The term "alkoxy" as used herein refers to a straight or branched chain aliphatic hydrocarbon radicals of 1 to 20 carbon atoms, unless the chain length is limited thereto, bonded to an oxygen atom, including, but not limited to, methoxy, ethoxy, n-propoxy, isopropoxy, and the like. Preferably, the alkoxy chain is 1 to 10 carbon atoms in length, more preferably 1 to 8 carbon atoms in length, and even more preferred 1 to 6 carbon atoms in length.

The term "aryl" as used herein by itself or as part of another group refers to monocyclic or bicyclic aromatic groups containing from 6 to 12 carbons in the ring portion, preferably 6-10 carbons in the ring portion, such as the carbocyclic groups phenyl, naphthyl and tetrahydronaphthyl.

The term "arylene" as used herein refers to divalent aryl groups (e.g., monocyclic or bicyclic aromatic groups) containing from 6 to 12 carbons in the ring portion, preferably 6-10 carbons in the ring portion, that are derived from removal of a hydrogen atom from two ring carbon atoms. Examples of arylene groups include, but are not limited to o-phenylene, naphthylene, benzene-1,2-diyl and the like.

The term "cycloalkyl" as used herein by itself or as part of another group refers to cycloalkyl groups containing 3 to 9 carbon atoms, more preferably, 3 to 8 carbon atoms. Typical examples are cyclopropyl, cyclobutyl, cyclopentyl, cyclohexyl, cycloheptyl, cyclooctyl and cyclononyl.

The term "halogen" or "halo" as used herein by itself or as part of another group refers to chlorine, bromine, fluorine or iodine.

The term "heteroaryl" as used herein refers to groups having 5 to 14 ring atoms; 6, 10 or 14 $7\pi$-electrons shared in a cyclic array; and containing carbon atoms and 1, 2 or 3 oxygen, nitrogen or sulfur heteroatoms. Examples of heteroaryl groups include thienyl, imadizolyl, oxadiazolyl, isoxazolyl, triazolyl, pyridyl, pyrimidinyl, pyridazinyl, furyl, pyranyl, thianthrenyl, pyrazolyl, pyrazinyl, indolizinyl, isoindolyl, isobenzofuranyl, benzoxazolyl, xanthenyl, 2H-pyrrolyl, pyrrolyl, 3H-indolyl, indolyl, indazolyl, purinyl, 4H-quinolizinyl, isoquinolyl, quinolyl, phthalazinyl, naphthyridinyl, quinazolinyl, phenanthridinyl, acridinyl, perimidinyl, phenanthrolinyl, phenazinyl, isothiazolyl, phenothiazinyl, isoxazolyl, furazanyl, and phenoxazinyl groups. Especially preferred heteroaryl groups include 1,2,3-triazole, 1,2,4-triazole, 5-amino 1,2,4-triazole, imidazole, oxazole, isoxazole, 1,2,3-oxadiazole, 1,2,4-oxadiazole, 3-amino-1,2,4-oxadiazole, 1,2,5-oxadiazole, 1,3,4-oxadiazole, pyridine, and 2-aminopyridine. The term "heteroarylene" as used herein refers to divalent heteroaryl groups that are derived from removal of a hydrogen atom from two ring atoms.

The term "heterocycle," "heterocyclic," or "heterocyclic ring", as used herein except where noted, represents a stable 5- to 7-membered mono- or bicyclic or stable 7- to 10-membered bicyclic heterocyclic ring system any ring of which may be saturated or unsaturated, and which consists of carbon atoms and from one to three heteroatoms selected from the group consisting of N, O and S, and wherein the nitrogen and sulfur heteroatoms may optionally be oxidized, and the nitrogen heteroatom may optionally be quaternized, and including any bicyclic group in which any of the above-defined heterocyclic rings is fused to a benzene ring. Especially useful are rings containing one oxygen or sulfur, one to three nitrogen atoms, or one oxygen or sulfur combined with one or two nitrogen atoms. The heterocyclic ring may be attached at any heteroatom or carbon atom which results in the creation of a stable structure. Examples of such

heterocyclic groups include piperidinyl, piperazinyl, 2-oxopiperazinyl, 2-oxopiperidinyl, 2-oxopyrrolodinyl, 2-oxoazepinyl, azepinyl, pyrrolyl, 4-piperidonyl, pyrrolidinyl, pyrazolyl, pyrazolidinyl, imidazolyl, imidazolinyl, imidazolidinyl, pyridyl, pyrazinyl, pyrimidinyl, pyridazinyl, oxazolyl, oxazolidinyl, isoxazolyl, isoxazolidinyl, morpholinyl, thiazolyl, thiazolidinyl, isothiazolyl, quinuclidinyl, isothiazolidinyl, indolyl, quinolinyl, isoquinolinyl, benzimidazolyl, thiadiazoyl, benzopyranyl, benzothiazolyl, benzoxazolyl, furyl, tetrahydrofuryl, tetrahydropyranyl, thienyl, benzothienyl, thiamorpholinyl, thiamorpholinyl sulfoxide, thiamorpholinyl sulfone, and oxadiazolyl. Morpholino is the same as morpholinyl.

The term "alkylamino" as used herein by itself or as part of another group refers to an amino group which is substituted with one alkyl group having from 1 to 6 carbon atoms. The term "dialkylamino" as used herein by itself or as part of an other group refers to an amino group which is substituted with two alkyl groups, each having from 1 to 6 carbon atoms.

The term "alkylthio" as used herein by itself or as part of an other group refers to an thio group which is substituted with one alkyl group having from 1 to 10 carbon atoms, or, preferably, from 1 to 6 carbon atoms.

Generally and unless defined otherwise, the phrase "optionally substituted" used herein refers to a group or groups being optionally substituted with one or more substituents independently selected from the group consisting of amino, hydroxy, nitro, halogen, cyano, thiol, $C_{1-6}$ alkyl, $C_{2-6}$ alkenyl, $C_{2-6}$ alkynyl, $C_{3-6}$ cycloalkyl, and $C_{1-6}$ aryl.

The terms "treat," "treated," or "treating" as used herein refers to both therapeutic treatment and prophylactic or preventative measures, wherein the object is to prevent or slow down (lessen) an undesired physiological condition, disorder or disease, or to obtain beneficial or desired clinical results. For the purposes of this invention, beneficial or desired clinical results include, but are not limited to, alleviation of symptoms; diminishment of the extent of the condition, disorder or disease; stabilization (*i.e.*, not worsening)of the state of the condition, disorder or disease; delay in onset or slowing of the progression of the condition, disorder or disease; amelioration of the condition, disorder or disease state; and remission (whether partial or total), whether detectable or undetectable, or enhancement or improvement of the condition, disorder or disease. Treatment includes eliciting a clinically significant response without excessive levels of side effects. Treatment also includes prolonging survival as compared to expected survival if not receiving treatment.

The term "animal" as used herein includes, but is not limited to, humans and non-human vertebrates such as wild, domestic and farm animals.

In some aspects of the invention, the copolymers of the present invention are derivatives referred to as prodrugs. The expression "prodrug" denotes a derivative of a known direct acting drug, which derivative has enhanced delivery characteristics and therapeutic value as compared to the drug, and is transformed into the active drug by an enzymatic or chemical process.

When any variable occurs more than one time in any constituent or in any of the copolymers recited for any of the Formulae above (for example, Formulae I, II, III, IIIa, IIIb, IIIc, IIId, or IIIe), its definition on each occurrence is independent of its definition at every other occurrence. Also, combinations of substituents and/or variables are permissible only if such combinations result in stable compounds.

It is understood that the present invention encompasses the use of stereoisomers, diastereomers and optical isomers of the copolymers of the present invention, as well as mixtures thereof, for treating microbial infections, killing or inhibiting the growth of a microorganism, and providing an antidote to low molecular weight heparin overdose in an animal. Additionally, it is understood that stereoisomers, diastereomers and optical isomers of the copolymers of the present invention, and mixtures thereof, are within the scope of the invention. By way of non-limiting example, the mixture may be a racemate or the mixture may comprise unequal proportions of one particular stereoisomer over the other. Additionally, the copolymers of the present invention may be provided as a substantially pure stereoisomers, diastereomers and optical isomers.

In another aspect of the invention, the copolymers of the present invention, in particular, copolymers with cationic side chains, can be provided in the form of an acceptable salt (*i.e.*, a pharmaceutically acceptable salt) for treating microbial infections, killing or inhibiting the growth of a microorganism, and providing an antidote to low molecular weight heparin overdose in an animal. Copolymer salts can be provided for pharmaceutical use, or as an intermediate in preparing the pharmaceutically desired form of the copolymer. One copolymer salt that can be considered to be acceptable is the hydrochloride acid addition salt. For example, chloride ion can be present as a counter ion for copolymers having cationic side chains. Hydrochloride acid addition salts are often acceptable salts when the pharmaceutically active agent has an amine group that can be protonated. Since a copolymer of the invention may be polyionic, such as a polyamine, the acceptable copolymer salt may be provided in the form of a poly(amine hydrochloride). Other acceptable copolymer salts include conjugate bases of pharmaceutically acceptable acids, such as, for example, trifluoroacetate, the conjugate base of the pharmaceutically acceptable acid trifluoroacetic acid (TFA).

The copolymers of the present invention have been shown to possess antimicrobial activity. Thus, the copolymers of the present invention can be used as antimicrobial agents and, for example, can be used in a method of treating microbial infections in an animal.

Thus, the invention is directed to a method of treating a microbial infection in an animal in need thereof, by administering to the animal a copolymer of the present invention.

For example, in some aspects, the invention is directed to a method of treating a microbial infection in an animal in need thereof, the method comprising administering to the animal an effective amount of a pharmaceutical composition comprising a random copolymer of Formula III, as defined above, and a pharmaceutically acceptable carrier or diluent, or an effective amount of a pharmaceutical composition comprising a random copolymer having a monomer unit of Formula I and a monomer unit of Formula II, as defined above.

The copolymers of the present invention can be used to treat a microbial infection caused by any type of microorganism, including, but not limited to, bacteria, algae, fungi, yeast, mycoplasmas, mycobacterial, parasites and protozoa. The copolymers of the present invention are therefore effective in treating bacterial infections, fungal infections, viral infections, yeast infections, mycoplasmid infections, mycobacterial infections, or protozoal infections.

The copolymers of the present invention have also been shown to possess antiviral activity and can be used as antiviral agents.

Thus, in some aspects, the invention is directed to a method of treating a viral infection in an animal in need thereof, the method comprising administering to the animal an effective amount of a pharmaceutical composition comprising a random copolymer of Formula III, as defined above, and a pharmaceutically acceptable carrier or diluent, or an effective amount of a pharmaceutical composition comprising a random copolymer having a monomer unit of Formula I and a monomer unit of Formula II, as defined above.

The copolymers of the present invention can also be used in methods of treating fungal infections.

Immunocompromised individuals are at serious risk for developing systemic fungal infections and the high incidence of cancer and AIDS underscores the need for developing effective and safe antifungal therapies. Many of the existing antifungal drugs act on molecular targets involved in cell wall synthesis (Debono, M., and Gordee, R.S., Ann. Rev. Microbiol. 48:471-497 (1994)). However, many of these targets are also found in mammalian cells which can lead to unwanted side-effects, and current therapies are associated with serious clinical complications including hepatic and kidney toxicities. Furthermore, as with bacterial infections, drug-resistant fungi are emerging at an alarming rate (DeLucca, A.J., and Walsh, T.J., Antimicob. Agents Chemother. 43:1-11 (1999)). Therefore, there is a strong need for the development of novel approaches for systemic and topical agents that can rapidly, effectively and safely control fungal infections while minimizing the potential for the development of resistance to their mechanism of action.

The copolymers of the present invention have also been shown to possess antifungal activity and thus can be used as antifungal agents, for example, in a method of treating fungal infections in an animal.

Thus, in some aspects, the invention is directed to a method of treating a fungal infection in an animal in need thereof, the method comprising administering to the animal an effective amount of a pharmaceutical composition comprising a random copolymer of Formula III, as defined above, and a pharmaceutically acceptable carrier or diluent, or an effective amount of a pharmaceutical composition comprising a random copolymer having a monomer unit of Formula I and a monomer unit of Formula II, as defined above.

The copolymers of the invention can also be used as antidotes for hemorrhagic complications associated with low molecular weight heparin therapy.

Heparin has been commonly used as an anticoagulant and antithrombotic agent in the hospital setting. However, there are several pharmacokinetic parameters of standard heparin (SH) that complicate therapy. For example, the high serum protein-binding activity of SH precludes subcutaneous administration and its rapid and unpredictable plasma clearance necessitates constant monitoring of activated partial thromboplastin time to assess effectiveness (Turpie, A.G.G., Am. Heart J. 135:S329-S335 (1998)). More recently, low molecular weight heparin derivatives (LMWH) have become the standard of care for the management of major vessel thrombotic conditions (Hirsh, J., and Levine, M.N., Blood. 79:1-17 (1992)). Nevertheless, LMWHs have gained popularity over standard heparin (SH) as antithrombotic agents because of their improved pharmacokinetics and more predictable anticoagulant responses to weight-adjusted doses. LMWHs are formed by enzymatic or chemical cleavage of heparin and are effective factor Xa inhibitors because they contain the high affinity pentasaccharide sequence. However, they are not effective thrombin inhibitors (Hirsh, J., and Levine, M.N., Blood. 79:1-17 (1992)).

Both SH and LMWH have a high net negative (anionic) charge. Hemorrhagic complications are associated with antithrombotic treatments with both agents and an overdose may result in serious bleeding. Protamine, by virtue of its positive charge, can neutralize the effects of the heparin but protamine therapy also has serious adverse, side-effects including hypotension, pulmonary hypertension and impairment of certain blood cells including platelets and lymphocytes (Wakefield, T.W., et al., J. Surg. Res. 63:280-286 (1996)). Therefore, there is a strong need for the development of safe and effective antidotes for hemorrhagic complications associated with SH and LMWH antithrombotic therapies.

The copolymers of the present invention have been shown to inhibit the anticoagulation effects of heparin, in particular, low molecular weight heparin, and can be used as antidotes for hemorrhagic complications associated with low molecular weight heparin therapy.

Thus, in some aspects, the invention is directed to a method of providing an antidote to low molecular weight heparin overdose in an animal in need thereof, the method comprising administering to the animal an effective amount

of a pharmaceutical composition comprising a random copolymer of Formula III, as defined above, and a pharmaceutically acceptable carrier or diluent, or an effective amount of a pharmaceutical composition comprising a random copolymer having a monomer unit of Formula I and a monomer unit of Formula II, as defined above.

In some aspects of the invention, the copolymers of the present invention are useful as disinfectants. For example, coatings and paints adhesives are all exposed to microbial contamination and are used in locations where microbial growth is undesirable. Thus, the copolymers of the present invention are incorporated into polishes, paints, sprays, or detergents formulated for application to surfaces to inhibit the growth of a bacterial species thereon. These surfaces include, but are not limited to surfaces, such as, countertops, desks, chairs, laboratory benches, tables, floors, bed stands, tools or equipment, doorknobs, and windows. Copolymers of the present invention are also incorporated into soaps, cosmetics, lotions, such as hand lotions, and handwashes. The present cleansers, polishes, paints, sprays, soaps, cosmetics, lotions, handwashes, or detergents contain copolymers of the present invention that provide a bacteriostatic property to them. They can optionally contain suitable solvent(s), carrier(s), thickeners, pigments, fragrances, deodorizers, emulsifiers, surfactants, wetting agents, waxes, or oils. For example, in some aspects of the invention, the copolymers are incorporated into a formulation for external use as a pharmaceutically acceptable skin cleanser, particularly for the surfaces of human hands. Cleansers, polishes, paints, sprays, soaps, lotions, handwashes, and detergents are the like containing the copolymers of the present invention are useful in homes and institutions, particularly but not exclusively in hospital settings for the prevention of nosocomial infections.

In other aspects of the invention, the copolymers of the invention are useful as preservatives and can be used in a method for killing or inhibiting the growth of a microbial species in a product. For example, the copolymers of the invention can be used as preservatives in cosmetics.

The copolymers also can be added to foodstuffs as a preservative. Foodstuffs that can be treated with copolymers of the invention include, but are not limited to, non-acidic foods, such as mayonnaise or other egg products, potato products, and other vegetable or meat products. The copolymers for adding to the foodstuff can be part of any comestible formulation that can also include a suitable medium or carrier for convenient mixing or dissolving into a particular foodstuff. The medium or carrier is preferably one that will not interfere with the familiar flavor of the food of interest, such as are known by the artisan skilled in food processing techniques.

In yet other aspects of the invention, the copolymers of the present invention provide a surface-mediated microbicide that only kills organisms in contact with the surface and are useful as surface-mediated disinfectants or preservatives.

Any object that is exposed to or susceptible to bacterial or microbial contamination can be treated with the copolymers of the present invention to provide a microbial surface. To provide a microbial surface, copolymers of the present invention are attached to, applied on or incorporated into almost any substrate including but not limited to woods, paper, synthetic polymers (plastics), natural and synthetic fibers, natural and synthetic rubbers, cloth, glasses and ceramics by appropriate methods including covalent bonding, ionic interaction, coulombic interaction, hydrogen bonding or cross-linking. Examples of synthetic polymers include elastically deformable polymers which may be thermosetting or thermoplastic including, but not limited to polypropylene, polyethylene, polyvinyl chloride, polyethylene terephthalate, polyurethane, polyesters, such as polylactide, polyglycolide, rubbers such as polyisoprene, polybutadiene or latex, polytetrafluoroethylene, polysulfone and polyethylenesulfone polymers or copolymers. Examples of natural fibers include cotton, wool and linen.

The incidence of infection from food-borne pathogens is a continuing concern and antimicrobial packaging material, utensils and surfaces would be valuable. In the health care and medical device areas the utility of antimicrobial instruments, packaging and surfaces are obvious. Products used internally or externally in humans or animal health including, but not limited to, surgical gloves, implanted devices, sutures, catheters, dialysis membranes, water filters and implements, all can harbor and transmit pathogens.

Copolymers of the present invention are incorporated into any of these devices or implements to provide surface-medicated antimicrobial surfaces that will kill or inhibit the growth of organisms in contact with the surface. For example, copolymers of the present invention can be incorporated into spinnable fibers for use in materials susceptible to bacterial contamination including, but not limited to, fabrics, surgical gowns, and carpets. Copolymers of the invention can also be used to coat or incorporate into HVAC systems and electronic components to kill or inhibit the growth of organisms on these surfaces. Also, ophthalmic solutions and contact lenses easily become contaminated and cause ocular infections. Antimicrobial storage containers for contact lens and cleaning solutions incorporating copolymers of the present invention would thus be very valuable.

Thus, in some embodiments, the present invention is directed to a method of killing or inhibiting the growth of a microorganism, the method comprising contacting the microorganism with an effective amount of a copolymer described above, for example, a random copolymer of Formula III, as defined above, or a random copolymer having a monomer unit of Formula I and a monomer unit of Formula II, as defined above.

The copolymers of the present invention are synthesized using free-radical polymerization in the presence of a chain transfer agent. Standard methods of free radical polymerization are known to those of skill in the art. (See, for

example, Mayo, F.R, J. Am. Chem. Soc. 65:2324-2329 (1943). See also "Polymer Synthesis: Theory and Practice" Third edition, D. Braun, H. Cherdron, H. Ritter, Springer-Verlag Berlin Heidelberg New York; Sanda, F., et al., Journal of Polymer Science: Part A: Polymer Chemistry, Vol. 36, 1981-1986 (1998); Henriquez, C., et al., Polymer 44:5559-5561 (2003); and De La Fuente, J.L., and Madruga, E.L., Journal of Polymer Science: Part A: Polymer Chemistry, Vol. 38, 170-178 (2000). See also Example 1 below, which provides a method for the synthesis of polymethacrylate random copolymers.) For example, the copolymers of the present invention are synthesized by direct polymerization of two vinyl monomers, each containing a C-C double bond, such as, for example, styrene or methylmethacrylate, to produce random copolymers.

A general scheme illustrating free-radical polymerization of a polymer, as shown in Scheme 1 below, in the presence of a chain transfer agent is illustrated in Figure 1A.

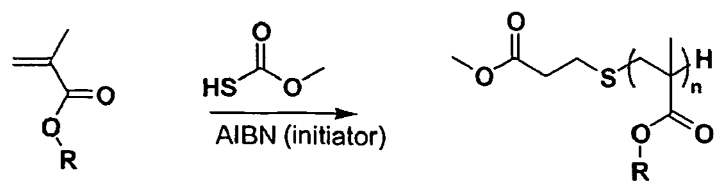


(1)

Scheme 1

Where appropriate, a protecting group can be added to a side chain group of a monomer to protect the side chain during radical polymerization. For example, the tert-butoxycarbonyl ("BOC") protecting group may be used to protect the free amine group of the monomer 2-aminoethyl methacrylate hydrochloride. See Example 1 and Scheme 2. Methods for chemically protecting reactive groups are known to those of skill in the art. See, for example, "Protective Groups in Organic Synthesis" Third edition, T. W. Greene, P. G. M. Wuts, John Wiley & Sons, Inc. (1999); and, for a description of radical polymerization of monomers having Boc protective groups, Sanda, F., et al., Journal of Polymer Science: Part A: Polymer Chemistry, Vol. 36, 1981-1986 (1998).

Monomers used in the synthesis of the copolymers of the present invention can be obtained commercially or prepared by methods known to those of skill in the art. For example, 2-aminoethyl methacrylate hydrochloride is commercially available.

The copolymers of the present invention can be tested for antimicrobial activity by methods well known to those of skill in the art. See, for example, Tew, G.N., *et al.* (Tew, G.N., et al., Proc. Natl. Acad. Sci. USA 99:5110-5114 (2002)). Antimicrobial testing can be carried out using the micro-broth dilution technique with E. *coli,* or, if desired, another bacterial strain, such as, for example, *B. subtilis, P. aeruginosa, K. pneumoniae, S. typhimurium, N. gonorrhoeae, B. megaterium, S. aureus, E. feacalis, M luteus* , or *S. pyogenes.* Other specific bacterial strains that can be screened include ampicillin and streptomycin-resistant *E. coli* D31, vancomycin-resistant *Enterococcus faecium* A436, and methicillin-resistant *S. aureus* 5332. Any copolymer found to be active can be purified to homogeneity and re-tested to obtain an accurate $IC_{50}$. Secondary screens include *Klebsiella pneumoniae* Kpl, and *Salmonella typhimurium* S5, and *Pseudomonus aeruginosa* 10. Traditionally, the micro-broth dilution technique only evaluates a single data point between 18-24 hours; however, the measurements can be extended to 24 hr to monitor cell growth through the entire growth phase. These experiments are performed in LB medium (which is a rich medium typically used to grow cells for protein expression) and represent a critical initial screen for activity. Since salt concentration, proteins, and other solutes can affect the activities of antibiotics, materials that show no activity in rich medium can be re-tested in minimal medium (M9) to determine if rich medium is limiting activity. No relationship between the media and the activity has been observed which is consistent with the mode of action this is believed to be through general membrane disruption.

Standard assays can be performed to determine whether a copolymer of the present invention is bacteriostatic or bactericidal. Such assays are well known to those of skill in the art and are performed, for example, by incubating *E. coli* cells overnight with the copolymer being tested, and then plating the mixture on agar plates according to procedures well known to those of skill in the art. See, for example, Tew, G.N*., et al.* (Tew, G.N., et al., Proc. Natl. Acad. Sci. USA 99:5110-5114 (2002)), and Liu, D., and DeGrado, W. F. (Liu, D., and DeGrado, W.F., J. Amer. Chem. Soc. 123:7553-7559 (2001)).

Assays for determining the antiviral and antifungal activity of copolymers of the present invention are also well known to those of skill in the art. For examples of antiviral assays, see Belaid et al., (Belaid, A., et al., J. Med. Virol. 66: 229-234 (2002)), Egal *et al.,* (Egal, M., et al., Int. J. Antimicrob. Agents 13:57-60 (1999)), Andersen *et* a/., (Andersen, J.H., et al., Antiviral Rs. 51:141-149 (2001)), and Bastian, A., and Schafer, H. (Bastian, A., and Schafer, H., Regul. Pept. 15:157-161 (2001)). See also Cole, A.M., et al., Proc. Natl. Acad. Sci USA 99:1813-1818 (2002). For examples of antifungal assays, see Edwards, J.R., et al., Antimicrobial Agents Chemotherapy 33:215-222 (1989), and Broekaert,

W.F., et al., FEMS Microbiol. Lett. 69:55-60 (1990). The entire contents of each of these documents is fully incorporated herein by reference.

Assays for measuring the cytotoxic selectivity for copolymers of the present invention toward bacteria and eukaryotic cells are well known to those of skill in the art. For example, cytotoxic selectivity can be assessed by determining the hemolytic activity of the copolymers. Hemolytic activity assays are performed by measuring the degree of hemolysis of human erythrocytes following incubation in the presence of the copolymer and determining $HC_{50}$ values. $HC_{50}$ values represent the concentration of compound that results in 50% hemoglobin release. See, for example, Kuroda, K, and DeGrado, W.F., J Amer. Chem. Soc. 127:4128-4129 (2005) and Liu, D., and DeGrado,W.F., J Amer. Chem. Soc. 123: 7553-7559 (2001), and references cited therein. See also Javadpour, M.M., et al., J. Med. Chem. 39:3107-3113 (1996).

Vesicle leakage assays can also be used to confirm whether a copolymer of the present invention interacts with and disrupt phospholipid bilayers, a model for cellular membranes. Vesicle leakage assays are well known to those of skill, in the art. See, for example, Tew, G. N., *et al.* (Tew, G.N., et al., Proc. Natl. Acad. Sci. USA 99:5110-5114 (2002)), and references cited therein.

Assays for determining the heparin-neutralizing activity of copolymers of the present invention are well known to those of skill in the art and are commonly performed using either an activated partial thromboplastin time assay (for example, by measuring the delay in clotting times for activated plasma in the presence of a fixed concentration of heparin, in the absence and presence of a test compound) or a Factor X assay. See, for example, Kandrotas (Kandrotas, R.J., Clin. Pharmacokinet. 22:359-374 (1992)), Wakefield *et al.* (Wakefield, T.W., et al., J. Surg. Res. 63:280-286 (1996)), and Diness, V., and Østergaard, P.B. (Diness, V.O., and Østergaard, P.B., Thromb. Haemost. 56:318-322 (1986)), and references cited therein. See also Wong, P.C., et al., J. Pharm. Exp. Therap. 292:351-357 (2000), and Ryn-McKenna, J.V., et al., Thromb. Haemost. 63:271-274 (1990).

The copolymers of the present invention can be used to kill or inhibit the growth of any of the following microbes or mixtures of the following microbes, or, alternatively, can be administered to treat local and/or systemic microbial infections or illnesses caused by the following microbes or mixtures of the following microbes: Gram-positive cocci, for example Staphylococci (*Staph. aureus, Staph. epidermidis*) and Streptococci (*Strept. agalactiae, Strept. faecalis, Strept. pneumoniae, Strept. pyogenes*); Gram-negative cocci (*Neisseria gonorrhoeae and Yersinia pestis*) and Gram-negative rods such as Enterobacteriaceae, for example *Escherichia coli, Hamophilus influenzae,* Citrobacter (*Citrob. freundii, Citrob. divernis*), Salmonella and Shigella, and Francisella (*Francisella tularensis*); Gram-positive rods such as Bacillus (*Bacillus anthracis, Bacillus thuringenesis*); furthermore Klebsiella (*Klebs. pneumoniae, Klebs. oxytoca*), Enterobacter (*Ent. aerogenes, Ent. agglomerans*), Hafnia, Serratia (*Serr. marcescens*), Proteus (*Pr. mirabilis, Pr. rettgeri, Pr. vulgaris*), Providencia, Yersinia, and the genus Acinetobacter. Furthermore, the antimicrobial spectrum of the copolymers of the present invention covers the genus Pseudomonas (*Ps. aeruginosa, Ps. maltophilia*) and strictly anaerobic bacteria such as, for example, *Bacteroides fragilis,* representatives of the genus Peptococcus, Peptostreptococcus and the genus Clostridium; furthermore Mycoplasmas (*M. pneumoniae, M hominis, Ureaplasma urealyticum*) as well as Mycobacteria, for example Mycobacterium tuberculosis. This list of microbes is purely illustrative and is in no way to be interpreted as restrictive.

Examples of microbial infections or illness that can be treated by administration of the copolymers of the present invention include, but are not limited to, microbial infections or illnesses in humans such as, for example, otitis, pharyngitis, pneumonia, peritonitis, pyelonephritis, cystitis, endocarditis, systemic infections, bronchitis (acute and chronic), septic infections, illnesses of the upper airways, diffuse panbronchiolitis, pulmonary emphysema, dysentery, enteritis, liver abscesses, urethritis, prostatitis, epididymitis, gastrointestinal infections, bone and joint infections, cystic fibrosis, skin infections, postoperative wound infections, abscesses, phlegmon, wound infections, infected burns, burns, infections in the mouth (including, e.g., but not limited to, periodontal disease and gingivitis), infections after dental operations, osteomyelitis, septic arthritis, cholecystitis, peritonitis with appendicitis, cholangitis, intraabdominal abscesses, pancreatitis, sinusitis, mastoiditis, mastitis, tonsileitis, typhoid, meningitis and infections of the nervous system, salpingitis, endometritis, genital infections, pelveoperitonitis and eye infections.

Examples of viral infections that can be treated by administration of the copolymers of the present invention include, but are not limited to, viral infections caused by human immunodeficiency virus (HIV-1, HIV-2), hepatitis virus (*e.g.*, hepatitis A, hepatitis B, hepatitis C, hepatitis D, and hepatitis E viruses), herpesviruses (*e.g.*, herpes simplex virus types 1 and 2, varicella-zoster virus, cytomegalovirus, Epstein Barr virus, and human herpes viruses types 6, 7, and 8), influenza virus, respiratory syncytial virus (RSV), vaccinia virus, and adenoviruses. This list is purely illustrative and is in no way to be interpreted as restrictive.

Examples of fungal infections or illnesses that can be treated by administration of the copolymers of the present invention include, but are not limited to, fungal infections caused by Chytridiomycetes, Hyphochrytridiomycetes, Plasmodiophoromycetes, Oomycetes, Zygomycetes, Ascomycetes, and Basidiomycetes. Fungal infections which can be inhibited or treated with compositions of the copolymers provided herein include, but are not limited to: Candidiasis, including, but not limited to, onchomycosis, chronic mucocutaneous candidiasis, oral candidiasis, epiglottistis, esophagitis, gastrointestinal infections, genitourinary infections, for example, caused by any Candida species, including, but not

limited to, *Candida albicans, Candida tropicalis, Candida (Torulopsis) glabrata, Candida parapsilosis, Candida lusitaneae, Candida rugosa* and *Candida pseudotropicalis;* Aspergillosis, including, but not limited to, granulocytopenia caused, for example, by, Aspergillus spp. Including, but not limited, to *Aspergillus fumigatus, Aspergillus favus, Aspergillus niger* and *Aspergillus terreus;* Zygomycosis, including, but not limited to, pulmonary, sinus and rhinocerebral infections caused by, for example, zygomycetes such as Mucor, Rhizopus spp., Absidia, Rhizomucor, Cunningamella, Saksenaea, Basidobolus and Conidobolus; Cryptococcosis, including, but not limited, to infections of the central nervous system, e.g., meningitis, and infections of the respiratory tract caused by, for example, *Cryptococcus neoformans;* Trichosporonosis caused by, for example, *Trichosporon beigelii;* Pseudallescheriasis caused by, for example, *Pseudallescheria boydii;* Fusarium infection caused by, for example, Fusarium such as *Fusarium solani, Fusarium moniliforme* and *Fusarium proliferartum;* and other infections such as those caused by, for example, Penicillium spp. (generalized subcutaneous abscesses), Trichophyton spp., for example, *Trichophyton mentagrophytes* and *Trichophyton rubrum,* Stachybotrys spp., for example, *S. chartarum,* Drechslera, Bipolaris, Exserohilum spp., *Paecilomyces lilacinum, Exophila jeanselmei* (cutaneous nodules), *Malassezia furfur* (folliculitis), Alternaria (cutaneous nodular lesions), *Aureobasidium pullulans* (splenic and disseminated infection), Rhodotorula spp. (disseminated infection), Chaetomium spp. (empyema), *Torulopsis candida* (fungemia), Curvularia spp. (nasophamygeal infection), Cunninghamella spp. (pneumonia), *H. Capsulatum, B. dermatitidis, Coccidioides immitis, Sporothrix schenckii* and *Paracoccidioides brasiliensis, Geotrichum candidum* (disseminated infection). The copolymers of the present invention can also be used to kill or inhibit the growth of any of the fungi listed above. This list is purely illustrative and is in no way to be interpreted as restrictive.

The copolymers of the present invention can be administered to a human subject. Thus, in some aspects of the invention, the copolymers are administered to a human.

The methods disclosed above also have veterinary applications and can be used to treat a wide variety of non-human vertebrates. Thus, in other aspects of the invention, the copolymers of the present invention are administered in the above methods to non-human vertebrates, such as wild, domestic, or farm animals, including, but not limited to, cattle, sheep, goats, pigs, dogs, cats, and poultry such as chicken, turkeys, quail, pigeons, ornamental birds and the like.

The following are examples of microbial infections in non-human vertebrates that can be treated by administering a copolymer of the present invention: Pig: coli diarrhoea, enterotoxaemia, sepsis, dysentery, salmonellosis, metritis-mastitis-agalactiae syndrome, mastitis; ruminants (cattle, sheep, goat): diarrhoea, sepsis, bronchopneumonia, salmonellosis, pasteurellosis, mycoplasmosis, genital infections; horse: bronchopneumonias, joint ill, puerperal and post-puerperal infections, salmonellosis; dog and cat: bronchopneumonia, diarrhoea, dermatitis, otitis, urinary tract infections, prostatitis; poultry (chicken, turkey, quail, pigeon, ornamental birds and others): mycoplasmosis, *E. coli* infections, chronic respiratory tract illnesses, salmonellosis, pasteurellosis, psittacosis. This list is purely illustrative and is in no way to be interpreted as restrictive.

For those applications in which the copolymers of the present invention are used as disinfectants and/or preservatives, *e.g*., in cleansers, polishers, paints, sprays, soaps, or detergents, the copolymers are incorporated into the cleanser, polisher, paint, spray, soap, or detergent formulation, optionally in combination with suitable solvent(s), carner(s), thickeners, pigments, fragrances, deodorizers, emulsifiers, surfactants, wetting agents, waxes, or oils. If the copolymer is to be used as a preservative in a foodstuff, the copolymer can be added to the foodstuff as part of any comestible formulation that can also include a suitable medium or carrier for convenient mixing or dissolving into the foodstuff. The amount of copolymer added to or incorporated into the cleanser, polisher, soap, etc. formulation or into the foodstuff will be an amount sufficient to kill or inhibit the growth of the desired microbial species and can easily be determined by one of skill in the art.

For those applications in which the copolymers of the invention are used as surface-mediated microbicides, *e.g*., in some applications as disinfectants and as preservatives (*e.g*., including, but not limited to, medical devices such as catheters, bandages, and implanted devices, or food containers and food handling implements), the copolymers can be attached to, applied on or incorporated into almost any substrate including, but not limited to, woods, paper, synthetic polymers (plastics), natural and synthetic fibers, natural and synthetic rubbers, cloth, glasses and ceramics by appropriate methods, including covalent bonding, ionic interaction, coulombic interaction, hydrogen bonding or cross-linking.

Procedures for attaching, applying, and incorporating the copolymers of the present invention into appropriate materials and substrates are disclosed in WIPO Publ. No. WO 02/100295, the contents of which are fully incorporated herein by reference. Appropriate substrates and materials are also disclosed in WO 02/100295.

The copolymers of the present invention can be administered in the conventional manner by any route where they are active. Administration can be systemic, topical, or oral. For example, administration can be, but is not limited to, parenteral, subcutaneous, intravenous, intramuscular, intraperitoneal, transdermal, oral, buccal, or ocular routes, or intravaginally, by inhalation, by depot injections, or by implants. Thus, modes of administration for the copolymers of the present invention (either alone or in combination with other pharmaceuticals) can be, but are not limited to, sublingual, injectable (including short-acting, depot, implant and pellet forms injected subcutaneously or intramuscularly), or by use of vaginal creams, suppositories, pessaries, vaginal rings, rectal suppositories, intrauterine devices, and transdermal forms such as patches and creams.

Specific modes of administration will depend on the indication (*e.g.*, whether the copolymers are administered to treat a microbial infection, or to provide an antidote for hemorrhagic conditions associated with heparin therapy). The mode of administration can depend on the pathogen or microbe to be targeted. The selection of the specific route of administration and the dose regimen is to be adjusted or titrated by the clinician according to methods known to the clinician in order to obtain the optimal clinical response. The amount of copolymer to be administered is that amount which is therapeutically effective. The dosage to be administered will depend on the characteristics of the subject being treated, *e.g.*, the particular animal treated, age, weight, health, types of concurrent treatment, if any, and frequency of treatments, and can be easily determined by one of skill in the art (*e.g.*, by the clinician).

Pharmaceutical formulations containing the copolymers of the present invention and a suitable carrier can be solid dosage forms which include, but are not limited to, tablets, capsules, cachets, pellets, pills, powders and granules; topical dosage forms which include, but are not limited to, solutions, powders, fluid emulsions, fluid suspensions, semi-solids, ointments, pastes, creams, gels and jellies, and foams; and parenteral dosage forms which include, but are not limited to, solutions, suspensions, emulsions, and dry powder; comprising an effective amount of a copolymer of the present invention. It is also known in the art that the active ingredients can be contained in such formulations with pharmaceutically acceptable diluents, fillers, disintegrants, binders, lubricants, surfactants, hydrophobic vehicles, water soluble vehicles, emulsifiers, buffers, humectants, moisturizers, solubilizers, preservatives and the like. The means and methods for administration are known in the art and an artisan can refer to various pharmacologic references for guidance. For example, Modern Pharmaceutics, Banker & Rhodes, Marcel Dekker, Inc. (1979); and Goodman & Gilman's The Pharmaceutical Basis of Therapeutics, 6th Edition, MacMillan Publishing Co., New York (1980) can be consulted.

The copolymers of the present invention can be formulated for parenteral administration by injection, *e.g.*, by bolus injection or continuous infusion. The copolymers can be administered by continuous infusion subcutaneously over a period of about 15 minutes to about 24 hours. Formulations for injection can be presented in unit dosage form, *e.g.*, in ampoules or in multi-dose containers, with an added preservative. The compositions can take such forms as suspensions, solutions or emulsions in oily or aqueous vehicles, and can contain formulatory agents such as suspending, stabilizing and/or dispersing agents.

For oral administration, the copolymers can be formulated readily by combining these compounds with pharmaceutically acceptable carriers well known in the art. Such carriers enable the compounds of the invention to be formulated as tablets, pills, dragees, capsules, liquids, gels, syrups, slurries, suspensions and the like, for oral ingestion by a patient to be treated. Pharmaceutical preparations for oral use can be obtained by adding a solid excipient, optionally grinding the resulting mixture, and processing the mixture of granules, after adding suitable auxiliaries, if desired, to obtain tablets or dragee cores. Suitable excipients include, but are not limited to, fillers such as sugars, including, but not limited to, lactose, sucrose, mannitol, and sorbitol; cellulose preparations such as, but not limited to, maize starch, wheat starch, rice starch, potato starch, gelatin, gum tragacanth, methyl cellulose, hydroxypropylmethyl-cellulose, sodium carboxymethylcellulose, and polyvinylpyrrolidone (PVP). If desired, disintegrating agents can be added, such as, but not limited to, the cross-linked polyvinyl pyrrolidone, agar, or alginic acid or a salt thereof such as sodium alginate.

Dragee cores can be provided with suitable coatings. For this purpose, concentrated sugar solutions can be used, which can optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments can be added to the tablets or dragee coatings for identification or to characterize different combinations of active compound doses.

Pharmaceutical preparations which can be used orally include, but are not limited to, push-fit capsules made of gelatin, as well as soft, sealed capsules made of gelatin and a plasticizer, such as glycerol or sorbitol. The push-fit capsules can contain the active ingredients in admixture with filler such as, *e.g.*, lactose, binders such as, *e.g.*, starches, and/or lubricants such as, *e.g.*, talc or magnesium stearate and, optionally, stabilizers. In soft capsules, the active compounds can be dissolved or suspended in suitable liquids, such as fatty oils, liquid paraffin, or liquid polyethylene glycols. In addition, stabilizers can be added. All formulations for oral administration should be in dosages suitable for such administration.

For buccal administration, the copolymer compositions can take the form of, *e.g.*, tablets or lozenges formulated in a conventional manner.

For administration by inhalation, the copolymers for use according to the present invention are conveniently delivered in the form of an aerosol spray presentation from pressurized packs or a nebulizer, with the use of a suitable propellant, *e.g.*, dichlorodifluoromethane, trichlorofluoromethane, dichlorotetrafluoroethane, carbon dioxide or other suitable gas. In the case of a pressurized aerosol the dosage unit can be determined by providing a valve to deliver a metered amount. Capsules and cartridges of, *e.g.*, gelatin for use in an inhaler or insufflator can be formulated containing a powder mix of the compound and a suitable powder base such as lactose or starch.

The copolymers of the present invention can also be formulated in rectal compositions such as suppositories or retention enemas, *e.g.*, containing conventional suppository bases such as cocoa butter or other glycerides.

In addition to the formulations described previously, the copolymers of the present invention can also be

formulated as a depot preparation. Such long acting formulations can be administered by implantation (for example subcutaneously or intramuscularly) or by intramuscular injection.

Depot injections can be administered at about 1 to about 6 months or longer intervals. Thus, for example, the compounds can be formulated with suitable polymeric or hydrophobic materials (for example as an emulsion in an acceptable oil) or ion exchange resins, or as sparingly soluble derivatives, for example, as a sparingly soluble salt.

In transdermal administration, the copolymers of the present invention, for example, can be applied to a plaster, or can be applied by transdermal, therapeutic systems that are consequently supplied to the organism.

Pharmaceutical compositions of the copolymers also can comprise suitable solid or gel phase carriers or excipients. Examples of such carriers or excipients include but are not limited to calcium carbonate, calcium phosphate, various sugars, starches, cellulose derivatives, gelatin, and polymers such as, *e.g.*, polyethylene glycols.

The copolymers of the present invention can also be administered in combination with other active ingredients, such as, for example, adjuvants, protease inhibitors, or other compatible drugs or compounds where such combination is seen to be desirable or advantageous in achieving the desired effects of the methods described herein (*e.g.*, controlling infection caused by harmful microorganisms, or treating hemorrhagic complications associated with heparin therapy.). For example, the copolymers of the present invention can be administered with other antibiotics, including, but not limited to, vancomycin, ciprofloxacin, merapenem, oxicillin, and amikacin.

The following examples will serve to further typify the nature of this invention but should not be construed as a limitation in the scope thereof, which scope is defined solely by the appended claims.

EXAMPLE 1

Synthesis and Characterization of Polymethacrylate Random Copolymers

A series of polymethacrylate random copolymers were synthesized using the process of free radical polymerization and tested for antimicrobial activity using *in vitro* assays. Cationic and hydrophobic groups were randomly distributed along the polymer chain during the radical polymerization synthesis to produce random copolymers having the following general structure:

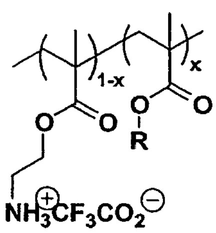

wherein x is the mole fraction of hydrophobic monomer units and 1-x is the mole fraction of cationic monomer units. A chain transfer agent was used to control the molecular weight of the copolymers. The series of random copolymers varied in both the percentage and identity of hydrophobic groups distributed along the polymer backbone and molecular weight of the copolymer.

Methods

<u>Instrumentation</u>. $^1$H NMR spectra were obtained on Varian Unity 500 NMR spectrometer. Gel permeation chromatography (GPC) measurements were carried out using two columns (PLgel, 5μm, mixed-C, Polymer laboratories) connected in series and a refractive index detector at room temperature. THF was used as an eluent. Molecular weights ($M_n$ and $M_w$) of polymers were calculated based on calibration using monodisperse polystyrene standards.

<u>Synthesis of Boc-protected methacrylate monomer (**1**)</u>. Methacryloyl chloride (6.5 mL, 65 mmol) was added to *tert*-butyl N-(2-hydroxyethyl) carbamate (10 g, 62 mmol) in THF (100 mL) and triethylamine (9.5 mL, 68mmol) cooled in a dry ice/acetone bath. The reaction mixture was allowed to warm at room temperature overnight, and the resultant precipitate was filtered off. The filtrate was concentrated by evaporation and the residue diluted with $CH_2Cl_2$, washed with water, 1N NaOH and brine. The organic layer was dried over $Na_2SO_4$, and the solvent was evaporated under reduced pressure. The product was purified by silica gel column chromatography ($CH_2Cl_2$ (90): EtOAc (10)). The obtained solid was recrystallized from $CH_2Cl_2$/hexane to give the product, 4.74 g (31% yield).

<u>Synthesis of amphiphilic polymethacrylate copolymers (**6**)</u>. Monomers **1** and **4** (0.435 mmol total) were dissolved in dioxane or acetonitrile (0.5 mL) containing AIBN (0.716 mg, **4**.35 μmol) and methyl 3-mercaptopropionate as a chain transfer agent and purged with Ar for 2 minutes. The reaction mixture was stirred at 60°C overnight, and the solvent

was evaporated off. The oily residue was diluted with $CH_2Cl_2$ and dropped into hexane. The obtained precipitate was collected by filtration and dried under vacuum. Monomer compositions for the resultant copolymer (polymer 5) were calculated via integration of the signals from methylene protons of the side chains in $^1$H NMR spectra in $CDCl_3$.

The copolymer (polymer **5**) (10-20 mg) was dissolved in TFA (1 mL) and stirred for 1 hour at room temperature. After TFA was removed by evaporation, the oily residue was rinsed with diethyl ether. The resultant precipitate was collected by centrifugation with further rinses of diethyl ether, and dried under vacuum overnight or lyophilized to give copolymer **6** as a white powder.

<u>Antimicrobial assays</u>. Each polymer was dissolved in DMSO (5 mg/mL). This solution was mixed with water to make a series of stock solutions of 2-fold dilution, which were diluted 10-fold when added to 96-well plates containing *E. coli* solutions (Mueller-Hilton medium). The polymer solutions with *E. coli* were incubated at 36˚C for 18 hours, and cell growth was measured by optical density at λ=595nm (Liu, D., et al., Angew. Chem. Int. Ed, 43:1158 (2004); Tew, G.N., et al., PNAS 99:5110 (2002)).

Results

Synthesis of polymethacrylate polymers using a chain transfer agent.

The molecular weights of the polymers were controlled using a chain transfer agent (CTA) during the radical polymerizations. To determine the degree of polymerization at various concentrations of CTA, Boc-protected amine monomer **1** was polymerized with n-butyl methacrylate using an azobisbutyronitrile (AIBN) radical initiator in the presence of methyl 3-mercaptpropionate as a chain transfer agent (CTA), to give Boc-protected polymer **2** (Scheme 2).

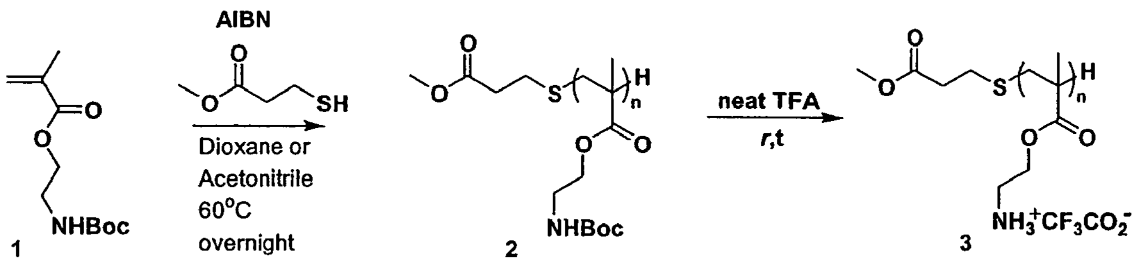


1 2 3

Scheme 2

The polymers were characterized by $^1$H NMR and gel permeation chromatography (GPC) as described above. The results are presented in Table 1. Because the signal from its terminal methyl group is resolved from other peaks of polymers in $^1$H NMR spectra, methyl 3-mercaptpropionate provides a useful marker for determining the average degree of polymerization ("DP") of a polymer that has been synthesized using this thiol compound as a CTA. Accordingly, NMR analysis was used to determine the DP for polymer **2** by integrating the signals from the methyl group of the CTA residue in the polymer terminus relative to the methylene proton in the monomer side chains of the polymer. The DP was calculated for the polymer at various CTA concentrations using the Mayo equation (Mayo, F.R., J. Am. Chem. Soc. 65: 2324 (1943); see also Figs. 1A and 1B):

$$\frac{1}{DP} = \frac{1}{DP_0} + C_T \frac{[CTA]}{[Monomer]}$$

where $DP_0$ represents the degree of polymerization in the absence of CTA, and $C_T$ represents a chain transfer constant. Figure 2 shows the plot of the reciprocal of DPs of polymer 2 obtained at different CTA concentrations against [CTA]/[1]. The data gave a straight line. The chain transfer constant, CT, deduced from the slope is 0.86. The Boc group of polymer 2 was removed by TFA to give cationic polymer 3.

**Table 1.** Characterization of Boc-protected polymer 2

| [CTA]/[1] | $M_n^a$ | $M_w^a$ | PDI$^b$ | Yield (%) | DP$^c$ |
|---|---|---|---|---|---|
| 0.01 | 10300 | 17140 | 1.66 | 89 | 77 |

(continued)

| [CTA]/[1] | $M_n$[a] | $M_w$[a] | PDI[b] | Yield (%) | DP[c] |
|---|---|---|---|---|---|
| 0.05 | 2560 | 4610 | 1.80 | 84 | 22 |
| 0.1 | 1163 | 1920 | 1.65 | 66 | 11 |

a) GPC (THF, polystyrene standard).
b) Defined by $M_w/M_n$. $M_w$ represents the weight-average molecular weight;
$M_n$ represents the number-average molecular weight.
c) Determined by $^1$H NMR analysis.

Synthesis of polymethacrylate random copolymers.

Monomer **1** was polymerized with comonomers **4** bearing different hydrophobic groups in the presence of a thiol as a CTA (Scheme 3).

Scheme 3

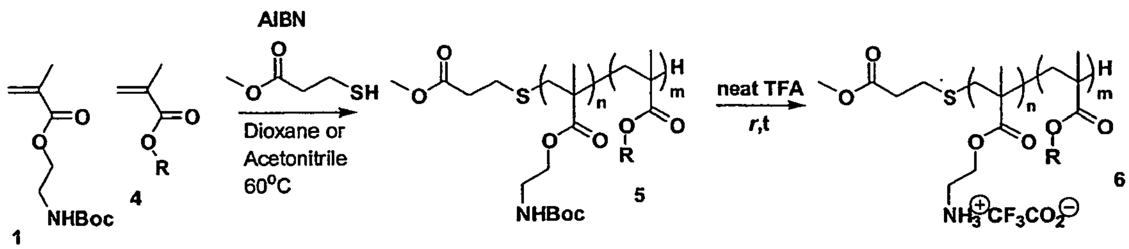

1 4 5 6

R = ethyl, butyl, hexyl, isobutyl, or benzyl.

In order to make a diverse library of polymers, feed ratios of monomer **1** to comonomers and CTA concentrations were varied. Table 2 shows the characterization results for copolymers 5 with ethyl methacrylate or benzyl methacrylate polymerized in the presence of 10 mol % of CTA relative to the total amount of monomers. $^1$H NMR analysis for the copolymers detemined the average monomer compositions of the polymers and their DPs. The monomer compostions in the resultant polymers were close to the feed compositions, and the DPs for both polymers were quite similar. The copolymers 5 were deprotected by TFA to give cationic polymers 6. The polymers were readily soluble to water.

**Table 2.** Characterization of random copolymers

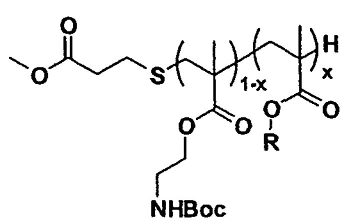

| R | $X_{feed}$[a] (mol%) | X[b] (mol%) | Mn[c] | Mw[c] | PDI[d] | Yield | Dp[b] |
|---|---|---|---|---|---|---|---|
| 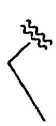 | 10 | 10 | 3170 | 4580 | 1.44 | 62 | 16 |
| | 20 | 17 | 2910 | 3950 | 1.36 | 69 | 17 |
| | 40 | 35 | 1710 | 2160 | 1.26 | 58 | 17 |
| | 60 | 52 | 2320 | 3020 | 1.30 | 60 | 15 |
| | 80 | 73 | 2000 | 2550 | 1.28 | 62 | 19 |
| | 100 | 100 | 1210 | 1860 | 1.54 | n.d. | n.d. |

(continued)

| R | $X_{feed}$[a] (mol%) | X[b] (mol%) | Mn[c] | Mw[c] | PDI[d] | Yield | Dp[b] |
|---|---|---|---|---|---|---|---|
| 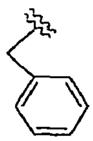 | 10 | 11 | 1920 | 3250 | 1.69 | 90 | 17 |
| | 20 | 22 | 1500 | 2530 | 1.69 | 90 | 16 |
| | 40 | 41 | 1470 | 2630 | 1.79 | 75 | 15 |
| | 60 | 62 | 1150 | 2120 | 1.84 | 82 | 16 |
| | 80 | 80 | 1360 | 2420 | 1.78 | 88 | 16 |
| | 100 | 100 | 1770 | 3300 | 1.86 | n.d. | n.d. |

a) Feed composition.
b) Determined by $^1H$ NMR analysis.
c) Determined by GPC (THF, polystyrene standard)
d) Defined by $M_w/M_n$.

The series of copolymers 6 were tested for antimicrobial activity using *in vitro* antimicrobial assays as described above. Minimum inhibitory concentrations (MIC) were measured with *E. coli* for the series of polymers 6. The data for the ethyl methacrylate copolymers are listed in Table 3. The polymers exhibited higher activity (lower MICs) as the percentage of hydrophobic groups increased. The anionic polymer analogues with carboxylic acids are inactive. The same tendency was observed for other polymers bearing long alkyl chains, but the activity was diminished at higher percentage of hydrophobic groups (data not shown). Lower activity was also observed for larger molecular weight polymers (data not shown).

**Table 3.** Characterization and antimicrobial activity of ethyl methacrylate cationic polymers **6**

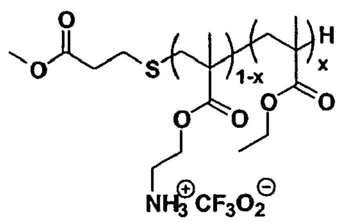

| $X_{feed}$ (mol%) | X (mol%) | DP[a] | MIC[b] (μg/mL) |
|---|---|---|---|
| 10 | 11 | 21 | 500 |
| 20 | 19 | 20 | 125 |
| 40 | 34 | 18 | 63 |
| 60 | 54 | 18 | 63 |

a) 10 mol% CTA was used in the polymerizations.
b) Measured with *E. coli*

EXAMPLE 2

Antimicrobial Activities of a Series of Polymethacrylate Random Copolymers

Five series of polymethacrylate random copolymers of the following general structure were synthesized and tested for antimicrobial activity as described in Example 1 and Scheme 3 above.

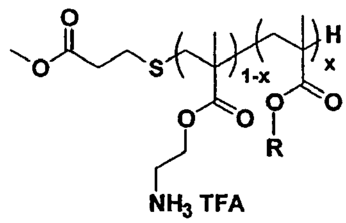

R = ethyl, butyl, hexyl, isobutyl, or benzyl.

The chain transfer agent methyl 3-mercaptpropionate was used to control the molecular weight of the copolymers. For each series of copolymers, feed ratios of monomer **1**

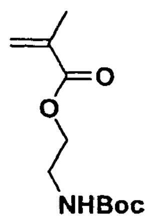

**1**

to hydrophobic comonomer (i.e., ethyl methacrylate, butyl methacrylate, hexyl methacrylate, isobutyl methacrylate, or benzyl methacrylate) and CTA concentrations were varied to produce random copolymers varying in both the percentage and identity of hydrophobic groups distributed along the polymer backbone and molecular weight. Four categories for copolymers were synthesized, based on MW range (average DP): large ("L"; MW approx. 30,000-10,000); medium ("M"; MW approx. 4,000-2,000; CP = 15-20); small ("S"'; MW approx. 2,500; DP = 12); and extra-small ("XS"; MW approx. 2,000-1,000; DP = 6-8). An additional limited series of anionic COOH-ethyl copolymer 10 was prepared.

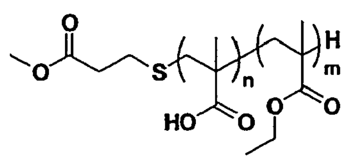

**10**

Each series of copolymers were tested for antimicrobial activity using *E. coli* in the antimicrobial assay described in Example 1. Deprotected monomer 1, 2-aminoethyl methacrylate hydrochloride, was tested as a control. The results are presented in Table 4.

**Table 4.**

| Results: MIC (μg/mL) for *E.coli* (incubation time: 18 hours) |
|---|

(continued)

| Polymers | | Size composition of hydrophobic groups (%) | | | | | | | | |
|---|---|---|---|---|---|---|---|---|---|---|
| | | 0 | 10 | 20 | 30 | 40 | 50 | 60 | 70 | 80 |
| 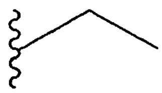 Ethyl | L[a] | | 125 | 63 | 63 | 63 | | >500 | >500 | |
| | M | | 500 | 125 | 63 | | 63 | | | |
| | s | | 500 | 500 | | 63 | 63 | | | |
| | XS[e] | >500 | 500 | 500 | | 63 | 31 | | 31 | |
| 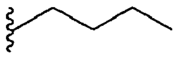 Butyl | L[a] | | 125 | 63 | | >500 | | >500 | | |
| | M | | - | 63 | | 63 | >500 | | | |
| | XS[e] | | 500[b] 125[c] | | 31 | | 16 | | | |
| 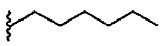 Hexyl | L[a] | | 125 | 63 | | >500 | | >500 | | |
| | M | | 125 | 63 | >500 | | | | | |
| | XS[e] | | 125 | 31 | 31 | 16 | | | | |
| 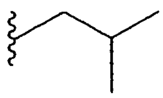 Iso-Butyl | L[a] | | 63 | 63 | | >500 | | | | |
| | M | | 500 | 63 | 31 | | >500 | | | |
| | XS[e] | | 250 | 63 | | 16 | 16 | 31 | | |
| 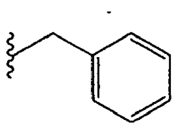 Benzyl | L[a] | | 250 | 125 | | >500 | >500 | >500 | | |
| | M | | 125 | 63 | | >500 | | >500 | | >500 |
| | XS[e] | | 125 | 63 | 63 | | 31 | | | |
| COOH-Ethyl | XS | >500 | >500 | >500 | | | | | | |
| Monomer[d] | | >500 | | | | | | | | |

[Polymer] = 500, 250, 125, 62.5, 31.3, 15.6, 7.8, 3.9 μg/mL (2-fold dilution series).
a) Theoretical composition of hydrophobic groups in polymerizations was used.
b) 8% butyl groups.
c) 13% butyl groups.
d) Commercially available; re-crystallized from ethyl acetate.
e) Containing 10-20% unreacted amine monomers.

EXAMPLE 3

Synthesis and Characterization of *n*-Butyl Methacrylate Random Copolymers

Three series of *n*-butyl methacrylate random copolymers were prepared using the process of free-radical polymerization in the presence of a chain transfer agent and characterized according to the methods described in Scheme 4 below and in Example 1, and in Kuroda et al., J. Am. Chem. Soc. 127:4128-4129 (2005), with supporting information at http://pubs.acs.org. The entire contents of Kuroda et al., J. Am. Chem. Soc. 127:4128-4129 (2005), with supporting information, are incorporated by reference herein in their entirety.
Synthesis of random copolymers. R = butyl.

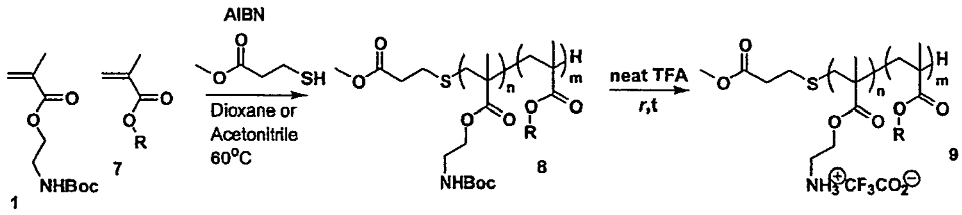


Scheme 4

Boc-protected amine monomer **1** was polymerized as indicated in Scheme 3 with *n*-butyl methacrylate monomer **7** using an azobisbutyronitrile (AIBN) radical initiator in the presence of methyl 3-mercaptproponate as a chain transfer agent (CTA). The CTA was used to control the degree of polymerization, and thus the molecular weights, of the resultant polymers. Three series of *n*-butyl methacrylate random copolymers (copolymers 8) having different monomer compositions and molecular weights were produced by varying the feed ration of monomers and CTA. $^1$H NMR analysis provided determination of the average monomer compositions and degree of polymerization (DP). Molecular weights (number-average) were calculated from the DP values determined by $^1$H NMR analysis. The obtained boc-protected copolymers were deprotected by neat TFA and the precipitation of the copolymers in diethyl ether gave copolymers **9.** Copolymers with low molecular weights were purified by size exclusion column chromatography (Sephadex LH-20 in methanol) due to difficulty in precipitating the low molecular weight copolymers.

The synthetic procedures for each series are as follows:

Synthetic procedures for amphiphilic polymethacrylate derivatives: Polymer series 1 and 2:

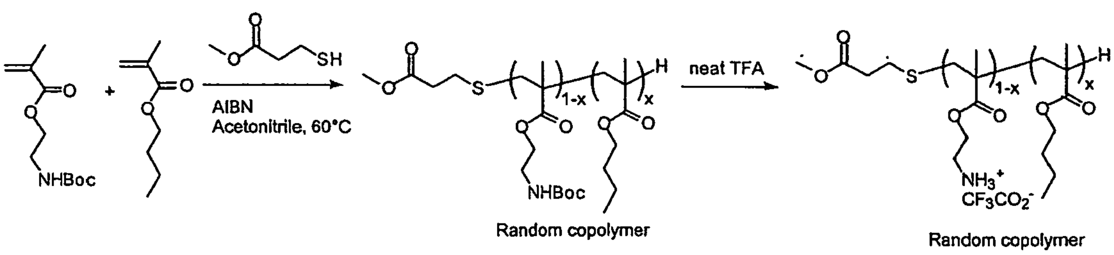


Boc-protected polymers: *N*-(*tert*-butoxycarbonyl)aminoethyl methacrylate and *n*-butyl methacrylate (0.435mmol total) were dissolved in acetonitrile (0.5mL) containing AIBN (0.716mg, 4.35μmol) and methyl 3-mercaptopropionate as a chain transfer agent and purged with Ar for 2 minutes. The reaction mixture was stirred at 60°C overnight, and the solvent was evaporated off. The oily residue was diluted with $CH_2Cl_2$ and dropped into hexane. The obtained precipitate was collected by centrifugation and dried under vacuum. The mole percentage of butyl groups ($MP_{Bu}$) for the polymers was calculated via integration ratio of the signals from methylene protons of the monomer side chains in $^1$H NMR spectra ($CDCl_3$). The DP was determined by integration of the signals from methylene protons of the side chains relative to those from methyl protons of MMP at the.polymer terminal. $^1$H NMR (500MHz, $CDCl_3$) for the representative polymer ($MP_{Bu}$= 29 and DP= 30): δ 4.2-3.8 (bm, 59.49H), 3.691 (s, 3H), 3.390 (bs, 42.44H), 2.8-2.5 (m, 5.28H), 2.2-0.8 (m, 427.92H). Characterization results are summarized in Tables 5-1 and 5-2.

Deprotection of Boc-protected polymers: The Boc-protected polymer (10-20 mg) was dissolved in TFA (1mL) and stirred for 1 hour at room temperature. After TFA was removed by evaporation, the oily residue was rinsed with diethyl ether. The resultant precipitate was collected by centrifugation with further rinses of diethyl ether, and dried under vacuum overnight and lyophilized to give cationic polymers as a white powder. During the precipitation procedure after Boc-deprotection, the polymers that have large DPs selectively precipitated out from diethyl ether, and as a result, the collected polymers have larger DPs than those of the Boc-protected polymers. Characterization results were listed in Table S-1 and S-2. $^1$H NMR (500MHz, Methanol-$d_4$) for the representative polymer ($MP_{Bu}$= 27 and DP= 46): δ 4.4-4.1 (bs, 67.03H), 4.1-3.9 (bs, 25.34H), 3.681 (s, 3H), 2.8-2.5 (m, 8.71H), 2.3-1.8 (m, 76.93H), 1.7-0.8 (m, 243.35H)

**Table 5-1.** Characterization of polymer series **1**[a]

| Polymers | $MP_{Bu\ feed}$ (mol%)[b] | 0 | 10 | 20 | 30 | 40 | 50 | 60 |
|---|---|---|---|---|---|---|---|---|
| Boc-protected | $MP_{Bu}$(mol%) | 0 | 10 | 16 | 29 | 40 | 48 | 60 |
| | DP | 27 | 28 | 48 | 30 | 29 | 29 | 27 |
| | Yield (%) | 94 | 92 | 86 | 93 | 81 | 88 | 68 |
| Deprotected | $MP_{Bu}$(mol %) | 0 | 12 | 20 | 27 | 39 | 47 | 57 |
| | DP | 32 | 36 | 36 | 46 | 41 | 46 | 46 |

[a] [MMP]/[monomers] = 0.05 in polymerizations. [b]The percentage of butyl methacrylate relative to total amount of monomers in polymerizations.

**Table** 5-2. Characterization of polymer series **2**[a]

| Polymers | $MP_{Bu\ feed}$ (mol%)[b] | 0 | 10 | 20 | 30 | 40 | 50 | 60 |
|---|---|---|---|---|---|---|---|---|
| Boc-protected | $MP_{Bu}$ (mol %) | 0 | 12 | 20 | 29 | 37 | 47 | 55 |
| | DP | 16 | 17 | 17 | 18 | 17 | 19 | 22 |
| | Yield (%) | 99 | 87 | 78 | 75 | 87 | 74 | 54 |
| Deprotected | $MP_{Bu}$(mol %) | 0 | 12 | 20 | 28 | 37 | 45 | 53 |
| | DP | 20 | 19 | 21 | 24 | 23 | 22 | 31 |

[a.][MMP]/[monomers] = 0.10 in polymerizations.
[b.]The percentage of butyl methacrylate relative to total amount of monomers in polymerizations.

Synthetic procedures for amphiphilic polymethacrylate derivatives Polymer series 3: Polymer series of **3** was prepared by the same procedure as series **1** and **2** except Boc-protected polymers were purified by column chromatography using Sephadex LH-20 (Amersham Pharmacia Biotech AB) and methanol as an eluent to remove unreacted MMP and monomers because of difficulty in precipitation of polymers. During the precipitation procedure after Boc-deprotection, the polymers that have large DPs and low percentages of butyl groups selectively precipitated out from diethyl ether, and as a result, the collected polymers have percentages of butyl groups lower than 50% and larger DPs than those of the Boc-protected polymers. Characterization results are summarized in Table 5-3.

**Table 5-3.** Characterization of polymer series 3[a]

| Polymers | | | | | | | | | |
|---|---|---|---|---|---|---|---|---|---|
| Polymers | $MP_{Bu}$ feed (mol%)[b] | 0 | 10 | 20 | 30 | 35 | 40 | 45 |
| Boc-protected | $MP_{Bu}$ (mol %) | 0 | 10 | 17 | 23 | 34 | 37 | 41 |
| | DP | 4.3 | 4.1 | 4.8 | 5.6 | 5.4 | 6.4 | 5.9 |
| Deprotected | $MP_{Bu}$ (mol %) | 0 | 9 | 17 | 24 | 29 | 32 | 37 |
| | DP | 4.8 | 4.9 | 5.7 | 6.5 | 6.2 | 7.7 | 7.6 |

| Polymers | $MP_{Bu}$ feed (mol%)[b] | 50 | 55 | 60 | 65 |
|---|---|---|---|---|---|
| Boc-protected | $MP_{Bu}$ (mol %) | 48 | 51 | 58 | 52 |
| | DP | 6.8 | 6.2 | 5.9 | 6 |
| Deprotected | $MP_{Bu}$ (mol %) | 40 | 41 | 44 | 47 |
| | DP | 8.6 | 8.6 | 8.7 | 8.5 |

[a] [MMP]/[monomers] = 0.50 in polymerizations
[b] The percentage of butyl methacrylate relative to total amount of monomers in polymerizations.

Molecular weights of polymers were calculated using the data of $MP_{Bu}$ and DP with molecular weights of monomers and MMP and are plotted as a function of $MP_{Bu}$ in Figure S-1.

Samples of the *n*-butyl methacrylate copolymers (copolymers 9) were assayed for antimicrobial and hemolytic activity as described in Example 1 and below. Solubility limits were also determined for each copolymer series as described below.

Antimicrobial assays: Each polymer was dissolved in DMSO (5mg/mL). This solution was mixed with water to make a series of stock solutions of 2-fold dilution. Solutions containing DMSO without polymers were prepared as a control. The bacterial strain *Escherichia coli* D31 (ampicillin- and streptomycin-resistant) was grown in Mueller-Hinton broth (MH broth) at 37°C overnight, and the bacterial growth was measured by turbidity as optical density at $\lambda$ = 600 ($OD_{600}$) in a 1mL plastic disposable cuvette (1cm path length) using an Eppendorf BioPhotometer. This cell culture was diluted with MH broth to give a cell suspension (20mL) with $OD_{600}$ = 0.1, which was incubated at 37°C for 1.5 hours. Healthy cell growth was confirmed by measuring $OD_{600}$ (valued between 0.5 and 0.6), and this cell suspension was further diluted to give a bacterial assay stock with $OD_{600}$ = 0.001. This *E. coli* stock (90μL) was added to each well in a 96 well sterile assay palate (Coster Clear Polystyrene 3370, Corning). The polymer stock solutions (10μL) or the control solutions (10μL) were added to the well. The assay plate was incubated at 37°C for 18 hours. Bacterial growth was detected at $OD_{595}$ using ThermoLabsystems Multiskan Spectrum and was compared to that of MH broth without polymers and *E. coli.* All assays were carried out in triplicate in the same assay plate. MIC was defined as the lowest polymer concentration to completely inhibit bacterial growth in at least two samples of the triplicate measurements. The MIC reported in the article is the average value of 4 independent experiments performed from cell cultures prepared separately on different days.

Hemolysis Assays: Polymer stock solutions and control solutions were prepared by the same procedure as in antimicrobial testing. Freshly drawn human red blood cells (RBCs) were obtained by centrifuging a whole blood and removing plasma and white blood cells. The RBCs (1mL) was diluted with 9mL of TBS buffer (10mM Tris buffer, pH=7.0, 150mM NaCl) and this suspension was further diluted by a factor of 40 to give a RBC stock suspension (0.25% blood cells). This RBC stock (120μL), TBS buffer (15μL) and the polymer stock solutions (15μL) (or control solutions) were added to a 200μL centrifugation tube and incubated at 37°C for 1 hour. The tube was centrifuged at 4,000ppm for 5 minutes. Supernatant (30μL) was diluted with TBS buffer (100μL), and $OD_{414}$ of the solution was measured as hemoglobin concentration. Melittin was used as a positive control, and the most concentrated sample (100μg/mL) was used as a reference for 100% hemolysis. Control solutions containing serially decreasing amount of DMSO in the absence of polymers were used as a reference for 0% hemolysis. Percentage of hemolysis (P) was calculated from the equation:

$$P = [OD_{414}(polymer) - OD_{414}(control)] / [OD_{414}(melittin) - OD_{414}(control)]$$

$HC_{50}$ was obtained as the polymer concentration at 50% hemolysis, which was estimated by a curve fitting with the following equation:

$$P(C_p) = 100/[1+ (K/C_p)^n]$$

where $P(C_p)$ and K are a hemolysis curve for a given polymer concentration $(C_p)$ and $HC_{50}$ respectively. $K$ and n are variable parameters in the curve fitting. $HC_{50}$ is reported as the average value from 3 experiments independently performed on different days. See also Liu, D., and DeGrado, W.F., J. Amer. Chem. Soc. 123:7553-7559 (2001) and Kuroda, K, and DeGrado, W.F., J. Amer. Chem. Soc. 127:4128-4129 (2005)).

Solubility determinations: Polymer stock solutions prepared in antimicrobial testing or hemolysis assay were used. The polymer stock solutions (10μL) were added to assay media (90μL of MH broth or TBS buffer) in a 96-well plate and incubated at 37°C for 18 hours. Polymer precipitation was detected as turbidity at $OD_{595}$. Solubility limit was determined as the highest polymer concentration in a series, at which the $OD_{595}$ value of the polymer solution is same as that of a control. Solubility limit is reported as an average value of two independent experiments. In antimicrobial testing, MICs of the polymers with $MP_{Bu}$ higher than 40% in polymer series **1** could not be obtained due to turbidity caused by the precipitation of polymers. In the hemolysis assay medium (TBS buffer), no polymer precipitation was detected up to 500μg/mL (the most concentrated polymers in assays).

Table 6 presents the number-average molecular weight ("$M_n$") and number-average molecular weight range ("MW range") for each series of *n*-butyl methacrylate copolymers. Each copolymer was considered to have a distribution of molecular weights, which is generally expected for polymers synthesized by the process of free-radical polymerization. Figure 3 presents the molecular weights for each series of copolymers, which were calculated from the DP determined by NMR, plotted as a function of the percentage of butyl group of copolymer in each series.

**Table 6**. NW ranges and $M_n$ values for three series of n-butyl methacrylate copolymers

| Copolymer Series | MW range | $M_n$ |
|---|---|---|
| Series 1 (P-1-8.7K) | 7,900-10,100 | 8,700 |
| Series 2 (P-1-5K) | 4,500-6,000 | 5,000 |
| Series 3 (P-1-1.6K) | 1,300-1,900 | 1,600 |

Results of the antimicrobial assays are shown in Figure 4, in which the minimum inhibitory concentration ("MIC") of each copolymer is plotted as a function of the mole percent of copolymer hydrophobic group (butyl group). Figure 4 indicates that the MIC values decreased with increasing mole percentage of butyl group in each copolymer, leveling off at about 20-30 mole percent of butyl group for all three MW series of copolymers.

Copolymer precipitation in the antimicrobial assay media (Mueller-Hilton media) occurred above 20-30 mole percent of copolymer butyl groups, at copolymer concentrations of about 125-500 μg/mL. Figure 5 presents the solubility limit, in μg/mL, for each copolymer as a function of the mole percent of hydrophobic (butyl) group of the copolymer. Figure 5 indicates that those copolymers with lower molecular weights were more soluble in the assay media than copolymers with higher molecular weights. Figure 5 also indicates that copolymer solubility in the media decreased with increasing mole percentage of butyl group. This last result suggests that the hydrophobic polymers may aggregate or precipitate out of solution, especially those with hydrophobic mole percents of 30 or greater, those hydrophobic mole percent values at which copolymer MICs level off (see Figure 4).

Results of the hemolysis assays are presented in Figures 6A, 6B, and 6C, which also include antimicrobial assay data for comparison. Figures 6A-6C indicate that, in each of the three series of copolymers, the $HC_{50}$ for the copolymers decreased as the mole percentage of butyl groups ($MP_{Bu}$) increased. In the high $MP_{Bu}$ region (30-60%), the $HC_{50}$ values of the series of higher MW copolymers (series 2 and 1 (P-1-5K and P-1-8.7K)) reached a plateau of <1 μg/mL, which is lower than that of melittin (1.24 μg/mL). In contrast, the $HC_{50}$ values for lowest MW copolymer series (series 3 (P-1-1.6K)) decreased monotonically with increasing $MP_{Bu}$ and were about 1 order of magnitude higher relative to those of the two higher MW copolymer series for the same $MP_{Bu}$. This result provides a window of efficacy from about 10% to about 30% $MP_{Bu}$ in which the copolymers of series 3 are selectively toxic to bacterial cells with a maximum selectivity ($HC_{50}$/MIC) at about 17% $MP_{Bu}$ (FIG. 6D).

EXAMPLE 4

Antimicrobial and Hemolytic Activities of Four Series of Polymethacrylate Random Copolymers

The following four sets of random copolymers were synthesized and tested for antimicrobial and hemolytic activity:

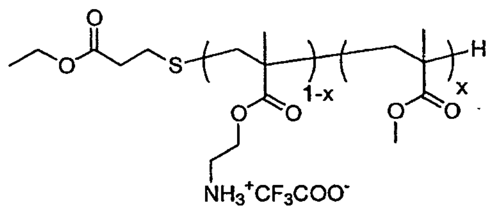

C-1 series

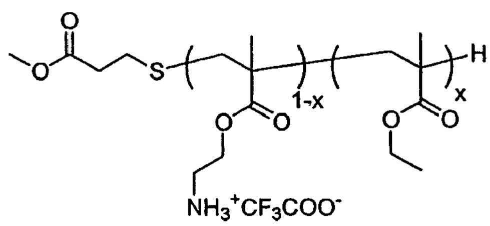

C-2 series

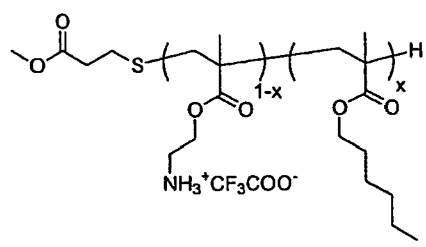

C-3 series

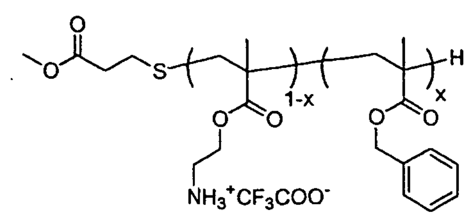

C-4 series

Each series comprises copolymers with varying mole percentages of hydrophobic R side chain ("X").

The random copolymers were synthesized using the general procedures described in Examples 1 and 2, and in Scheme 3 above, with the exception that, during synthesis of each set of copolymers, the concentration ratios of CTA to total monomer ([CTA]/[monomer]) used were 0.10, 0.20, and 0.30 (i.e., the copolymers in each set were polymerized in the presence of 10 mole percent, 20 mole percent, or 30 mole percent of CTA relative to the total amount of monomer). In addition, the C-1 series of copolymers were synthesized using ethyl 3-mercaptoproprionate as a chain transfer agent (CTA) instead of methyl 3- mercaptoproprionate. The remaining sets of copolymers (the C-2, C-3, and C-4 series) were synthesized using methyl 3-mercaptoproprionate as the CTA. Also, each series of copolymers were purified by precipitation rather than by column chromatography.

Number-average molecular weights were determined for each series of copolymers as described in Example 1. Figs. 7A, 8A, 9A and 10A present the number-average molecular weight determined for each series of copolymers plotted as a function of the mole percent of hydrophobic group (X (%)). Copolymers synthesized using a [CTA]/[monomer] ratio of 0.10, 0.20, or 0.30 are designated SH10, SH20, or SH30, respectively. Figs. 7A, 8A, 9A and 10A indicate that the number-average molecular weight of the copolymers decreases with increasing ratio of [CTA]/[monomer].

The copolymers were tested for antimicrobial and hemolytic activities as described in Examples **1** and 3 above and in Kuroda et al., J. Am. Chem. Soc. 127:4128-4129 (2005). The results are presented in Figs. 7B, 8B-8D, 9B-9D and 10B-10D. In the majority of Figs. 7B, 8B-8D, 9B-9D and 10B-10D, MIC values reach a plateau of approximately 16-8 μg/mL at hydrophobic group mole percentages of about 30-50 mol %, depending on the identity of the hydrophobic group. Figs. 7B, 8B-8D, 9B-9D and 10B-10D also indicate that the lower molecular weight copolymers having smaller hydrophobic groups (e.g., SH20 and SH30 copolymers of the C1 and C2 series) exhibit larger windows of efficacy (i.e., were selectively toxic to bacterial cells (*E.coli*) over mammalian cells (red blood cells)) compared with larger molecular weigh copolymers having larger hydrophobic groups (e.g., SH10 copolymers of the C3 and C4 series). This result suggests that the relative antimicrobial and hemolytic properties of the random copolymers of the present invention can be controlled by selecting the molecular weight and hydrophobic group of a the copolymer to produce antimicrobial copolymers that are non-toxic to mammals.

EXAMPLE 5

Synthesis and Characterization of Cationic Random Copolymers with Tertiary and Quarternary Amine Groups

Two series of polymethacrylate random copolymers with tertiary or quaternary amine groups were synthesized as described below and tested for antimicrobial activity as described in Examples 1 and 3 above.

The following two series of cationic copolymers (P-DMA and P-Q series) were synthesized as follows:

Synthesis of cationic random copolymers P-DMA and P-Q

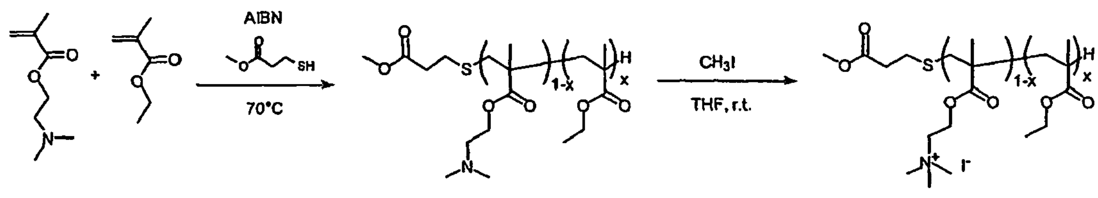


P-DMA

P-Q

Scheme 5

<u>Synthesis of P-DMA</u>: *N*-dimethylaminoethyl methacrylate and ethyl methacrylate (3.18 mmol total) were mixed with AIBN (5.2 mg, 31.8 μmol) and methyl 3-mercaptopropionate (MMP) (0.115 mL, 103 μmol) as a chain transfer agent and purged with Ar for 2 minutes. The reaction mixture was stirred at 70°C overnight. The resultant polymers were diluted with methanol and purified by column chromatography using Sephadex LH-20 (Amersham Pharmacia Biotech AB) and methanol as an eluent to remove unreacted MMP and monomers. The mole percentage of ethyl groups (X) for the polymers was calculated as described in Example 1 using the integration ratio of the signals from methylene protons of the monomer side chains and methyl protons of the polymer backbone in $^1H$ NMR spectra ($CDCl_3$). The degree of polymerization (DP) was determined by integration of the signals from methylene protons of the side chains relative to those from methyl protons of MMP at the polymer terminal.

<u>Synthesis of P-Q</u>: Methyl iodide (2.0M in t-butyl methyl ether) (0.5-0.1 mL, 3 eq to amino groups of the polymers) was added to the polymer (50 mg) in THF (1 mL) and stirred for 2 hour at room temperature. The resultant precipitate was separated by centrifugation, rinsed with THF and diethyl ether and dried under vacuum. The mole percentage of ethyl groups (X) for the polymers was calculated via integration ratio of the signals from methylene protons of the monomer side chains in $^1H$ NMR spectra ($D_2O$). The DP was determined by integration of the signals from methylene protons of the side chains relative to those from methyl protons of MMP at the polymer terminal.

The copolymers were tested for antimicrobial activity using the procedures described in Examples **1** and 3 above and in Kuroda et al., J. Am. Chem. Soc. 127:4128-4129 (2005). The results are presented in Table 7.

**Table 7**. Antimicrobial activities of cationic copolymers with tertiary or quaternary amines.

| Polymers | $X_{feed}$[a](%) | 0 | 10 | 20 | 40 | 60 | 80 |
|---|---|---|---|---|---|---|---|
| P-DMA | X(%) | 0 | 23 | 29 | 46 | 66 | 83 |
| | DP | 6.8 | 7.3 | 6.5 | 8.4 | 6.3 | 8.4 |
| | MIC (μg/mL) | >500 | >500 | 250 | 32 | 32 | [b] |
| P-Q | X(%) | 0 | 7 | 15 | 36 | 50 | 74 |
| | DP | - | 6.2 | 5.2 | 6.9 | 6.1 | 8.4 |
| | MIC (μg/mL) | 500 | 500 | >500 | >500 | 500 | 125 |

a) The mol % of ethyl groups during polymerizations
b) Not determined due to low solubility to aqueous solutions.

EXAMPLE 6

Antibacterial Activity of Polymethacrylate Random Copolymers Embedded in Polyurethane Films

The retention of antimicrobial activity of polymethacrylate random copolymers following incorporation in a polyurethane film is investigated. The retention of antimicrobial activity for one of the five series of polymethacrylate random copolymers of Example 2 is tested after the copolymers are incorporated into a sheet of polyurethane film.

For example, an untreated glass slide, untreated polyurethane film, and polyurethane film containing a sample of the lowest molecular-weight isobutyl methacrylate copolymers of Example 2 (MW approx. 2,000-1,000; (hydrophobic mole percentage = 40%) of Example 2)) are immersed in a culture of bacteria (*E. coli* D31) growing at 37°C for 72 hours. The isobutyl methacrylate copolymer-derivatized polyurethane is produced by swelling the film with solvent containing the copolymer and allowing it to dry. During this process the film is infiltrated with the isobutyl methacrylate copolymer. At the conclusion of the 62 hour incubation period, the untreated glass slide, untreated polyurethane film, and derivatized polyurethane film is inspected for bacterial growth. Little or no apparent growth of bacteria on the isobutyl methacrylate copolymer derivatized-polyurethane film indicates that the copolymer has retained its antibacterial activity when incorporated into the surface of the plastic film.

The experiment is repeated using *L. monocytogenes* in place of *E. coli.*

EXAMPLE 7

Antiviral Activity of Polymethacrylate Random Copolymers

One or more copolymers of the invention (e.g., the lowest molecular-weight hexyl methacrylate copolymers (MW approx. 2,000-1,000; hydrophobic mole percentage = 40%) of Example 2) are synthesized as described above and tested for their ability to inhibit HIV replication in cell culture. Two viruses are used in the infection assays: NLHX or YU2 that use CXCR4 or CCR5 as co-receptors, respectively. U87/CD4/CCR5 or U87/CD4/CXCR4 cells are seeded in 48-well plates at 3 x $10^4$ cells/well on the day prior to infection. Culture supernatants are removed from cells and replaced with pseudotyped luciferase reporter virus alone or pseudotyped luciferase reporter virus and copolymer at indicated final concentrations. Virus and compound are removed from cells approximately 16 hours post-infection, the cells are washed and then culture media was replenished. Cells are lysed and assayed for luciferase activity 3 days post infection. Results are presented as a percent of luciferase activity observed in the absence of copolymer.

EXAMPLE 8

Antifungal Activity of Polymethacrylate Random Copolymers

Several different genera of fungi are tested for their sensitivity to a set of polymethacrylate random copolymers of the present invention (e.g., the lowest molecular-weight butyl methacrylate copolymers (MW approx. 2,000-1,000; hydrophobic mole percentage = 50%) of Example 2). Both non-filamentous (yeast) and filamentous fungi are tested and specific fungi that are associated with various types of human infections are chosen for the screen (Table 8). One or more copolymers are tested for their antifungal activities. The antifungal assays are performed to determine the minimal inhibitory concentrations that result in complete inhibition of growth ($MIC_{100}$). All growth assays are done in a total of 1 ml volumes and growth is assessed by turbidity measurements. Additional antifungal assay conditions are described in Table 9.

**Table 8.** Clinical features of specific fungi.

| Organism | Clinical Features |
|---|---|
| **Yeast** | |
| *Candida albicans* ATCC 10231 | Mucosal infections (skin, GI, urinary tract, reproductive organs) |
| **Filamentous fungi** | |
| *Aspergillus fumigatus* ATCC 1028 | Allergic disease, sinusitis bronchopulmonary infections and systemic infections in immune-compromised individuals |
| *Cryptococcus neoformans* ATCC 24067 | Opportunistic pathogen causing systemic infections in immune-compromised individuals |
| *Trichophyton mentagrophytes* ATCC 9533 | Skin infections (dermatophytosis) |
| *Trichophyton rubrum* ATCC 10218 | Chronic infections of the skin and nails, most widely distributed dermatophyte |
| **Control** | |
| *E. coli* ATCC 25922 | Verify compound integrity and activity, verify assay conditions |

**Table 9.** Additional anti-fungal assay conditions

| **Method Conditions** | *Candida albicans* | *Aspergillus fumigatus* | *Cryptococcus neoformans* | *Trichophyton mentagrophytes* | *Trichophyton rubrum* | *E.coli* (ATCC 25922) |
|---|---|---|---|---|---|---|
| Culture Medium | Fluid Sabouraud Medium | Potatoe Dextrose Broth | Fluid Sabouraud Medium | Potatoe Dextrose Broth | Fluid Sabouraud Medium | Nutrient Broth |
| Incubation Time | 20 hours | 2 days | 2 days | 3 days | 3 days | 20 hours |
| Incubation Temp. | 37˚C | 28˚C | 37˚C | 28˚C | 28˚C | 37˚C |

EXAMPLE 9

Ability of Polymethacrylate Random Copolymers to Inhibit the Anticoagulation Effects of Low Molecular Weight Heparin

Several of the amphiphilic polymethacrylate random copolymers of the invention (e.g., the lowest molecular-weight butyl methacrylate copolymers (MW approx. 2,000-1,000; hydrophobic mole percentage = 50%) of Example 2) are synthesized and tested for their ability to inhibit the anticoagulation effects of heparin. It is assumed that heparin-neutralizing activity is largely dependent on the charge and charge distribution characteristics of the copolymers rather than on their hydrophobic qualities.

The delay in clotting times of activated plasma caused by a fixed concentration of heparin is tested in the presence of increasing concentrations of the copolymers. The clotting time of activated plasma in the presence of 1 unit (0.2 μg/ml) heparin is measured in the presence and absence of four concentrations of copolymer. The assay is performed four times for each concentration of copolymer and the average clotting time determined. Dose response data are collected.

An activated partial thromboplasin time assay (clotting assay) is used to determine clotting time. The assay is performed as follows: A plasma sample (0.1 mL) containing heparin or heparin and the copolymer to be tested is pipetted into a test cuvette and incubated at 37˚C for about 2 minutes. Reconstituted Cephalinex (a phospholipids platelet substitute which can be obtained from Bio/Data Corporation) (0.1 mL) is added to the plasma sample. The mixture is

incubated at 37˚C for about 5 minutes. A 25 mM calcium chloride solution, prewarmed to 37˚C, is added (0.1 mL) to the mixture, and clotting time is recorded using a fibrometer.

Antagonism of the delay in clotting time caused by low molecular weight heparin (LMWH) is also investigated. The clotting time of activated plasma in the presence of 4.6 μg/ml LMWH is measured in the absence and presence of three concentrations of copolymer. Dose response data are collected.

The LMWH-antagonizing activity of the copolymers are also investigated by measuring the delay in clotting time in whole blood induced by three different concentrations of LMWH (LeoPharm, 1 μg/ml) in the presence or absence of one or more concentrations of copolymer. The performance of assays in whole blood are carried out in consideration of pharmaceutical applications, because such assays indicate whether potential serum protein binding by the copolymer that could impact biological activity *in vivo* is an issue. The assays are performed similarly to assays employing activated plasma and dose response data are collected.

Having now fully described this invention, it will be understood to those of ordinary skill in the art that the same can be performed within a wide and equivalent range of conditions, formulations, and other parameters without affecting the scope of the invention or any embodiment thereof.

All documents, e.g., scientific publications, patents, patent applications and patent publications, recited herein are hereby incorporated by reference in their entirety to the same extent as if each individual document was specifically and individually indicated to be incorporated by reference in its entirety. Where the document cited only provides the first page of the document, the entire document is intended, including the remaining pages of the document.

## Claims

**1.** A random copolymer of Formula III:

$$\mathbf{A\text{-}(B)_{n1}\text{-}(D)_{m1}\text{-}H} \qquad \mathbf{III}$$

or an acceptable salt or solvate thereof, wherein:

A is the residue of a chain transfer agent;
B is $-[CH_2-C(R^{11})(B_{11})]-$, wherein $B_{11}$ is $-X_{11}-Y_{11}-Z_{11}$, and
$X_{11}$ is carbonyl (-C(=O)-) or optionally substituted $C_{1-6}$ alkylene; or $X_{11}$ is absent;
$Y_{11}$ is O, NH, or optionally substituted $C_{1-6}$ alkylene; or $Y_{11}$ is absent;
$Z_{11}$ is $-Z_{11A}-Z_{11B}$, wherein:
$Z_{11A}$ is alkylene, arylene, or heteroarylene, any of which is optionally substituted; or $Z_{11A}$ is absent;
$Z_{11B}$ is -guanidino, -amidino, $-N(R^3)(R^4)$ or $-N^+(R^3)(R^4)(R^5)$, wherein $R^3$, $R^4$, and $R^5$ are independently hydrogen, alkyl, aminoalkyl, aryl, heteroaryl, heterocyclic, or aralkyl; or
$Z_{11}$ is pyridinium

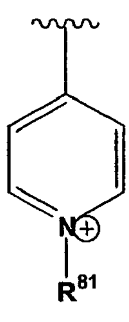

,

or phosphonium

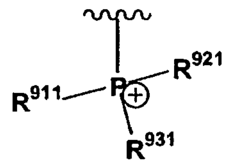

wherein $R^{81}$, $R^{911}$, $R^{921}$, and $R^{931}$ are independently hydrogen or alkyl;
D is $-[CH_2-C(R^{21})(D_{21})]-$, wherein $D_{21}$ is $-X_{21}-Y_{21}-Z_{21}$, and
$X_{21}$ is carbonyl (-C(=O)-) or optionally substituted $C_{1-6}$ alkylene; or $X_{21}$ is absent;
$Y_2$, is O, NH, or optionally substituted $C_{1-6}$ alkylene, or $Y_{21}$ is absent;
$Z_{21}$ is alkyl, cycloalkyl, alkoxy, aryl, or aralkyl, any of which is optionally substituted;
$R^{11}$ and $R^{21}$ are independently hydrogen or $C_{1-4}$ alkyl;
$m_1$, the mole fraction of D monomer, is about 0.1 to about 0.9;
and
$n_1$, the mole fraction of B monomer, is $1-m_1$;
wherein the copolymer is a random copolymer of B and D monomers, and
wherein the copolymer has a degree of polymerization of about 5 to about 50.

**2.** The random copolymer of claim 1, wherein A is $C_{1-4}$ alkoxycarbonyl($C_{1-4}$)alkylthio.

**3.** The random copolymer of claim 2, wherein:

$X_{11}$ and $X_{21}$ are carbonyl;
$Y_{11}$ and $Y_{21}$ are O;
$Z_{11}$ is $-Z_{11A}-Z_{11B}$, wherein $Z_{11A}$ is $C_{1-6}$ alkylene optionally substituted with $C_{1-4}$ alkyl or aryl; and $Z_{11B}$ is $-N(R^{31})(R^{41})$ or $-N^+(R^{31})(R^{41})(R^{51})$, wherein $R^{31}$, $R^{41}$, and $R^{51}$ are independently hydrogen, $C_{1-4}$ alkyl, amino($C_{1-4}$)alkyl, $C_{1-6}$ aryl, or $C_{1-6}$ ar($C_{1-4}$)alkyl;
$Z_{21}$ is $C_{1-6}$ alkyl, $C_{1-6}$ aryl, or $C_{1-6}$ ar($C_{1-4}$)alkyl; and
$R^{11}$ and $R^{21}$ are independently hydrogen or methyl;

wherein the copolymer has a degree of polymerization of about 5 to about 25.

**4.** The random copolymer of claim 3, wherein $Z_{11A}$ is $C_{1-4}$ alkylene optionally substituted with methyl or ethyl; and $R^{31}$, $R^{41}$, and $R^{51}$ are independently hydrogen or methyl.

**5.** The random copolymer of claim 2, wherein $Z_{11}$ is pyridinium

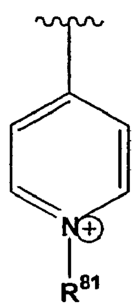

,

or phosphonium

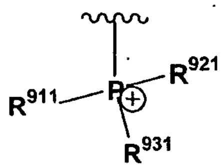

wherein $R^{81}$, $R^{911}$, $R^{921}$, and $R^{931}$ are independently hydrogen or $C_{1-4}$ alkyl.

**6.** The random copolymer of claim 2, wherein:

$X_2$ is carbonyl or optionally substituted $C_{1-4}$ alkylene;
$Y_{21}$ is O or NH; and
$Z_{21}$ is $C_{1-6}$ alkyl, $C_{1-6}$ aryl, or $C_{1-6}$ ar($C_{1-4}$)alkyl.

**7.** The copolymer of claim 6, wherein $Z_{21}$ is methyl, ethyl, *n*-butyl, isobutyl, hexyl, or benzyl.

**8.** The random copolymer of claim 1, wherein $m_1$ is about 0.35 to about 0.60.

**9.** The random copolymer of claim 1, wherein the degree of polymerization (DP) is about 5 to about 10.

**10.** The random copolymer of claim 1, wherein the random copolymer is of Formula IIIa:

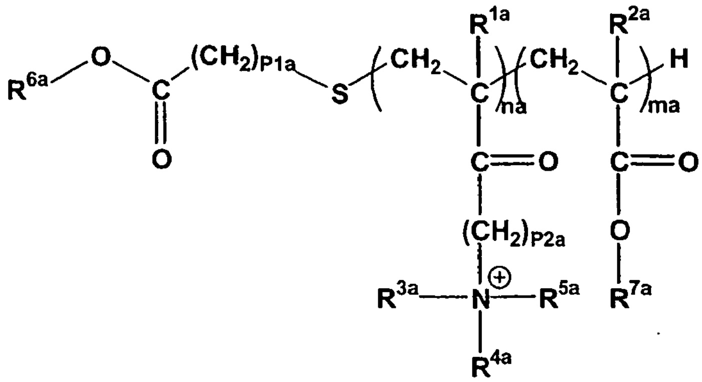

**IIIa**

or an acceptable salt or solvate thereof, wherein:

$R^{1a}$ and $R^{2a}$ are independently hydrogen or methyl;
$R^{3a}$, $R^{4a}$, and $R^{5a}$ are independently hydrogen or $C_{1-4}$ alkyl;
$R^{6a}$ is $C_{1-4}$ alkyl;
$R^{7a}$ is $C_{1-10}$ alkyl, $C_{1-6}$ aryl, or $C_{1-6}$ ar($C_{1-4}$)alkyl;
$p_{1a}$ is 1 to 4;
$p_{2a}$ is 1 to 6;and
$m_a$ is about 0.35 to about 0.55; and $n_a$ is 1-$m_a$;
wherein the copolymer has a degree of polymerization of about 5 to about 25.

**11.** The random copolymer of claim 10, wherein:

$R^{1a}$ and $R^{2a}$ are independently methyl;
$R^{3a}$, $R^{4a}$, and $R^{5a}$ are independently hydrogen or methyl;
$R^{6a}$ is methyl or ethyl;
$R^{7a}$ is $C_{1-4}$ alkyl, e.g., methyl, ethyl, propyl, or butyl;
$p_{1a}$ is 1 or 2;

$p_{2a}$ is 1, 2 or 3;
$m_a$ is about 0.35 to about 0.55; and $n_a$ is 1-$m_a$.

**12.** The random copolymer of claim 10, wherein the random copolymer is of Formula IIIb:

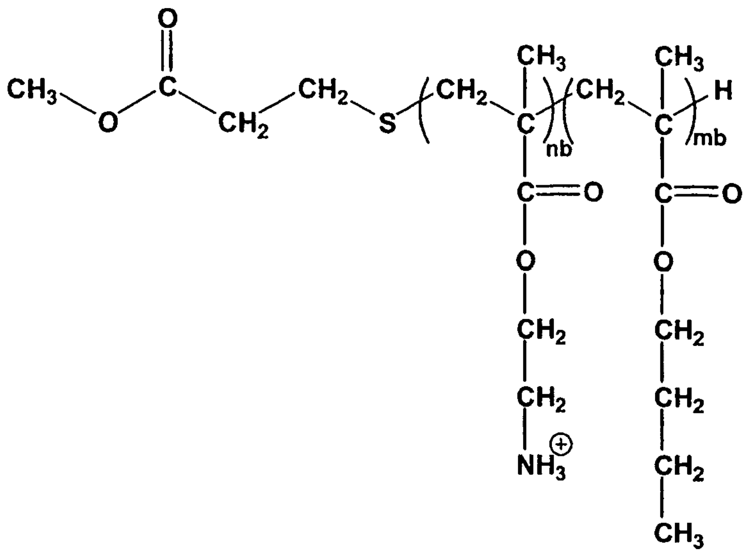

IIIb

or an acceptable salt or solvate thereof, wherein:

$m_b$ is about 0.45 to about 0.55; $n_b$ is 1-$m_b$; and
the degree of polymerization is about 5 to about 10.

**13.** The random copolymer of claim 10, wherein the random copolymer is of Formula IIIc:

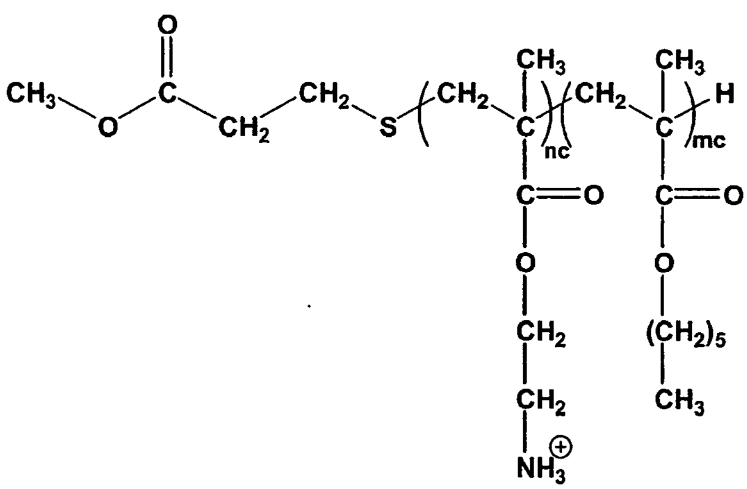

IIIc

or an acceptable salt or solvate thereof, wherein:

$m_c$ is about 0.35 to about 0.45; $n_c$ is 1-$m_c$; and
the degree of polymerization is about 5 to about 10.

**14.** The random copolymer of claim 10, wherein the random copolymer is of Formula IIId:

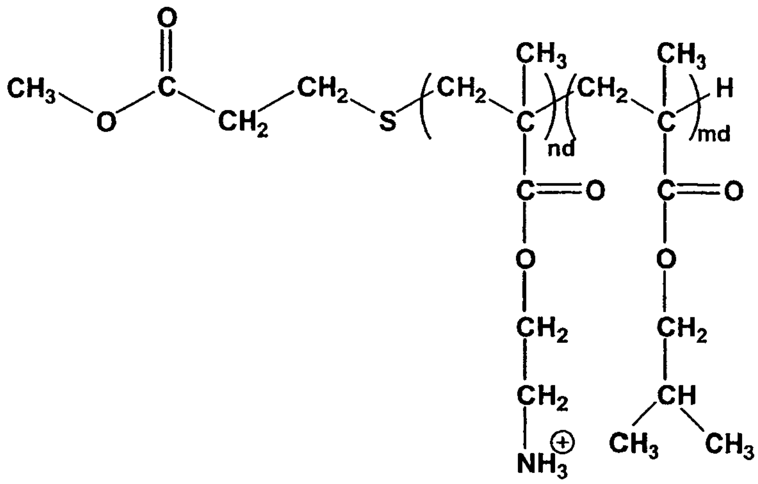

IIId

or an acceptable salt or solvate thereof, wherein:

$m_d$ is about 0.45 to about 0.55; $n_d$ is 1-$m_d$; and
the degree of polymerization is about 5 to about 10.

**15.** The random copolymer of claim 10, wherein the random copolymer is of Formula IIIe:

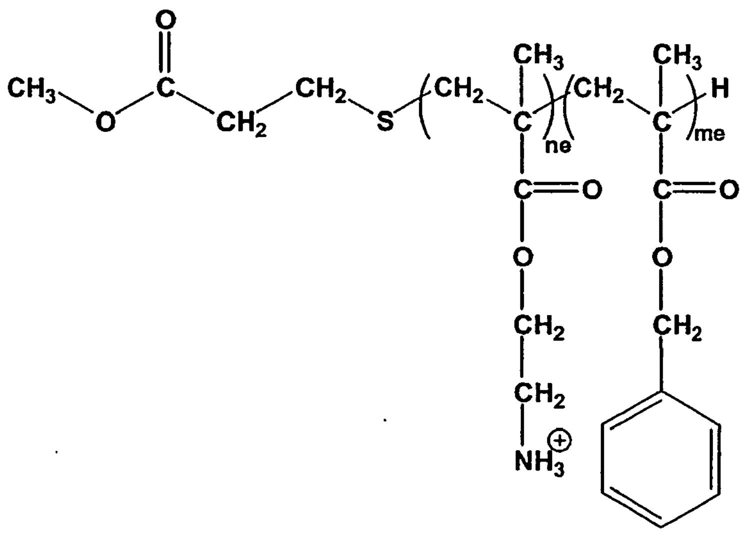

IIIe

or an acceptable salt or solvate thereof, wherein:

$m_e$ is about 0.50 to about 0.60; $n_e$ is 1-$m_e$; and
the degree of polymerization is about 5 to about 10.

**16.** A pharmaceutical composition comprising the random copolymer of claim 1 and a pharmaceutically acceptable carrier or diluent.

**17.** A method of treating a microbial infection in an animal in need thereof, said method comprising administering to the animal an effective amount of the pharmaceutical composition of claim 16.

**18.** The method of claim 17, wherein the microbial infection is an oral infection.

**19.** The method of claim 17, wherein the microbial infection is a bacterial infection, a fungal infection, or a viral infection.

**20.** A method of killing or inhibiting the growth of a microorganism, said method comprising contacting the microorganism witch an effective amount of the random copolymer of claim 1.

**21.** The method of claim 20, wherein the microorganism is a bacterial cell, a fungus, or a virus.

**22.** The method of claim 20, wherein the random copolymer is present on a substrate.

**23.** The method of claim 22, wherein the random copolymer is covalently attached to the substrate.

**24.** The method of claim 22, wherein the substrate is selected from the group consisting of wood, synthetic polymers, natural fibers, synthetic fibers, cloth, paper, rubber and glass.

**25.** An antimicrobial composition comprising the copolymer of claim 1 and a composition selected from the group consisting of paints, lacquers, coatings, varnishes, caulks, grouts, adhesives, resins, films, cosmetics, soaps, lotions, hand washes, and detergents.

**26.** A method of providing an antidote to low molecular weight heparin overdose in an animal, said method comprising administering to the animal the pharmaceutical composition of claim 16.

**27.** A random copolymer having a monomer unit of Formula **I**:

$$-[CH_2-C(R^1)(B_1)]_n- \quad \textbf{I}$$

wherein:

$R^1$ is hydrogen or $C_{1-4}$ alkyl;
$B_1$ is $-X_1-Y_1-Z_1$, wherein:
$X_1$ is carbonyl (-C(=O)-) or optionally substituted $C_{1-6}$ alkylene;
or $X_1$ is absent;
$Y_1$ is O, NH, or optionally substituted $C_{1-6}$ alkylene; or $Y_1$ is absent;
$Z_1$ is $-Z_{1A}-Z_{1B}$, wherein:
$Z_{1A}$ is alkylene, arylene, or heteroarylene, any of which is optionally substituted; or $Z_{1A}$ is absent;
$Z_{1B}$ is -guanidino, -amidino, $-N(R^3)(R^4)$ or $-N^+(R^3)(R^4)(R^5)$, wherein $R^3$, $R^4$, and $R^5$ are independently hydrogen, alkyl, aminoalkyl, aryl, heteroaryl, heterocyclic, or aralkyl; or
$Z_1$ is pyridinium

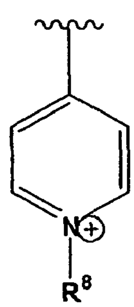

,

or phosphonium

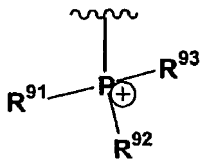

,

wherein $R^8$, $R^{91}$, $R^{92}$, and $R^{93}$ are independently hydrogen or alkyl;
n is 1-m, wherein m is as defined below;
and a monomer unit of Formula **II**:

$$-[CH_2\text{-}C(R^2)(D_2)]_m \qquad \textbf{II}$$

or an acceptable salt or solvate thereof,

wherein:

$R^2$ is hydrogen or $C_{1-4}$ alkyl;
$D_2$ is $-X_2-Y_2-Z_2$, wherein
$X_2$ is carbonyl (-C(=O)-) or optionally substituted $C_{1-6}$ alkylene, or $X_2$ is absent;
$Y_2$ is O, NH, or optionally substituted $C_{1-6}$ alkylene, or $Y_2$ is absent;
$Z_2$ is alkyl, cycloalkyl, alkoxy, aryl, or aralkyl, any of which is optionally substituted; and
m is about 0.1 to about 0.9;

wherein the copolymer has a degree of polymerization of about 5 to about 50; and
wherein a chain transfer agent A is used to control the degree of polymerization of the random copolymer.

**28.** The random copolymer of claim 27, wherein the chain transfer agent A is

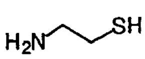

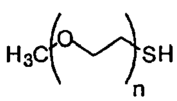

n=3-120

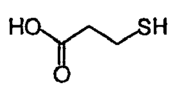

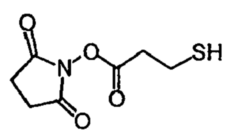

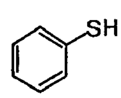

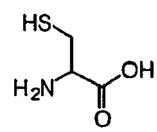

Cys

or an alkoxycarbonylalkylthiol.

**29.** The random copolymer of claim 28, wherein the chain transfer agent A is

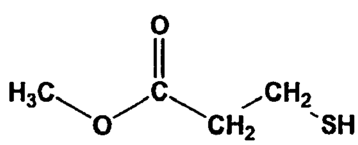

| | |
|---|---|
| Initiation:<br>$I \xrightarrow{k_I} I^\bullet$ | 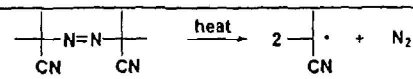 (1) |
| $I^\bullet + M \xrightarrow{k_I'} I\text{-}M^\bullet$ | 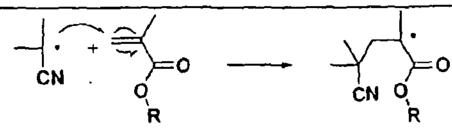 (2) |
| Propagation:<br>$I\text{-}M^\bullet + M \rightarrow I\text{-}M\text{-}M^\bullet \xrightarrow[+M]{} P^\bullet$<br>$P^\bullet + M \xrightarrow{k_p} P^\bullet$ | (3)<br>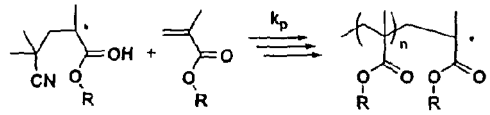 (4) |
| Termination:<br>Radical-radical coupling<br>$P^\bullet + P^\bullet \xrightarrow{k_c} P\text{-}P$ | 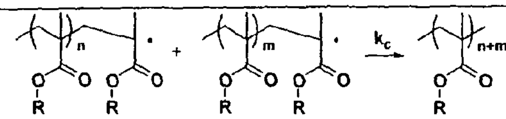 (5) |
| Disproportionation<br>$P^\bullet + P^\bullet \xrightarrow{k_d} P + P$ | 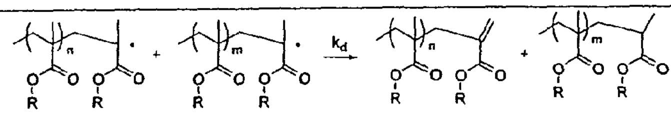 (6) |
| Chain Transfer<br>$P^\bullet + SX \xrightarrow{k_{trans}} P\text{-}X + S^\bullet$ | 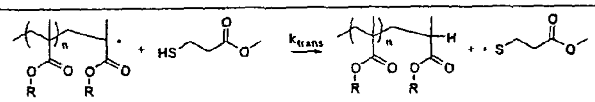 (7) |
| $S^\bullet + M \xrightarrow{k_p} P^\bullet$ | 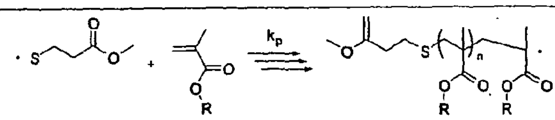 (8) |

**FIG. 1A**

$$\text{Polymer length} = DP = \frac{\text{Propagation}}{\text{Termination of radicals}} \tag{9}$$

$$= \frac{k_p[P^\bullet][M]}{k_c[P^\bullet]^2 + k_d[P]^2 \quad + k_{trans}[P^\bullet][SX]} \tag{10}$$

$$\frac{1}{DP} = \frac{k_c[P^\bullet]^2 + k_d[P]^2 \quad + k_{trans}[P^\bullet][SX]}{k_p[P^\bullet][M]} \tag{11}$$

$$= \frac{k_c[P^\bullet]^2 + k_d[P]^2}{k_p[P^\bullet][M]} + \frac{k_{trans}[P^\bullet][SX]}{k_p[P^\bullet][M]} \tag{12}$$

$$= \frac{1}{DP_0} + C\frac{[SX]}{[M]} \qquad C = \frac{k_{trans}}{k_p} \tag{13}$$

**FIG. 1B**

# FIG. 2

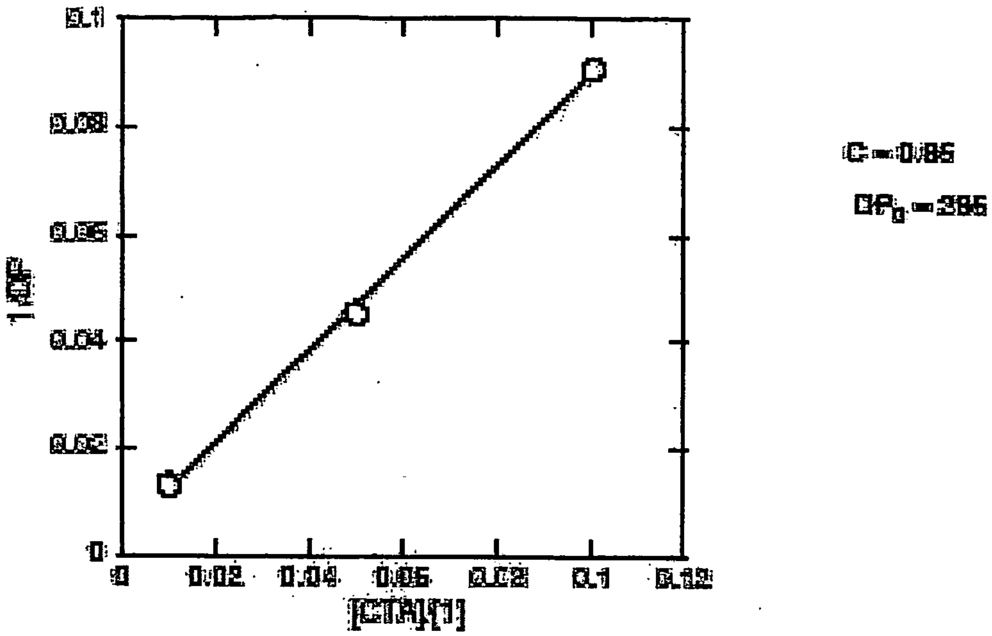

FIG. 3

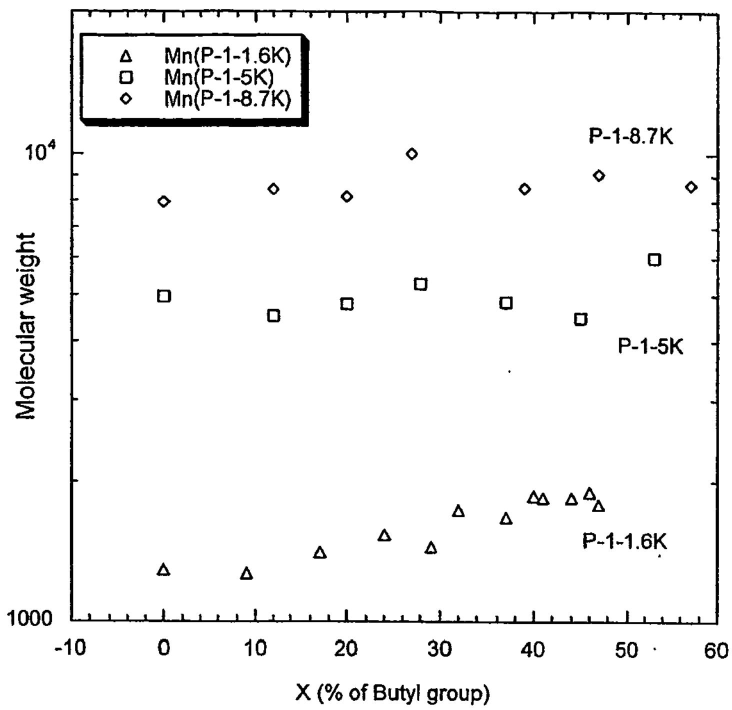
Mn(P-1-1.6K)
Mn(P-1-5K)
Mn(P-1-8.7K)
P-1-8.7K
P-1-5K
P-1-1.6K
Molecular weight
$10^4$
1000
-10
0
10
20
30
40
50
60
X (% of Butyl group)

# FIG. 4

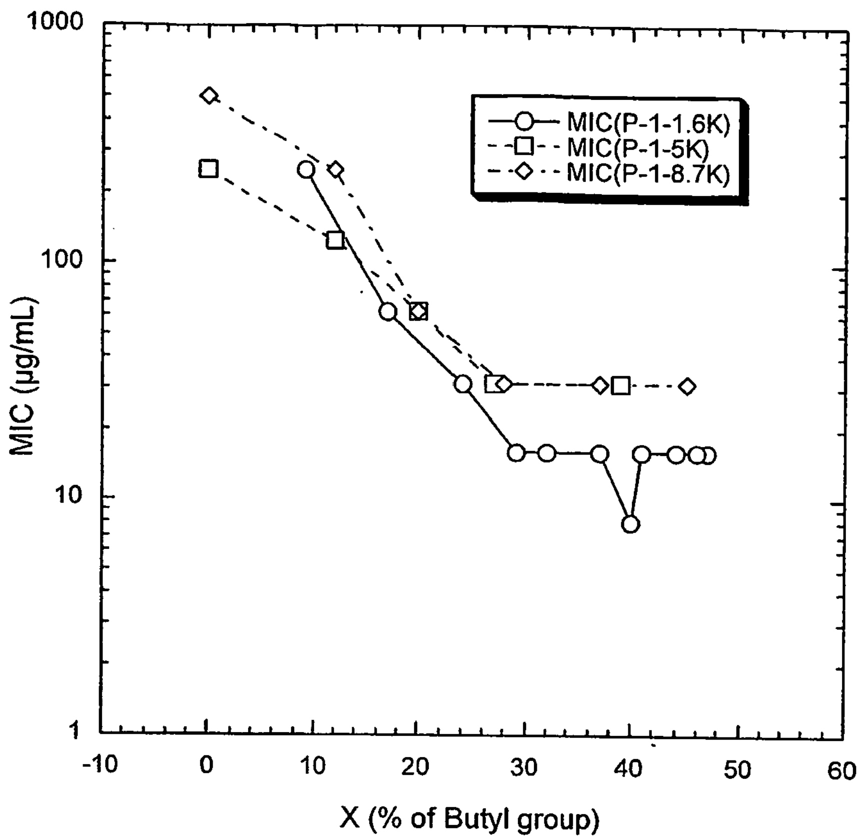
1000
100
10
1
MIC (μg/mL)
-10
0
10
20
30
40
50
60
X (% of Butyl group)
MIC(P-1-1.6K)
MIC(P-1-5K)
MIC(P-1-8.7K)

# FIG. 5

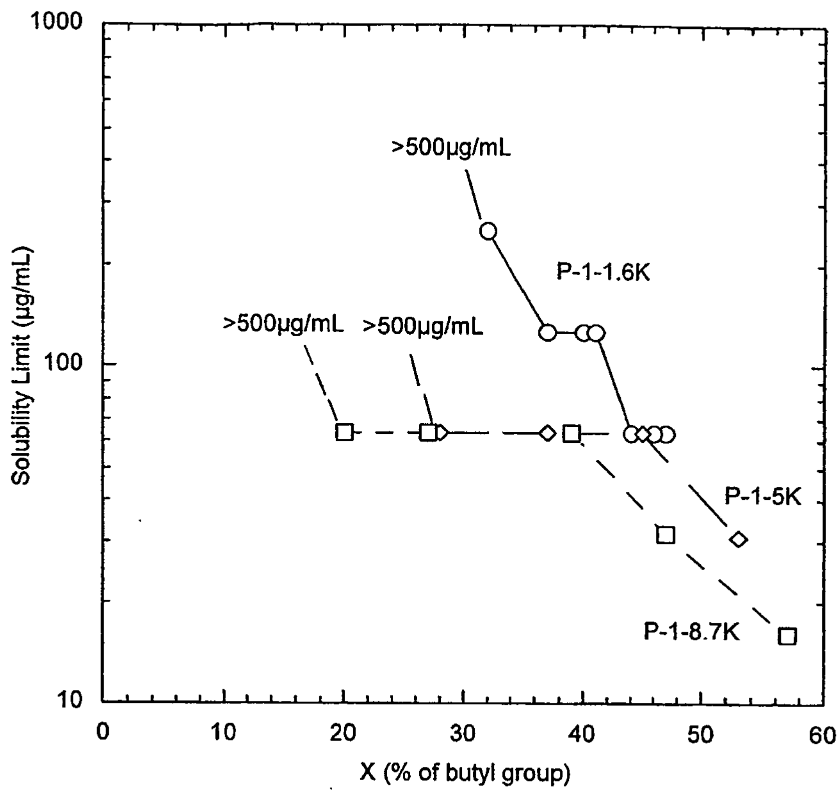
1000
100
10
Solubility Limit (µg/mL)
0
10
20
30
40
50
60
X (% of butyl group)
>500µg/mL
P-1-1.6K
>500µg/mL
>500µg/mL
P-1-5K
P-1-8.7K

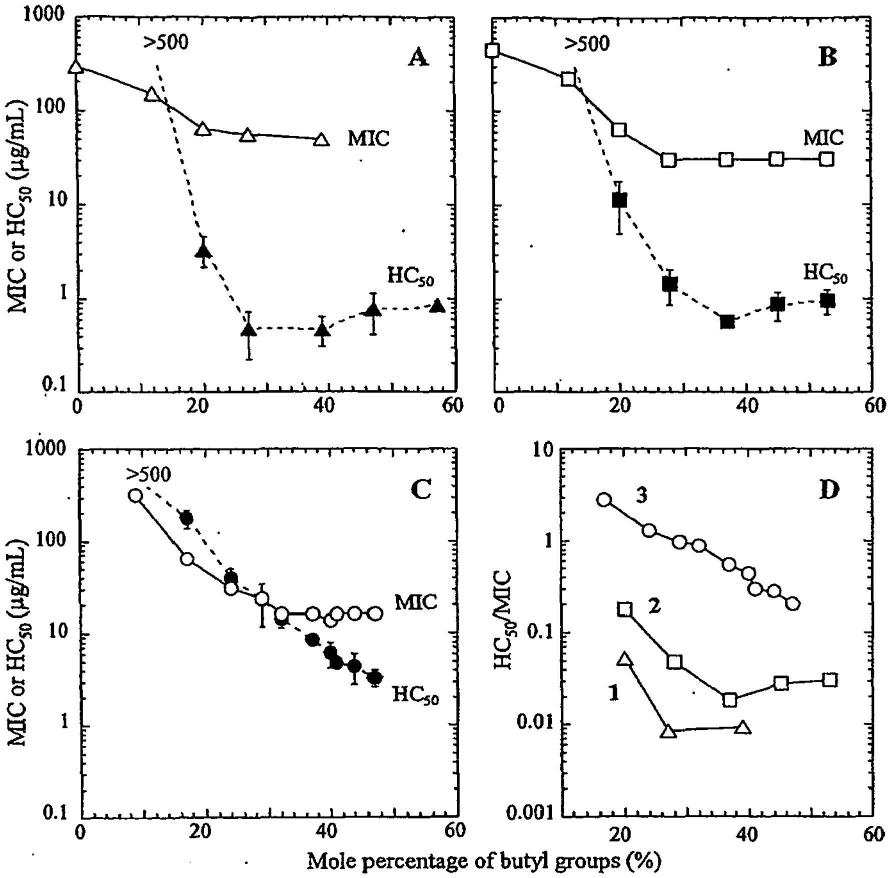

1000
100
10
1
0.1
MIC or $HC_{50}$ (μg/mL)
>500
A
MIC
$HC_{50}$
0
20
40
60
>500
B
MIC
$HC_{50}$
C
MIC
$HC_{50}$
D
$HC_{50}$/MIC
3
2
1
0.01
0.001
Mole percentage of butyl groups (%)


# FIGs. 6A-6D

# FIG. 7A

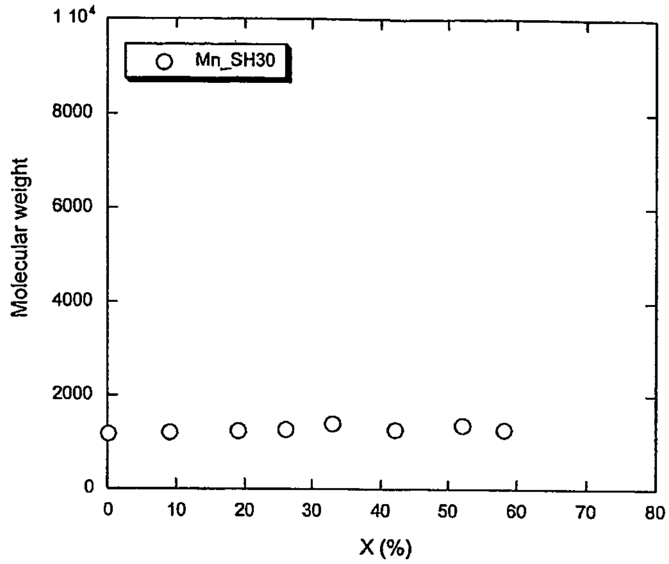

1 10^4
8000
6000
4000
2000
0
Molecular weight
Mn_SH30
0
10
20
30
40
50
60
70
80
X (%)


# FIG. 7B

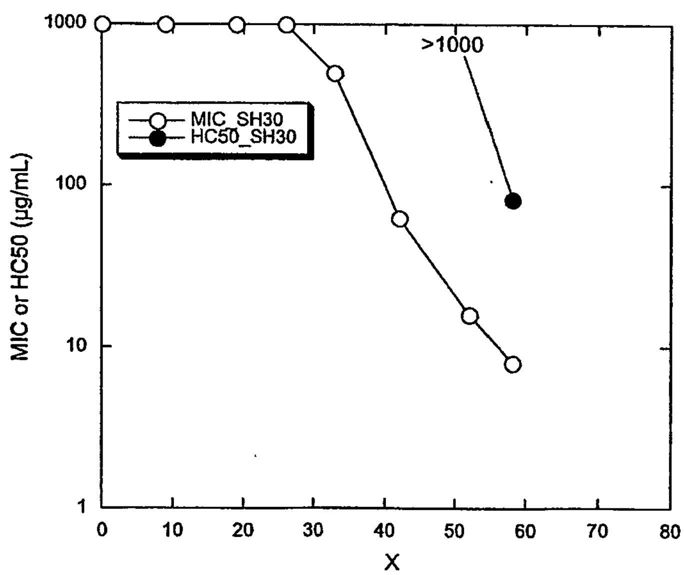

1000
100
10
1
MIC or HC50 (µg/mL)
>1000
MIC_SH30
HC50_SH30
0
10
20
30
40
50
60
70
80
X

# FIG. 8A

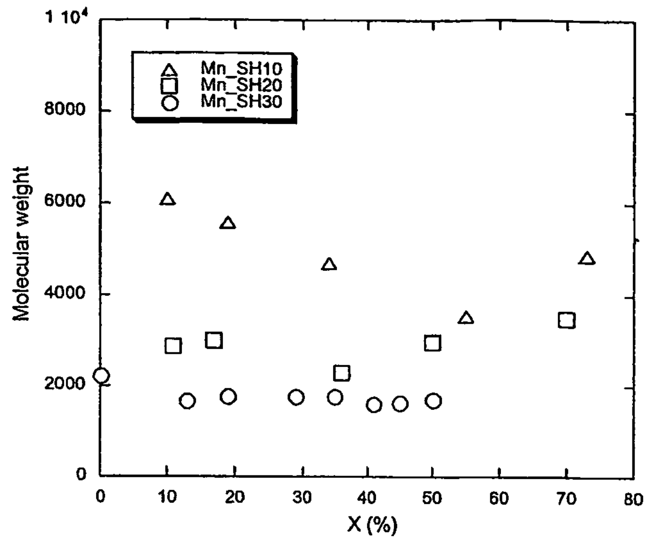
1 10⁴
8000
6000
4000
2000
0
Molecular weight
Mn_SH10
Mn_SH20
Mn_SH30
0
10
20
30
40
50
60
70
80
X (%)

# FIG. 8B

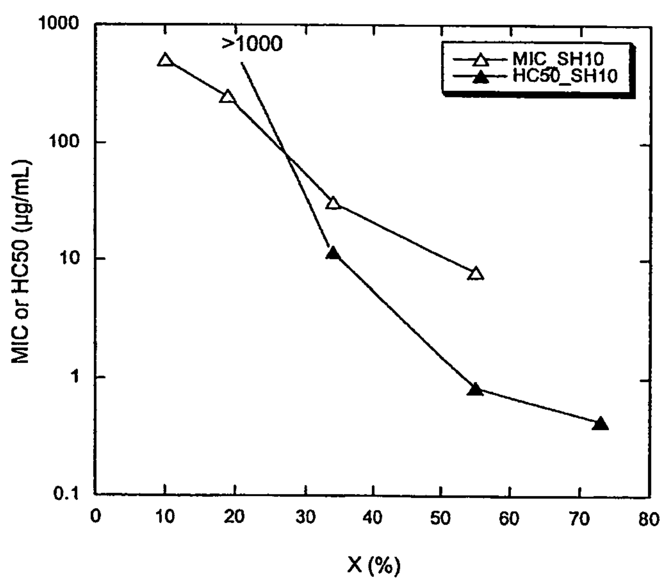
1000
100
10
1
0.1
MIC or HC50 (µg/mL)
>1000
MIC_SH10
HC50_SH10
0
10
20
30
40
50
60
70
80
X (%)

# FIG. 8C

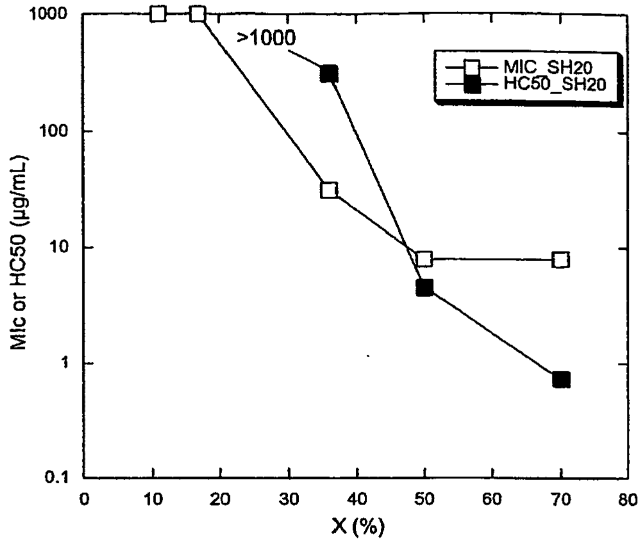
1000
100
10
1
0.1
MIc or HC50 (µg/mL)
>1000
MIC_SH20
HC50_SH20
0
10
20
30
40
50
60
70
80
X (%)

# FIG. 8D

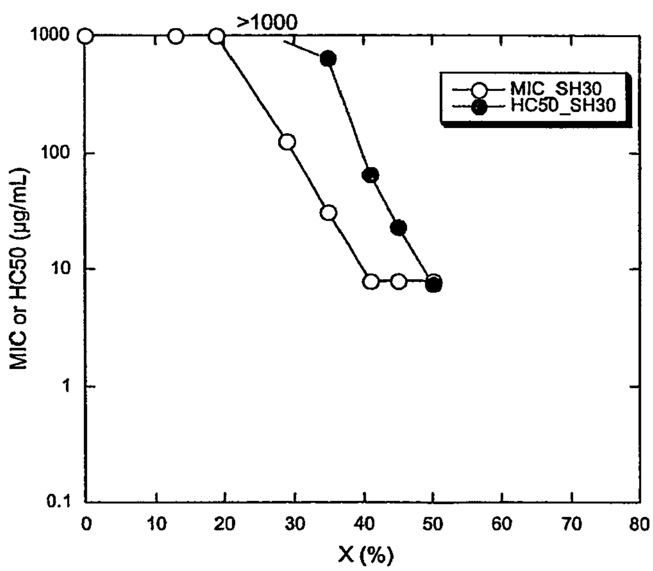
1000
100
10
1
0.1
MIC or HC50 (µg/mL)
>1000
MIC_SH30
HC50_SH30
0
10
20
30
40
50
60
70
80
X (%)

FIG. 9A

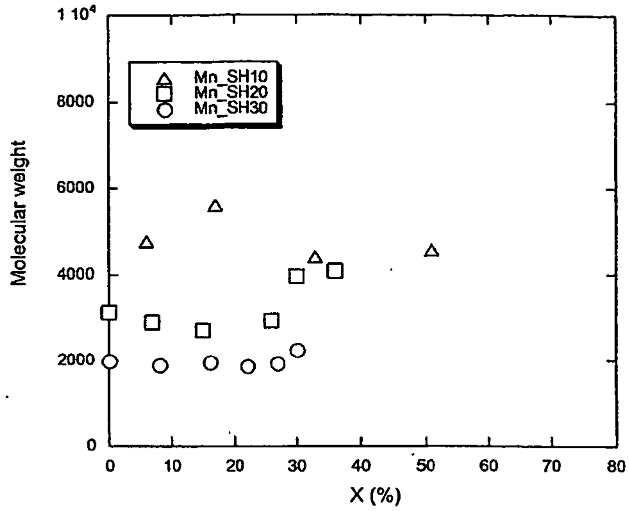
1 10^4
8000
6000
4000
2000
0
Molecular weight
Mn_SH10
Mn_SH20
Mn_SH30
0
10
20
30
40
50
60
70
80
X (%)

FIG. 9B

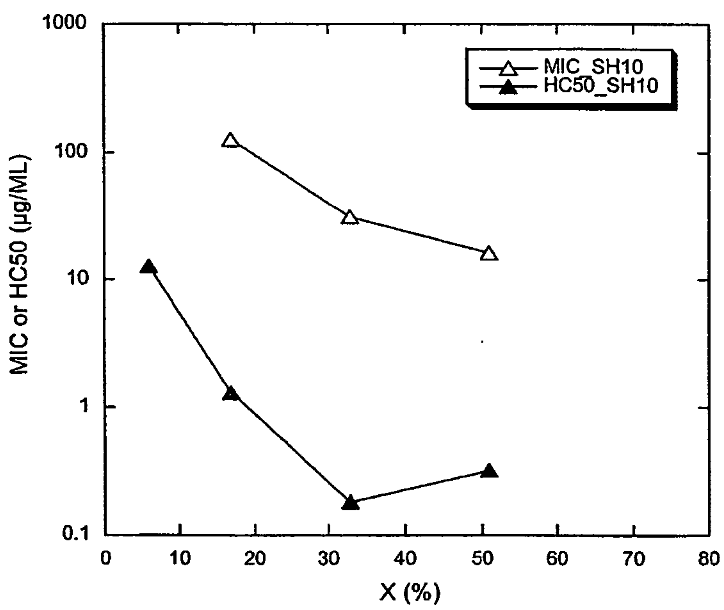
1000
100
10
1
0.1
MIC or HC50 (μg/ML)
MIC_SH10
HC50_SH10
0
10
20
30
40
50
60
70
80
X (%)

# FIG. 9C

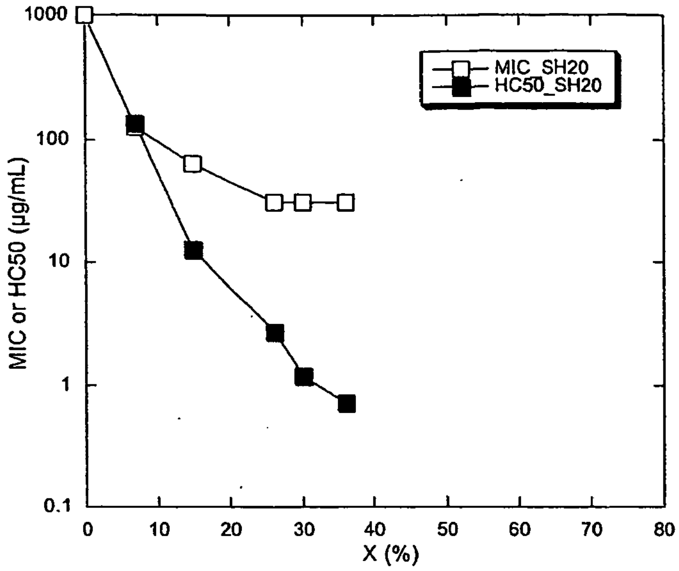
1000
100
10
1
0.1
MIC or HC50 (µg/mL)
0
10
20
30
40
50
60
70
80
X (%)
MIC_SH20
HC50_SH20

# FIG. 9D

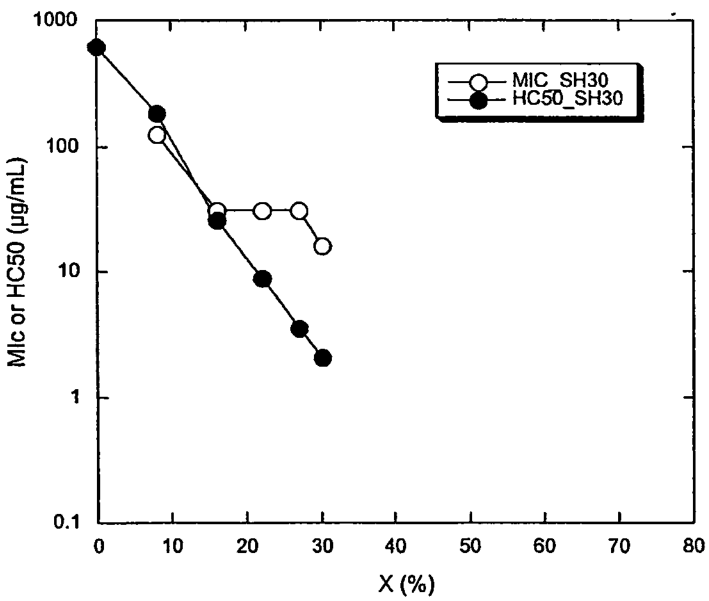
1000
100
10
1
0.1
MIc or HC50 (µg/mL)
0
10
20
30
40
50
60
70
80
X (%)
MIC_SH30
HC50_SH30

# FIG. 10A

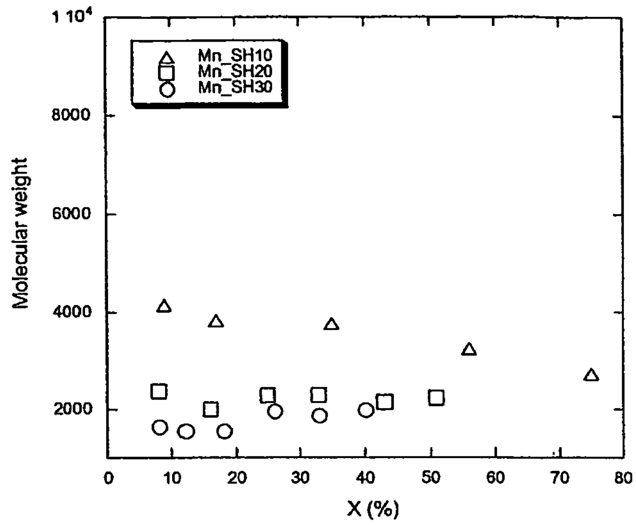


# FIG. 10B

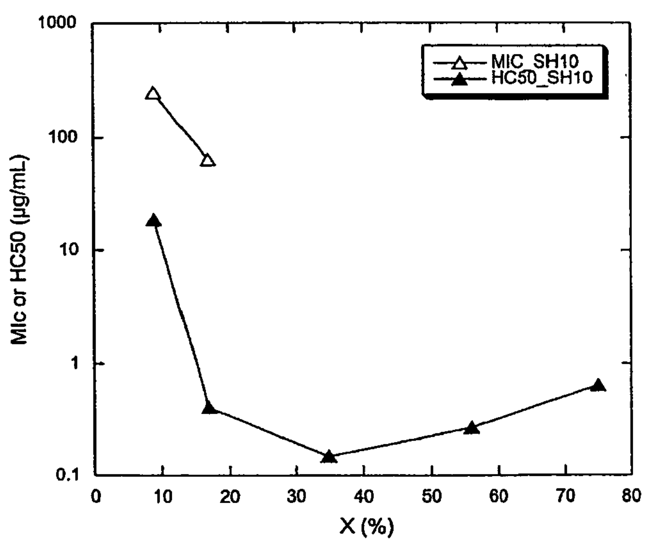

# FIG. 10C

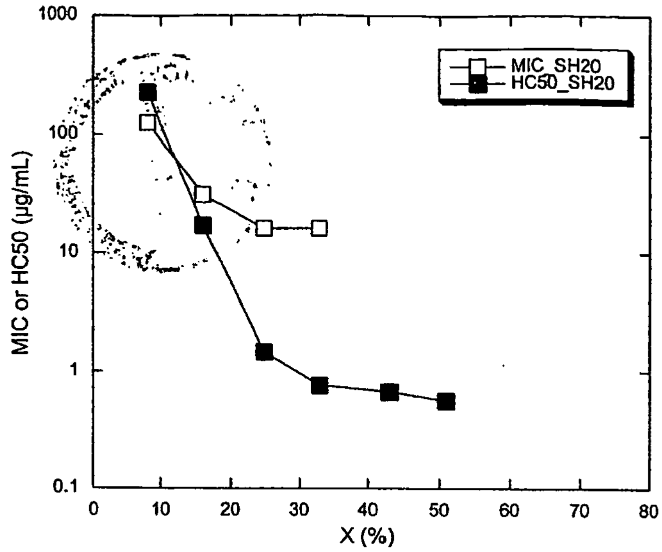


# FIG. 10D

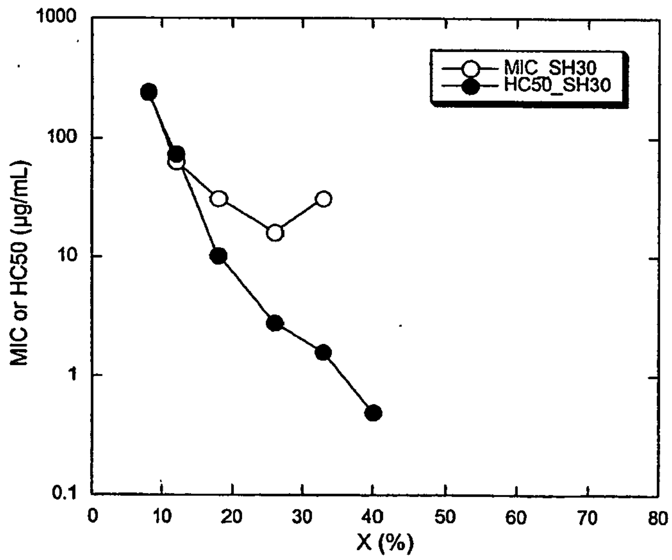

Europäisches Patentamt
European Patent Office
Office européen des brevets

# EUROPEAN SEARCH REPORT

Application Number
EP 11 00 0960

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | US 5 021 311 A (KATO EIICHI [JP] ET AL) 4 June 1991 (1991-06-04)<br>* Macromonomer M-25 *<br>* table 2 *<br>* column 4, line 43 - column 15, line 40 *<br>* Macromonomer M-5 *<br>* table 5 *<br>* the whole document * | 1,27,28<br>1-16,25, 27-29 | INV.<br>C08F226/06<br>A61K31/765<br>C08F2/38<br>C08F220/18<br>C08F220/34<br>C08F220/36<br>C08F230/02 |
| X<br>Y | US 5 967 714 A (OTTERSBACH PETER [DE] ET AL) 19 October 1999 (1999-10-19)<br>* example 1 *<br>* claim 4; example 4 * | 1-16,25, 27-29<br>1-16,25, 27-29 | |
| Y | US 6 552 142 B1 (MEFFERT HELMUT [DE] ET AL) 22 April 2003 (2003-04-22)<br>* abstract *<br>* column 9, lines 23-46 * | 1-16,25, 27-29 | |
| Y | US 6 107 397 A (BLANKENBURG RAINER [DE] ET AL) 22 August 2000 (2000-08-22)<br>* abstract; example 8; table 1 * | 1-16,25, 27-29 | TECHNICAL FIELDS SEARCHED (IPC)<br>C08F |
| Y | US 5 049 383 A (HUTH HANS-ULLRICH [DE] ET AL) 17 September 1991 (1991-09-17)<br>* abstract *<br>* column 9, lines 6-10 * | 1-16,25, 27-29 | |
| Y | US 2004/052746 A1 (TAMARESELVY KRISHNAN [US] ET AL) 18 March 2004 (2004-03-18)<br>* abstract *<br>* paragraphs [0103] - [0108] *<br>* paragraph [0163] * | 1-16,25, 27-29 | |

-/--

The present search report has been drawn up for all claims

1

| Place of search | Date of completion of the search | Examiner |
|---|---|---|
| The Hague | 15 April 2011 | Dalet, Pierre |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 11 00 0960

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br>Y | WO 01/72715 A (UNIV CALIFORNIA [US]; SUN GANG [US]; SUN YUYU [US])<br>4 October 2001 (2001-10-04)<br>* abstract *<br>* table 2 *<br>----- | 1-16,25,<br>27,29<br><br>1-16,25,<br>27,29 | |
| Y | ODIAN G: "RADICAL CHAIN POLYMERIZATION", PRINCIPLES OF POLYMERIZATION, NEW YORK, NY, US,<br>1 January 1970 (1970-01-01), pages 161-278, XP000391599,<br>* pages 203-216 *<br>----- | 1-16,25,<br>27,29 | |
| A,P | US 6 878 387 B1 (PETEREIT HANS-ULRICH [DE] ET AL) 12 April 2005 (2005-04-12)<br>* example 2 *<br>* the whole document *<br>----- | 1-29 | |
| A | GB 1 324 087 A (COMMW SCIENT IND RES ORG)<br>18 July 1973 (1973-07-18)<br>* page 2; table III *<br>* the whole document *<br>----- | 1-29 | TECHNICAL FIELDS SEARCHED (IPC) |
| A | WO 2004/026958 A (KIMBERLY CLARK CO [US])<br>1 April 2004 (2004-04-01)<br>* the whole document *<br>----- | 1-29 | |

The present search report has been drawn up for all claims

1

| Place of search | Date of completion of the search | Examiner |
|---|---|---|
| The Hague | 15 April 2011 | Dalet, Pierre |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 11 00 0960

This annex lists the patent family members relating to the patent documents cited in the above-mentioned European search report.
The members are as contained in the European Patent Office EDP file on
The European Patent Office is in no way liable for these particulars which are merely given for the purpose of information.

15-04-2011

| Patent document cited in search report | Publication date | Patent family member(s) | Publication date |
|---|---|---|---|
| US 5021311 A | 04-06-1991 | JP 2067563 A | 07-03-1990 |
| | | JP 2597160 B2 | 02-04-1997 |
| US 5967714 A | 19-10-1999 | AT 219881 T | 15-07-2002 |
| | | CA 2231101 A1 | 06-09-1998 |
| | | DE 19709075 A1 | 10-09-1998 |
| | | DK 862858 T3 | 14-10-2002 |
| | | EP 0862858 A1 | 09-09-1998 |
| | | JP 10251350 A | 22-09-1998 |
| | | NO 980981 A | 07-09-1998 |
| US 6552142 B1 | 22-04-2003 | AT 283874 T | 15-12-2004 |
| | | CN 1264712 A | 30-08-2000 |
| | | DE 19908184 A1 | 31-08-2000 |
| | | EP 1031585 A2 | 30-08-2000 |
| | | ES 2233231 T3 | 16-06-2005 |
| | | JP 2000248004 A | 12-09-2000 |
| US 6107397 A | 22-08-2000 | EP 0867455 A2 | 30-09-1998 |
| | | JP 10279637 A | 20-10-1998 |
| US 5049383 A | 17-09-1991 | AU 609461 B2 | 02-05-1991 |
| | | AU 1430388 A | 13-10-1988 |
| | | CA 1331725 C | 30-08-1994 |
| | | DE 3711680 A1 | 27-10-1988 |
| | | DK 185488 A | 08-10-1988 |
| | | EP 0286009 A2 | 12-10-1988 |
| | | FI 881560 A | 08-10-1988 |
| | | JP 63264502 A | 01-11-1988 |
| | | NO 881469 A | 10-10-1988 |
| | | PT 87172 A | 01-05-1988 |
| | | ZA 8802371 A | 23-09-1988 |
| US 2004052746 A1 | 18-03-2004 | AR 041233 A1 | 11-05-2005 |
| | | AT 332318 T | 15-07-2006 |
| | | AU 2003265662 A1 | 30-04-2004 |
| | | BR 0314242 A | 26-07-2005 |
| | | CA 2498529 A1 | 25-03-2004 |
| | | DE 60306687 T2 | 28-06-2007 |
| | | EP 1581568 A2 | 05-10-2005 |
| | | ES 2266904 T3 | 01-03-2007 |
| | | JP 4482452 B2 | 16-06-2010 |
| | | JP 2006512425 T | 13-04-2006 |
| | | KR 20050054941 A | 10-06-2005 |
| | | MX PA05002684 A | 05-05-2005 |
| | | WO 2004024779 A2 | 25-03-2004 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 11 00 0960

This annex lists the patent family members relating to the patent documents cited in the above-mentioned European search report.
The members are as contained in the European Patent Office EDP file on
The European Patent Office is in no way liable for these particulars which are merely given for the purpose of information.

15-04-2011

| Patent document cited in search report | Publication date | Patent family member(s) | Publication date |
|---|---|---|---|
| US 2004052746 A1 | | US 2008233069 A1 | 25-09-2008 |
| | | US 2004241130 A1 | 02-12-2004 |
| WO 0172715 A | 04-10-2001 | AU 5091801 A | 08-10-2001 |
| | | CA 2404255 A1 | 04-10-2001 |
| | | EP 1265869 A2 | 18-12-2002 |
| | | JP 2003528860 T | 30-09-2003 |
| | | US 2003216581 A1 | 20-11-2003 |
| | | US 6768009 B1 | 27-07-2004 |
| US 6878387 B1 | 12-04-2005 | AT 286724 T | 15-01-2005 |
| | | AU 6197499 A | 26-04-2000 |
| | | BR 9913104 A | 08-05-2001 |
| | | CA 2346062 A1 | 13-04-2000 |
| | | CN 1446083 A | 01-10-2003 |
| | | DE 19845358 A1 | 06-04-2000 |
| | | WO 0019984 A2 | 13-04-2000 |
| | | EP 1117387 A2 | 25-07-2001 |
| | | ES 2234303 T3 | 16-06-2005 |
| | | HK 1058152 A1 | 20-05-2005 |
| | | JP 4615124 B2 | 19-01-2011 |
| | | JP 2002526401 T | 20-08-2002 |
| | | MX PA01003392 A | 21-07-2003 |
| | | PL 347780 A1 | 22-04-2002 |
| | | SK 4262001 A3 | 08-10-2002 |
| GB 1324087 A | 18-07-1973 | NONE | |
| WO 2004026958 A | 01-04-2004 | AR 041314 A1 | 11-05-2005 |
| | | AU 2003228262 A1 | 08-04-2004 |
| | | BR 0314099 A | 21-06-2005 |
| | | EP 1539880 A1 | 15-06-2005 |
| | | KR 20050057204 A | 16-06-2005 |
| | | MX PA05002501 A | 27-05-2005 |
| | | US 2004063888 A1 | 01-04-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 02100295 A


### Non-patent literature cited in the description


- **ZASLOFF, M.** *Nature,* 2002, vol. 415, 289
- **BOMAN, H.G.** *Immunol. Rev.,* 2000, vol. 173, 5-16
- **HANCOCK, R.E. ; LEHRER, R.** *Trends Biotechnol.,* 1998, vol. 16, 82-88
- **ZASLOFF, M.** *Curr. Opin. Immunol.,* 1992, vol. 4, 3-7
- **ZASLOFF, M.** *Trends Pharmacol. Sci.,* 2000, vol. 21, 236-238
- **STEINER, H. et al.** *Nature,* 1981, vol. 292, 246-248
- **GANZ, T. et al.** *Eur. J. Haematol.,* 1990, vol. 44, 1-8
- **TANG, Y.Q. et al.** *Science,* 1999, vol. 286, 498-502
- **GANZ, T. et al.** *J. Clin. Invest.,* 1985, vol. 76, 1427-1435
- **LANDON, C. et al.** *Protein Sci.,* 1997, vol. 6, 1878-1884
- **ZHAO, C. et al.** *FEBS Lett.,* 1994, vol. 346, 285-288
- **PEGGION, E. et al.** *Biopolymers (Peptide Science),* 1998, vol. 43, 419-431
- **DEMPSEY, C.E.** *Biochim. Biophys. Acta,* 1990, vol. 1031, 143-161
- **DELUCCA, A.J. ; WALSH, T.J.** *Antimicob. Agents Chemother.,* 1999, vol. 43, 1-11
- **BELAID, A. et al.** *J. Med. Virol.,* 2002, vol. 66, 229-234
- **EGAL, M. et al.** *Int. J. Antimicrob. Agents,* 1999, vol. 13, 57-60
- **ANDERSEN, J.H. et al.** *Antiviral Rs.,* 2001, vol. 51, 141-149
- **BASTIAN, A. ; SCHAFER, H.** *Regul. Pept.,* 2001, vol. 15, 157-161
- **TOSSI, A., et al.** *Biopolymers,* 2000, vol. 55, 4-30
- **DEGRADO, W.F.** *Adv. Protein. Chem.,* 1988, vol. 39, 51-124
- **MALOY, W.L. ; KARI, U.P.** *Biopolymers,* 1995, vol. 37, 105-122
- **BOMAN, H.G. et al.** *Eur. J. Biochem.,* 1991, vol. 201, 23-31
- **OREN, Z. ; SHAI, Y.** *Biopolymers,* 1998, vol. 47, 451-463
- **LIU, D. et al.** *Angew. Chem. Int. Ed.,* 2004, vol. 43, 1158
- **TEW, G.N. et al.** *PNAS,* 2002, vol. 99, 5110
- **ILKER, M.F. et al.** *Macromolecules,* 2004, vol. 37, 694
- **ARNT, L. ; TEW, G.N.** *J. Am. Chem. Soc.,* 2002, vol. 124, 7664
- **GELMAN, M.A. et al.** *Org. Lett.,* 2004, vol. 6, 557
- **DEBONO, M. ; GORDEE, R.S.** *Ann. Rev. Microbiol.,* 1994, vol. 48, 471-497
- **TURPIE, A.G.G.** *Am. Heart J.,* 1998, vol. 135, S329-S335
- **HIRSH, J. ; LEVINE, M.N.** *Blood,* 1992, vol. 79, 1-17
- **HIRSH, J. ; LEVINE, M.N.** *Blood.,* 1992, vol. 79, 1-17
- **WAKEFIELD, T.W. et al.** *J. Surg. Res.,* 1996, vol. 63, 280-286
- **MAYO, F.R.** *J. Am. Chem. Soc.,* 1943, vol. 65, 2324-2329
- **D. BRAUN ; H. CHERDRON ; H. RITTER.** Polymer Synthesis: Theory and Practice. Springer-Verlag
- **SANDA, F. et al.** *Journal of Polymer Science: Part A: Polymer Chemistry,* 1998, vol. 36, 1981-1986
- **HENRIQUEZ, C. et al.** *Polymer,* 2003, vol. 44, 5559-5561
- **DE LA FUENTE, J.L. ; MADRUGA, E.L.** *Journal of Polymer Science: Part A: Polymer Chemistry,* 2000, vol. 38, 170-178
- **T. W. GREENE ; P. G. M. WUTS.** Protective Groups in Organic Synthesis. John Wiley & Sons, Inc, 1999
- **TEW, G.N. et al.** *Proc. Natl. Acad. Sci. USA,* 2002, vol. 99, 5110-5114
- **LIU, D. ; DEGRADO, W.F.** *J. Amer. Chem. Soc.,* 2001, vol. 123, 7553-7559
- **COLE, A.M. et al.** *Proc. Natl. Acad Sci USA,* 2002, vol. 99, 1813-1818

- **EDWARDS, J.R. et al.** *Antimicrobial Agents Chemotherapy,* 1989, vol. 33, 215-222
- **BROEKAERT, W.F. et al.** *FEMS Microbiol. Lett.,* 1990, vol. 69, 55-60
- **KURODA, K ; DEGRADO, W.F.** *J Amer. Chem. Soc.,* 2005, vol. 127, 4128-4129
- **LIU, D. ; DEGRADO,W.F.** *J Amer. Chem. Soc.,* 2001, vol. 123, 7553-7559
- **JAVADPOUR, M.M. et al.** *J. Med. Chem.,* 1996, vol. 39, 3107-3113
- **KANDROTAS, R.J.** *Clin. Pharmacokinet.,* 1992, vol. 22, 359-374
- **DINESS, V.O. ; ØSTERGAARD, P.B.** *Thromb. Haemost.,* 1986, vol. 56, 318-322
- **WONG, P.C. et al.** *J. Pharm. Exp. Therap.,* 2000, vol. 292, 351-357
- **RYN-MCKENNA, J.V. et al.** *Thromb. Haemost.,* 1990, vol. 63, 271-274
- **BANKER ; RHODES.** Modern Pharmaceutics. Marcel Dekker, Inc, 1979
- **GOODMAN ; GILMAN'S.** The Pharmaceutical Basis of Therapeutics. MacMillan Publishing Co, 1980
- **LIU, D. et al.** *Angew. Chem. Int. Ed,* 2004, vol. 43, 1158
- **MAYO, F.R.** *J. Am. Chem. Soc.,* 1943, vol. 65, 2324
- **KURODA et al.** *J. Am. Chem. Soc.,* 2005, vol. 127, 4128-4129
- **KURODA, K ; DEGRADO, W.F.** *J. Amer. Chem. Soc.,* 2005, vol. 127, 4128-4129